# EUROPEAN PATENT APPLICATION

(11) **EP 3 636 668 A2**
(43) Date of publication of application: **15.04.2020**
(21) Application number: 19185357.1
(22) Date of filing: 23.04.2008
(51) Int. Cl.: C07K 16/00, C07H 21/04, C12N 15/00, A61K 39/395, C07K 16/10, C07K 16/12, C07K 16/18, A61P 35/00, A61P 31/18, A61P 25/28, C12N 9/00

(54) **IMMUNOGLOBULINS DIRECTED TO BACTERIAL VIRAL AND ENDOGENOUS POLYPEPTIDES**

(30) Priority: 23.04.2007 US 913335 P
(62) Divisional of application: 08767415.6
(71) Applicant: Paul, Sudhir, Missouri City, TX 77459 (US); Planque, Stephanie, Houston, TX 77054 (US); Brown, Eric L., Houston, TX 77030 (US); Smith, Keri C., Houston, TX 77009 (US); Nishiyama, Yasuhiro, Houston, TX 77030 (US); Taguchi, Hiroaki, Houston, TX 77030 (US)
(72) Inventor: Paul, Sudhir, Missouri City, TX 77459 (US); Planque, Stephanie, Houston, TX 77054 (US); Brown, Eric L., Houston, TX 77030 (US); Smith, Keri C., Houston, TX 77009 (US); Taguchi, Hiroaki, Houston, TX 77030 (US)
(74) Representative: Schneiter, Sorin

(57) **Abstract**

Disclosed are antibodies (immunoglobulins) and fragments thereof that hydrolyze or bind polypeptide antigens belonging to Alzheimer's disease, *Staphyloccus aureus,* hepatitis C virus, and human immunodeficiency virus. Also disclosed are novel methods to improve the antigen reactivity of the immunoglobulins.

## Description

### ABSTRACT

Disclosed are antibodies (immunoglobulins) and fragments thereof that hydrolyze or bind polypeptide antigens belonging to *Staphyloccus aureus,* hepatitis C virus, human immunodeficiency virus and Alzheimer's disease. Also disclosed are novel methods to improve the antigen reactivity of the immunoglobulins.

### FIELD OF THE INVENTION

This invention relates to the fields of biochemistry, immunology, molecular biology and medicine. More specifically, the invention relates to preparation and use of immunoglobulins and fragments thereof with the ability to hydrolyze or bind to bacterial, viral and endogenous polypeptides.

### BACKGROUND OF THE INVENTION

Some immunoglobulins (Igs) have the ability to catalyze chemical reactions through the binding of an antigen, its chemical transformation and the conversion and release of one or more products. Transform of the chemical structure of antigens by such Igs can induce permanent inactivation of antigens. A single catalytic Ig molecule can hydrolyze thousands of antigen molecules over its biological lifetime, which enhances the biological potency of the catalyst compared to a stoichiometrically binding antibody. Catalytic Igs, therefore, can be developed as potent therapeutic agents capable of removing harmful polypeptide and other classes of antigens. Some Igs contain catalytic sites in their variable (V) domains that have properties similar to the catalytic sites of conventional serine protease class of enzymes. Igs with esterolytic and proteolytic activity have been reported [1-5]. Similarly, some Igs hydrolyze nucleic acids [6,7].

An appreciation of the structural organization of Igs is helpful in understanding the scope of the present invention. A brief review of this aspect follows. Generally, Igs contain light (L) chain and heavy (H) chain subunits. The V domains of these subunits contain the antigen binding site (paratope). Contacts with conventional antigenic epitopes occur mainly at the complementarity determining regions (CDRs) and to a lesser extent the framework regions (FR) of the V domains. The human Ig repertoire, defined as the number of Igs with different antigen binding site structures is estimated at 10¹¹-10¹². V domain diversity is generated by the following processes: (a) inheritance of about 50 germline genes each encoding the V domains of L and H subunits; (b) combinatorial diversity brought about by assembly of different L and H chains within the quartenary structure of Igs; (c) junctional diversity generated during recombination of the V and joining (J) gene segments of the L chain, and the V, diversity (D) and J gene segments of the H chain; and (d) rapid mutation occurring in the CDRs over the course of B cell clonal selection, a process entailing antigen binding to Igs expressed as components of the B cell receptor (BCR), and resulting in stimulation of division of the B cells expressing BCRs with the highest binding affinity. An additional level of diversity is offered by the use of different constant domains by Igs, that is, the µ, δ, γ, and α regions of the H chain and the κ and λ chains of the L chain. Early in the ontogeny of the immune response, Igs contain µ or δ constant regions. Later, isotype switching occurs, and the µ/δ regions are replaced by γ/α/ε in more differentiated Igs.

Various advances of technology in monoclonal and recombinant Ig techniques have accelerated the identification, selection and purification of catalytic Ig species. One approach to generating catalytic Igs involves immunizing an animal with a stable analog of the transition state of the reaction to be catalyzed and screening for Igs that bind more strongly to the transition state analog than to the corresponding substrate. The Igs, like enzymes, have a site that is complementary to the 3-D and ionic structure of the transition state analog. A large number of Igs synthesized in response to immunization with a transition state analog can bind the analog, but only a small minority will catalyze the reaction of interest. For example, only one catalytic Ig may be found for every 100-1,000 Igs screened. Another major challenge is that the catalytic activity of the Igs can be low compared to the naturally occurring enzymes, usually by a factor of 10³ or more.

To be medically useful, the catalytic Ig must also be specific for the desired target antigen. While promiscuous catalytic Igs capable of hydrolyzing polypeptides are common [5,8-10], specific catalytic Igs directed to medically important target proteins are rare.

Advances in the field of catalytic Igs, therefore, are dependent on identifying Igs that have high level catalytic activity and the correct epitope specificity enabling specific catalysis directed against the target antigen. The foregoing problems have been recognized for many years. Numerous solutions have been proposed, but none adequately address the problems that must be solved in isolating Igs that have the ability to specifically catalyze medically important biochemical reactions. Renewable and homogeneous sources of well-characterized catalytic Igs are needed for medical applications. A brief review of Ig technologies that may be useful in isolating catalytic Igs follows.

Traditional methods to clone Igs from humans consist of immortalizing lymphocytes derived from peripheral blood (or lymphoid tissues obtained by surgery), for example by transformation with Epstein Barr virus followed by fusion with a myeloma cell lines. The resultant hybridoma cell lines are screened for production of the desired Abs, for example by measuring the binding to a specific antigen by ELISA.

Methods are also available to clone the expressed Ig V domain repertoires in the form of libraries displayed on a suitable surface. The Ab fragments can be cloned as Fab fragments, single chain Fv (scFv) fragments or the L chain subunits. Fab and scFv constructs usually reproduce faithfully the binding activity of full-length Abs (e.g., [11]). Previous reports have documented the antigen binding activity of L chain subunit independent of its H chain partner, albeit at reduced strengths compared to native Abs [12,13]. The V domains of the scFv fragments are usually linked by flexible peptide linkers. Cloning of V domain repertoires is usually accomplished by recovering mRNA from lymphocytes and amplification by the reverse transcriptase-polymerase chain reaction. Mixtures of primers are employed to capture as large a proportion of the expressed repertoire as possible. The primers anneal to comparatively conserved FR1 and FR4 nucleotide stretches located at the 5' and 3' ends of the V domains, respectively, allowing amplification of V domains belonging diverse V gene families. To obtain expressible scFv constructs, the VL and VH domains are cloned into a suitable vector containing a short flexible peptide and an inducible promoter. Peptide tags such as the his6 tag are incorporated into the protein to enable rapid purification by metal affinity chromatography. The length and constitution of the peptide linker is an important variable in ensuring the appropriate intramolecular VL-VH interactions.

The next task is to isolate the minority of individual antigen combining sites with the desired antigen recognition characteristics. This can be accomplished using display technologies [14]. Vectors permitting display of recombinant proteins on the surfaces of phages, retroviruses, bacteria and yeast have been developed. For example, fusion proteins composed of Ig fragments linked to a phage coat protein are expressed from phagemid or phage vectors in bacteria. The recombinant phages display Ig fragments on their surface. The packaged phages contain single stranded DNA encoding the Ig fusion protein. Fractionation of phages based on binding to immobilized antigen yields, therefore, the VL/VH genes of Abs with the desired specificity. Phagemid vectors are useful because a codon at the junction of the Ab and phage coat protein genes is read as a sense codon by bacteria employed to package phages and as a stop codon by bacteria employed to obtain soluble Ab fragments free of the phage coat protein sequence.

Immunotherapeutic agents should preferably have a long half-life to avoid repeated infusions. The half-life of full-length IgG in human circulation is 2-3 weeks, compared to half-lives on the order of minutes for scFv constructs and free Ig L chain subunits. Therefore, various strategies have been developed to increase the stability of Ig fragments in vivo. Examples are the inclusion of a polyethylene glycol molecule at the Ig fragment terminus [15,16] or linkage to the constant region of Igs (Fc) [17]. The V domains can also be routinely recloned in vectors containing the constant domains of heavy and light chains. Expression of these vectors in suitable mammalian cells yields full-length Igs with increased half-life in vivo [18].

The properties of the target antigen are important in the success of medical applications of catalytic Igs. The targeted antigen can be chosen for Ig targeting based on the principles. First, the antigen should fulfill a pathogenic role. For example, the targeted antigen may interfere in some essential endogenous cellular or metabolic function. Alternatively, in the case of microbial antigens, the antigen should be important for growth of the microbe or it may be important in diverting host immune responses away from protection against the microbe. Second, removal of the antigen by Igs should not be associated with a deleterious side effect. This is particularly important in targeting of an endogenous antigen by Igs, e.g., amyloid β peptide in Alzheimer disease, as most endogenous antigens fulfill useful biological functions. In the case of microbial antigens, the danger of cross-reaction with endogenous antigens should be minimized. Immune complexes of antigens with Igs containing Fc regions have the potential of reacting with Fc receptors expressed on inflammatory cells and inducing undesirable inflammatory reactions. This danger is minimized if the Ig has catalytic activity, as the longevity of the immune complexes is reduced due to antigen chemical transformation and product release.
Examples of antigens suitable for Ig targeting are presented below.

*Amyloid β peptide (Aβ).* Aβ is the target of conventional non-catalytic Igs in ongoing clinical trials for the treatment of Alzheimer disease. A monoclonal IgG [19] and pooled polyclonal IgG from healthy humans [20] are under trial. The rationale for Aβ targeting is as follows. In 1907 that the first pathological lesion associated with dementia, the cerebral plaque, was reported by Alzheimer [21]. The cerebral lesion was called an "amyloid" plaque because iodine, which stained the cerebral plaque, also stains starch. The true chemical composition of the "amyloid" plaque was elusive until 1984 when Glenner and Wang discovered a means to solubilize the cerebral plaques in Alzheimer's disease (AD) and showed that they are composed principally of peptides Aβ1-40 and Aβ1-42. These peptides are identical but for the two additional amino acids, Ile and Ala, at the C-terminus of Aβ1-42 [22]. Both peptides are derived by proteolytic processing of the larger amyloid precursor protein (APP), which is composed of 770 amino acids and has the characteristics of a transmembrane protein [23]. APP is cleaved by β and γ secretases, releasing the Aβ peptides [24,25]. The role of Aβ peptides in the pathogenesis of AD is supported by findings that: (a) Familial AD is associated with mutations in the APP gene or the secretase genes; (b) Transgenic mice, expressing mutant human, APP genes develop an age-associated increase in cerebral Aβ peptides and amyloid plaques as well as cognitive decline [26,27]; (c) Mutant, human APP-tg mice that do not process APP to Aβ show no cognitive decline, suggesting that increased Aβ peptides and not the mutant APP is responsible for cognitive decline [28,29]; and (d) Synthetic Aβ peptides and their oligomers are neurotoxic *in vitro* [30,31].

*HIV gp120.* Igs directed to the HIV coat glycoprotein gp120 have long been under consideration for immunotherapy of HIV infection. A key step in HIV infection is the binding of gp120 to host cell CD4 receptors. Additionally, gp120 plays a significant role in viral propagation and demonstrates a toxic effect on cells that are not infected with HIV [32,33]. gp120 is toxic for neurons, it facilitates lyses of lympocytes by an antibody-dependent mechanism, and it increases the binding of complement components to cells [34-39]. It has been shown that monoclonal Igs can bind the CD4 binding site (e.g., [40,41]). However, gp120 expresses many antigenic epitopes, and the immunodominant epitopes are located in the variable regions of gp120. Igs to the immunodominant epitopes do not neutralize diverse strains with varying sequence of the variable gp120 regions. Igs to the conserved gp120 sequences are necessary for therapy of HIV infection. Such Igs can also be used as topical microbicides to prevent vaginal and rectal transmission of HIV via sexual intercourse. gp120 contains a B cell superantigenic epitopes, defined as an antigenic epitope to which Igs are present in the preimmune repertoire without the requirement of adaptive immune specialization [42] . Residues 421-433 of this epitope are also important in CD4 host receptor binding [43,44]. Igs to the superantigenic epitope hold the potential of neutralizing HIV broadly. However, superantigens are thought to be recognized mainly by conserved regions of Ig V domains, including the conserved regions of the FRs and CDRs. Adaptive improvement of the superantigen recognition function appears to be difficult. No monoclonal or polyclonal Igs with the ability to neutralize the entire range of HIV strains belonging to various clades are available.

*Staphylococcus aureus.* This bacterium is an opportunistic pathogen that colonizes the skin (primarily the anterior nasal vestibule) of approximately 30-50% (with 20-30% persistently colonized) of the population without causing clinical disease symptoms [45,46]. Persistently colonized individuals are designated 'carriers'. Such individuals are designated 'carriers'. A minority of humans harboring the bacterium develop clinical disease ranging from minor skin infections to lethal infections associated with abscess formation, endocarditis and pneumonia. Certain antibiotics are available for the treatment of *S. aureus-caused* disease, but the number of antibiotic-resistant strains is increasing rapidly. Host immunological factors play a role in determining susceptibility to initial colonization and development of *S. aureus* disease. However, certain virulence factors produced by *S. aureus* can downregulate host immune defenses profoundly, helping the bacterium colonize various anatomic sites and cause serious disease. The protective effect of Igs directed against *S. aureus* antigens has been reported in several experimental studies [47-49]. Persistent antigenic exposure in *S. aureus* carriers can be hypothesized to induce adaptive synthesis of protective Igs. An insufficient adaptive response may be a predisposing factor in progression of infection. As noted above, certain microbial antigens behave as B cell superantigens. If *S. aureus* proteins have superantigenic character, protective Igs to the bacterium may be produced spontaneously without prior infection. *S. aureus* produces a host of virulence factors important in bacterial adhesion, toxicity for host cells and modulation of the host immune syste. Selected *S. aureus* suitable for targeting by Igs are listed in Table 1.

**Table 1. Example S. aureus proteins suitable for targeting by Igs**

| *S. aureus* proteins | Function |
|---|---|
| Efb | Immune Impairment |
| Protein A | Immune Evasion |
| Map19 (Eap) | Immune Impairment |
| ClfA₂₂₉₋₅₄ | Fibrinogen/Fibronectin Adhesin |
| ClfB₂₀₁₋₅₄₂ | Fibrinogen/Cytokeratin Adhesin |
| FnbpA | Fibronectin Adhesin |
| CAN | Collagen Adhesin |
| SdrE₅₁₋₆₀₆ | Potential Adhsin |
| Alpha toxin | Toxin |
| LukF | Toxin |
| LukS | Toxin |

*Hepatitis C virus (HCV).* HCV infection leads to chronic hepatitis, liver failure and hepatocellular carcinoma. It is estimated that over 170 million people worldwide are infected with HCV with the majority leading to chronic disease [50]. HCV genotypes 1a, 1b, 2a and 2b are common in the United States. Liver transplantation often is necessary due to liver cirrhosis secondary to viral infection. The mainstay of current therapy is a combination of interferon and ribavirin, which leads to clinical improvement in a subpopulation of patients with chronic HCV infection. Clearly, more strategies are needed for treatment and prevention of HCV infection [51]. The E2 coat protein expressed by HCV is thought to be essential for viral infection by virtue of its role in host cell binding [52]. E2 contains hypervariable regions and comparatively conserved regions. Igs to the hypervariable regions are frequent in infected individuals [53]. Conventional non-catalytic Igs to E2 have been suggested to be important in control of virus infection [53]. Certain monoclonal Igs to E2 neutralize the virus and [54] are under consideration for therapy of HCV infection.

### References

1. Sun M, Gao QS, Li L, Paul S: Proteolytic activity of an antibody light chain. J Immunol 1994, 153:5121-5126.
2. Paul S: Catalytic activity of anti-ground state antibodies, antibody subunits, and human autoantibodies. Appl Biochem Biotechnol 1994, 47:241-253; discussion 253-245.
3. Li L, Sun M, Gao QS, Paul S: Low level formation of potent catalytic IgG fragments mediated by disulfide bond instability. Mol Immunol 1996, 33:593-600.
4. Li L, Paul S, Tyutyulkova S, Kazatchkine MD, Kaveri S: Catalytic activity of anti-thyroglobulin antibodies. J Immunol 1995, 154:3328-3332.
5. Kalaga R, Li L, O'Dell JR, Paul S: Unexpected presence of polyreactive catalytic antibodies in IgG from unimmunized donors and decreased levels in rheumatoid arthritis. J Immunol 1995, 155:2695-2702.
6. Shuster AM, Gololobov GV, Kvashuk OA, Bogomolova AE, Smirnov IV, Gabibov AG: DNA hydrolyzing autoantibodies. Science 1992, 256:665-667.
7. Matsuura K, Ikoma S, Yoshida K, Sinohara H: DNA-hydrolyzing activity of Bence Jones proteins. Biochem Biophys Res Commun 1998, 243:719-721.
8. Paul S, Li L, Kalaga R, Wilkins-Stevens P, Stevens FJ, Solomon A: Natural catalytic antibodies: peptide-hydrolyzing activities of Bence Jones proteins and VL fragment. J Biol Chem 1995, 270:15257-15261.
9. Planque S, Bangale Y, Song XT, Karle S, Taguchi H, Poindexter B, Bick R, Edmundson A, Nishiyama Y, Paul S: Ontogeny of proteolytic immunity: IgM serine proteases. J Biol Chem 2004, 279:14024-14032.
10. Mitsuda Y, Planque S, Hara M, Kyle R, Taguchi H, Nishiyama Y, Paul S: Naturally occurring catalytic antibodies: evidence for preferred development of the catalytic function in IgA class antibodies. Mol Biotechnol 2007, 36:113-122.
11. Pantoliano MW, Bird RE, Johnson S, Asel ED, Dodd SW, Wood JF, Hardman KD: Conformational stability, folding, and ligand-binding affinity of single-chain Fv immunoglobulin fragments expressed in Escherichia coli. Biochemistry 1991, 30:10117-10125.
12. Masat L, Wabl M, Johnson JP: A simpler sort of antibody. Proc Natl Acad Sci U S A 1994, 91:893-896.
13. Sun M, Li L, Gao QS, Paul S: Antigen recognition by an antibody light chain. J Biol Chem 1994, 269:734-738.
14. Marks JD, Hoogenboom HR, Bonnert TP, McCafferty J, Griffiths AD, Winter G: Bypassing immunization. Human antibodies from V-gene libraries displayed on phage. J Mol Biol 1991, 222:581-597.
15. Kubetzko S, Balic E, Waibel R, Zangemeister-Wittke U, Pluckthun A: PEGylation and multimerization of the anti-p185HER-2 single chain Fv fragment 4D5: effects on tumor targeting. J Biol Chem 2006, 281:35186-35201.
16. Krinner EM, Hepp J, Hoffmann P, Bruckmaier S, Petersen L, Petsch S, Parr L, Schuster I, Mangold S, Lorenczewski G, et al.: A highly stable polyethylene glycol-conjugated human single-chain antibody neutralizing granulocyte-macrophage colony stimulating factor at low nanomolar concentration. Protein Eng Des Sel 2006, 19:461-470.
17. Yang K, Basu A, Wang M, Chintala R, Hsieh MC, Liu S, Hua J, Zhang Z, Zhou J, Li M, et al.: Tailoring structure-function and pharmacokinetic properties of single-chain Fv proteins by site-specific PEGylation. Protein Eng 2003, 16:761-770.
18. McLean GR, Nakouzi A, Casadevall A, Green NS: Human and murine immunoglobulin expression vector cassettes. Mol Immunol 2000, 37:837-845.
19. DeMattos RB, Bales KR, Cummins DJ, Dodart JC, Paul SM, Holtzman DM: Peripheral anti-A beta antibody alters CNS and plasma A beta clearance and decreases brain A beta burden in a mouse model of Alzheimer's disease. Proc Natl Acad Sci U S A 2001, 98:8850-8855.
20. Dodel RC, Du Y, Depboylu C, Hampel H, Frolich L, Haag A, Hemmeter U, Paulsen S, Teipel SJ, Brettschneider S, et al.: Intravenous immunoglobulins containing antibodies against beta-amyloid for the treatment of Alzheimer's disease. J Neurol Neurosurg Psychiatry 2004, 75:1472-1474.
21. Alzheimer A: Uber eine eigenartige Erkrankung der Hirnrinde. Allg. Z. Psychiat. 1907, 64:146-148.
22. Glenner GG, Wong CW: Alzheimer's disease and Down's syndrome: sharing of a unique cerebrovascular amyloid fibril protein. Biochem Biophys Res Commun 1984, 122:1131-1135.
23. Kang J, Lemaire HG, Unterbeck A, Salbaum JM, Masters CL, Grzeschik KH, Multhaup G, Beyreuther K, Muller-Hill B: The precursor of Alzheimer's disease amyloid A4 protein resembles a cell-surface receptor. Nature 1987, 325:733-736.
24. Haass C, Schlossmacher MG, Hung AY, Vigo-Pelfrey C, Mellon A, Ostaszewski BL, Lieberburg I, Koo EH, Schenk D, Teplow DB, et al.: Amyloid beta-peptide is produced by cultured cells during normal metabolism. Nature 1992, 359:322-325.
25. Seubert P, Vigo-Pelfrey C, Esch F, Lee M, Dovey H, Davis D, Sinha S, Schlossmacher M, Whaley J, Swindlehurst C, et al.: Isolation and quantification of soluble Alzheimer's beta-peptide from biological fluids. Nature 1992, 359:325-327.
26. Games D, Adams D, Alessandrini R, Barbour R, Berthelette P, Blackwell C, Carr T, Clemens J, Donaldson T, Gillespie F, et al.: Alzheimer-type neuropathology in transgenic mice overexpressing V717F beta-amyloid precursor protein. Nature 1995, 373:523-527.
27. Morgan D, Diamond DM, Gottschall PE, Ugen KE, Dickey C, Hardy J, Duff K, Jantzen P, DiCarlo G, Wilcock D, et al.: A beta peptide vaccination prevents memory loss in an animal model of Alzheimer's disease. Nature 2000, 408:982-985.
28. Citron M, Oltersdorf T, Haass C, McConlogue L, Hung AY, Seubert P, Vigo-Pelfrey C, Lieberburg I, Selkoe DJ: Mutation of the beta-amyloid precursor protein in familial Alzheimer's disease increases beta-protein production. Nature 1992, 360:672-674.
29. Ohno M, Sametsky EA, Younkin LH, Oakley H, Younkin SG, Citron M, Vassar R, Disterhoft JF: BACE1 deficiency rescues memory deficits and cholinergic dysfunction in a mouse model of Alzheimer's disease. Neuron 2004, 41:27-33.
30. Solomon B, Koppel R, Frankel D, Hanan-Aharon E: Disaggregation of Alzheimer beta-amyloid by site-directed mAb. Proc Natl Acad Sci U S A 1997, 94:4109-4112.
31. Chromy BA, Nowak RJ, Lambert MP, Viola KL, Chang L, Velasco PT, Jones BW, Fernandez SJ, Lacor PN, Horowitz P, et al.: Self-assembly of Abeta(1-42) into globular neurotoxins. Biochemistry 2003, 42:12749-12760.
32. Kido H, Kamoshita K, Fukutomi A, Katunuma N: Processing protease for gp160 human immunodeficiency virus type I envelope glycoprotein precursor in human T4+ lymphocytes. Purification and characterization. J Biol Chem 1993, 268: 13406-13413.
33. Capon DJ, Ward RH: The CD4-gp120 interaction and AIDS pathogenesis. Annu Rev Immunol 1991, 9:649-678.
34. Gelderblom HR, Reupke H, Pauli G: Loss of envelope antigens of HTLV-III/LAV, a factor in AIDS pathogenesis? Lancet 1985, 2:1016-1017.
35. Brenneman DE, Westbrook GL, Fitzgerald SP, Ennist DL, Elkins KL, Ruff MR, Pert CB: Neuronal cell killing by the envelope protein of HIV and its prevention by vasoactive intestinal peptide. Nature 1988, 335:639-642.
36. Muller WE, Schroder HC, Ushijima H, Dapper J, Bormann J: gp120 of HIV-1 induces apoptosis in rat cortical cell cultures: prevention by memantine. Eur J Pharmacol 1992, 226:209-214.
37. Hober D, Jewett A, Bonavida B: Lysis of uninfected HIV-1 gp120-coated peripheral blood-derived T lymphocytes by monocyte-mediated antibody-dependent cellular cytotoxicity. FEMS Immunol Med Microbiol 1995, 10:83-91.
38. Laurent-Crawford AG, Coccia E, Krust B, Hovanessian AG: Membrane-expressed HIV envelope glycoprotein heterodimer is a powerful inducer of cell death in uninfected CD4+ target cells. Res Virol 1995, 146:5-17.
39. Stoiber H, Ebenbichler C, Schneider R, Janatova J, Dierich MP: Interaction of several complement proteins with gp120 and gp41, the two envelope glycoproteins of HIV-1. Aids 1995, 9:19-26.
40. Thali M, Olshevsky U, Furman C, Gabuzda D, Posner M, Sodroski J: Characterization of a discontinuous human immunodeficiency virus type 1 gp120 epitope recognized by a broadly reactive neutralizing human monoclonal antibody. J Virol 1991, 65:6188-6193.
41. Thali M, Furman C, Ho DD, Robinson J, Tilley S, Pinter A, Sodroski J: Discontinuous, conserved neutralization epitopes overlapping the CD4-binding region of human immunodeficiency virus type 1 gp120 envelope glycoprotein. J Virol 1992, 66:5635-5641.
42. Berberian L, Goodglick L, Kipps TJ, Braun J: Immunoglobulin VH3 gene products: natural ligands for HIV gp120. Science 1993, 261:1588-1591.
43. Karray S, Zouali M: Identification of the B cell superantigen-binding site of HIV-1 gp120. Proc Natl Acad Sci U S A 1997, 94:1356-1360.
44. Goodglick L, Zevit N, Neshat MS, Braun J: Mapping the Ig superantigen-binding site of HIV-1 gp120. J Immunol 1995, 155:5151-5159.
45. Kissane JM: Staphylococcal Infections. In Pathology of Infectious Diseases. Edited by Connor DH, Chandler FW, Schwartz HJ, Manz HJ, Lack EE: Appleton and Lange; 1997:805-816. vol I.]
46. Lowy FD: Staphylococcus aureus infections. N. Engl. J. Med. 1998, 339:520-532.
47. Mamo W, Boden M, Flock JI: Vaccination with Staphylococcus aureus fibrinogen binding proteins (FgBPs) reduces colonisation of S. aureus in a mouse mastitis model. FEMS Immunol Med Microbiol 1994, 10:47-53.
48. Lee JC: Development of Antistaphylococcal Vaccines. Curr Infect Dis Rep 2001, 3:517-524.
49. Schaffer AC, Solinga RM, Cocchiaro J, Portoles M, Kiser KB, Risley A, Randall SM, Valtulina V, Speziale P, Walsh E, et al.: Immunization with Staphylococcus aureus clumping factor B, a major determinant in nasal carriage, reduces nasal colonization in a murine model. Infect Immun 2006, 74:2145-2153.
50. Centers for Disease Control and Prevention. Disease burden from viral hepatitis A, B and C in the United States. URL: http://www.cdc.gov/nicdod/diseases/hepatitis/heptab3.htm 1997.
51. Dusheiko G: Side effects of interferon alpha in viral hepatitis. NIH Consensus Development Conference on Management of Hepatitis C 1997, Program and Abstracts IV:105-112.
52. Barth H, Liang TJ, Baumert TF: Hepatitis C virus entry: molecular biology and clinical implications. Hepatology 2006, 44:527-535.
53. Zeisel MB, Fafi-Kremer S, Fofana I, Barth H, Stoll-Keller F, Doffoel M, Baumert TF: Neutralizing antibodies in hepatitis C virus infection. World J Gastroenterol 2007, 13:4824-4830.
54. Galun E, Terrault NA, Eren R, Zauberman A, Nussbaum O, Terkieltaub D, Zohar M, Buchnik R, Ackerman Z, Safadi R, et al.: Clinical evaluation (Phase I) of a human monoclonal antibody against hepatitis C virus: safety and antiviral activity. J Hepatol 2007, 46:37-44.

### SUMMARY OF THE INVENTION

The present invention describes several classes of Ig fragments that display high level catalytic and binding activities and the desired bioactivity profiles. Thereby, the invention addresses the need for improved compositions and methods for the preparation of antibodies that can be used for medical applications.

One class of Ig fragments in the present invention consists of single domain VL constructs (IgV_{L} constructs) that contain a peptide tag (designated *t*) at their termini. The structure of the terminal *t* region can be varied without loss of IgV_{L} function). The purpose of including the *t* region is to interfere with noncovalent V domain association and maintenance in monomeric form. The invention discloses the unanticipated finding that the monomeric form of the VL domains expresses superior catalytic activity compared to physiological Ig combining sites composed of paired VL domain-VH domain structures.

Another class of Ig fragments in the present invention consists of two domain V_{L}-V_{L} constructs (IgV_{L2} constructs) expressing superior catalytic activity compared to physiological Ig combining sites. Evidently, pairing of two VL domains is more permissive for expression of catalytic activity compared to pairing of a VL domain with a VH domain.

Optionally, the *t* region can contain peptide or non-peptide structures that direct the IgV to the desired target anatomic site. For example, inclusion of polyanionic compound such as a putrescine in the *t* region can facilitate passage of the IgV across the blood-brain-barrier.

In another embodiment, the present invention describes the composition and properties of IgVs directed to amyloid β peptide (Aβ), *S. aureus* virulence factors and HIV gp120 identified by screening and fractionation of libraries composed of a large number of IgV constructs. The IgVs can be identified by random screening of a plurality of the IgV clones for the desired antigen recognition activities. Alternatively, the antigen or analogs of the antigen containing an electrophilic group can be used to fractionate phage displayed IgVs to isolate the IgV species with greatest activity. These methods can be employed to yield IgVs with the ability to recognize the antigen by noncovalent means or IgVs that catalyze the hydrolysis of the antigen.

Another embodiment of the invention concerns recognition of B cell superantigens by the V domains, which is thought to occur mainly at conserved V domain residues. Swapping of the framework regions (FRs) or complementarity determining regions (CDRs) of the VH domains by corresponding FRs or CDRs drawn from other V domains is shown to improve B cell superantigen recognition capability. Similarly, mutations introduced at individual amino acids in the FRs or CDRs can be employed to improve the ability of IgVs to recognize B cell superantigens.

The IgVs can be engineered further to improve their stability in vascular circulation and other anatomic sites, for example by linkage to a polyethylene glycol molecule or the Fc region of Igs. Aslo disclosed are novel full-length catalytic IgG*L* and IgM*L* molecules containing antigen combining sites composed of 2 VL domains instead of one VL domain and one VH domain. These molecules contain a VL domain is grafted in place of the VH domain within the heavy chain subunit, along with the VL domain contained in the light chain subunit.

Another embodiment of the invention consists of identifying polyclonal Igs from blood and mucosal secretions with enriched catalytic activity. The catalytic activity is directed to one or more protein, e.g., an *S. aureus* virulence factor, the HCV coat protein E2, the HIV coat protein gp120 or Aβ. For example, the invention discloses finings of HIV neutralization by long-term survivors of HIV infection that are a suitable source of pooled Igs for treatment and prevention of HIV infection. Likewise, screening of human donors has revealed highly variable levels of catalytic Igs directed to *S aureus* virulence factors and HCV E2. Pooled Igs from donors expressing high level catalytic activities to the appropriate antigens are conceived to be useful in various medical treatments, including treatment of antibiotic-resistant *S. aureus infection,* HCV infection and Alzheimer disease.

The present invention is also directed any may encompass to one or more of the following 38 embodiments and various combinations thereof, particular with the previous embodiments:
Embodiment 1: An immunoglobulin (Ig) variable (V) domain derived from a light chain subunit (VL) or heavy chain subunit (VH) of an immunoglobulin and having a defined antigenic-directed catalytic activity or binding activity, wherein the catalytic activity hydrolyzes one or more peptide bonds in amyloid β peptide, HIV gp120, a Staphylococcus aureus virulence factor, or HCV E2.
Embodiment 2: An immunoglobulin (Ig) variable (V) domain derived from a light chain subunit (VL) or heavy chain subunit (VH) of an immunoglobulin and having a defined antigenic-directed catalytic activity or binding activity.
Embodiment 3: The IgV domain of embodiment 1 or 2, further comprising a second IgV domain covalently linked thereto to form a dimer, said dimer having two VL domains or two VH domains.
Embodiment 4. The IgV domain of any one of embodiments 1-3, wherein the two IgV domains of the VL dimer are covalently linked by a peptide linker between the termini of the VL domains.
Embodiment 5. The IgV domain of any one of embodiments 1 to 4, further comprising a peptide or non-peptide tag effective to reduce or to eliminate intermolecular V domain aggregation reactions.
Embodiment 6. The IgV domain of embodiment 5, wherein the tag facilitates delivery of the IgV domain(s) to an anatomic site of interest.
Embodiment 7. The IgV domain of embodiment 6, wherein the anatomic site of interest is the blood-brain barrier.
Embodiment 8. The IgV domain of embodiment 7, wherein the tag is putrescine, TAT peptide or an antibody to a molecule expressed on cells forming the blood-brain-barrier.
Embodiment 9. The IgV domain of any of embodiments 1 to 8, further comprising a stabilizing molecule effective to reduce metabolic clearance.
Embodiment 10. The IgV domain of embodiment 9, wherein the stabilizing molecule is polyelthylene glycol at a C terminus of the domain or a protein domain linked thereto.
Embodiment 11. The IgV domain of any of embodiments 1 to 10, further comprising an Fc region of an immunoglobulin linked thereto.
Embodiment 12. The IgV domain of any of embodiments 1 to 11, wherein the IgV domain is derived from lymphoid cells from an organism with lupus, Alzheimer's disease, a Staphylococcus aureus infection, an HIV infection or an HCV infection. Embodiment 13. The IgV domain of any of embodiments 1 to 12, wherein the catalytic activity hydrolyzes one or more peptide bonds in amyloid β peptide, HIV gp120, a Staphylococcus aureus virulence factor, or HCV E2.
Embodiment 14. The IgV domain of embodiment 13, wherein the peptide bonds comprise amyloid β peptide, HIV gp120, a Staphylococcus aureus virulence factor, or HCV E2.
Embodiment 15. The IgV domain of any one of embodiments 1 to 14, wherein the binding activity is HIV gp120 binding.
Embodiment 16. The IgV domain of any one of embodiments 1 to 15, wherein the antigen is a B cell superantigen.
Embodiment 17. The IgV domain of embodiment 16, wherein the B cell superantigen is HIV gp120 or a Staphylococcus aureus virulence factor.
Embodiment 18. The IgV domain of embodiment 17, wherein the HIV gp120 antigen has an epitope comprising at least one or more of amino acids 421-433.
Embodiment 19. The IgV domain of any of embodiments 1 to 18, wherein one or both V domains have a mutation effective to increase the catalytic or binding activity by at least 50%.
Embodiment 20. The IgV domain of embodiment 19, wherein the mutation is a replacement of one or more of amino acids in one or more framework regions or one or more complementarity determining regions with one or more chemically different amino acids.
Embodiment 21. The IgV domain of embodiment 19, wherein the mutation is a replacement of one or more framework or complementarity regions of at least one V domain with a corresponding framework or complementarity determining regions of another V domain.
Embodiment 22. A monoclonal IgG₁ IgA or IgM antibody, comprising: one or more immunoglobulin variable domains of any of claims 1 to 20 recloned within a scaffold of full-length antibodies such that the variable light domains replace the variable heavy domains within a heavy chain subunit and the variable heavy domains replace the variable light domains within the light chain subunit.
Embodiment 23. An immunoglobulin variable (IgV) domain that is designated 2E6, 5D3, 1E4, 5H3, 1B4, 1B10, 1G2, 1G5, 2B4, 2B6, 2C6, 2C7, GL2, GL59, GL1 , JL683, JL651, JL606, JL678, JL427.
Embodiment 24. An immunoglobulin variable light chain domain that is designated SK18, SK45, SK2C2, or SK2F5.
Embodiment 25. A mutant immunoglobulin variable (IgV) domain that is designated scFv GL2-FR1m, scFv GL2-FR3m, scFv GL2-CDR1m, scFv GL2-CDR2m, scFv GL2-VHm.
Embodiment 26. An isolated and purified pooled immunoglobulin preparation, comprising immunoglobulins derived from three or more human donors expressing immunoglobulins with an antigen-directed catalytic activity greater than an average catalytic activity.
Embodiment 27. The pooled immunoglobulin preparation of embodiment 26, wherein the human donor antigen-directed catalytic is greater than the average catalytic activity by 25%, 50%, 75%, 100% or greater than 100%.
Embodiment 28. The pooled immunoglobulin preparation of embodiment 26 wherein the donor catalytic activity hydrolyzes one or more peptide bonds in amyloid β peptide, HIV gp120, a Staphylococcus aureus virulence factor, or HCV E2.
Embodiment 29. An isolated and purified pooled immunoglobulin preparation, comprising immunoglobulins derived from three or more human donors expressing immunoglobulins with an HIV neutralizing activity greater than an average neutralizing activity.
Embodiment 30. The pooled immunoglobulin preparation of embodiment 29 derived from selected human donors wherein the selection procedures consists of identify individual donors expressing immunoglobulins with HIV neutralizing activity to an antigen that is greater than the average neutralizing activity by 25%, 50%, 75%, 100% or greater than 100%.
Embodiment 31. The pooled immunoglobulin preparation of embodiments 26 or 29, wherein the class of the immunoglobulins is IgA, IgM, IgG or a mixture or combination thereof.
Embodiment 32. The pooled immunoglobulin preparation of embodiments 26 or 29, wherein the immunoglobulins are isolated from blood, saliva or milk or other mucosal secretion.
Embodiment 33. A pharmaceutical composition comprising one or more of the immunoglobulins of any of embodiments 1 to 32 and an adjuvant or a diluent.
Embodiment 34. A method for prophylactically or therapeutically treating a pathophysiological condition in a patient, comprising:
   administering to the patient a pharmacologically effective amount of the pharmaceutical composition of embodiment 33.
Embodiment 35. The method of embodiment 34, wherein the pathophysiological condition is a Staphyloccus aureus infection, an HCV infection, an HIV infection or Alzheimer's disease.
Embodiment 36. The method of embodiment 34, wherein the pooled immunoglobulin preparation is administered intravenously, intranasally, topically, intraperitoneally, intravaginally, intrarectal, or intracerebrally.
Embodiment 37. A method for identifying the immunoglobulin variable (IgV) domain(s) of any of embodiments 1 to 21, comprising:
   performing a random screening of a library containing a plurality of IgV domains or fractionating the library of IgV domains using antigen electrophilic analogs.
Embodiment 38. The method of embodiment 37, wherein the electrophilic analog is an analog of amyloid β peptide, HIV gp120, one or more Staphylococcus aureus virulence factors or HCV E2.

### DESCRIPTION OF THE DRAWINGS

For a more complete understanding of the features and advantages of the present invention, reference is now made to the detailed description of the invention along with the accompanying figures and in which:

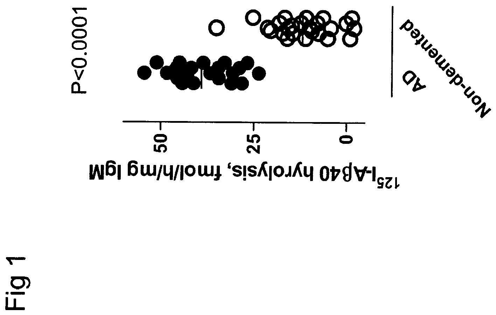

**Fig. 1** **Increased Aβ hydrolysis by IgMs from patients with Alzheimer's disease.** Shown are values of ¹²⁵I-Aβ40 (0.1 nM) hydrolysis (means of duplicates) incubated for 68 h with the IgM preparations (0.023 mg/ml) purified from AD patients (n=23) and elderly, non-demented control subjects (n=25). Hydrolytic activity was determined by separation of intact and degraded peptide by precipitation with trichloroacetic acid as in Taguchi H, et al, J Biol Chem 2008, 283, 4714. Values of Each point represents a different human subject. 2-tailed unpaired t-test.

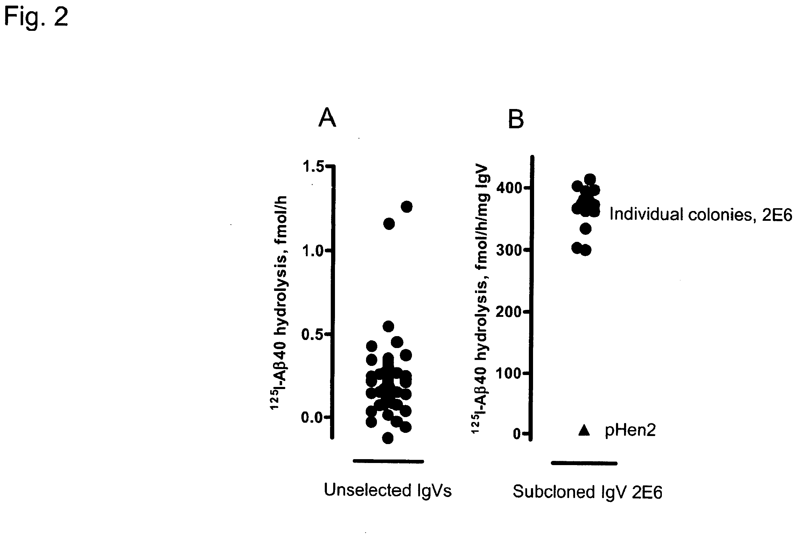

**Fig. 2** **IgV screening for ¹²⁵I-Aβ40 hydrolysis. *(A)*** Sixty three IgVs purified from randomly picked clones in our human IgV library were analyzed. Each point represents one clone. ¹²⁵I-Aβ40, 0.1 nM; 18 h. ***(B)*** Bacteria were transformed with phagemid DNA from clone 2E6, the highest activity clone in panel (A). Shown are specific activities for 15 IgV preparations purified from 15 individual colonies along with the identically purified empty vector control (pHEN2; closed triangle).

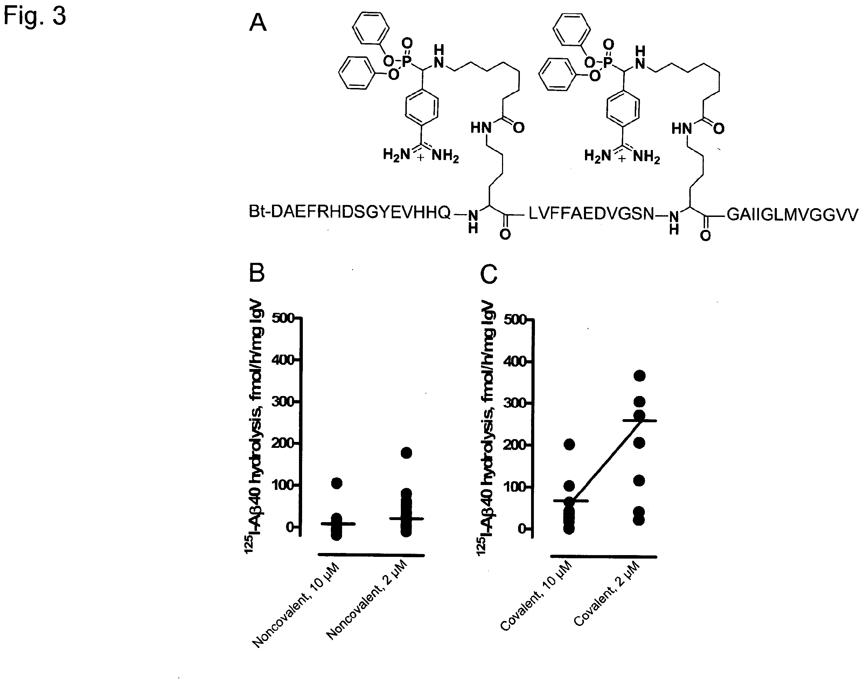

**Fig. 3** **Selection of Aβ hydrolyzing IgVs with E-Aβ40. *(A)*** Structure of E-Aβ40 used for phage selection. The electrophilic phosphonate groups placed within Aβ40 epitopes permit specific covalent binding to Igs displaying the nucleophilic reactivity coordinated with noncovalent Aβ40 recognition. ***(B)*** and ***(C),*** Phages in the IgV library (10⁷ clones) were allowed to bind biotinylated E-Aβ40 (2 or 10 µM), and bound phage particles were captured with immobilized anti-biotin antibodies. Phages associated noncovalently with E-Aβ40 were retrieved by elution with excess nonbiotinylated Aβ40 (100 µM; noncovalent selection). Covalently bound phages were retrived by elution with an acidic buffer (pH 2.7; covalent selection). Hydrolysis of ¹²⁵I-Aβ40 (0.1 nM) by IgVs obtained from noncovalently and covalently selected phages is shown in panels B and C, respectively. Each point represents an individual IgV clone.

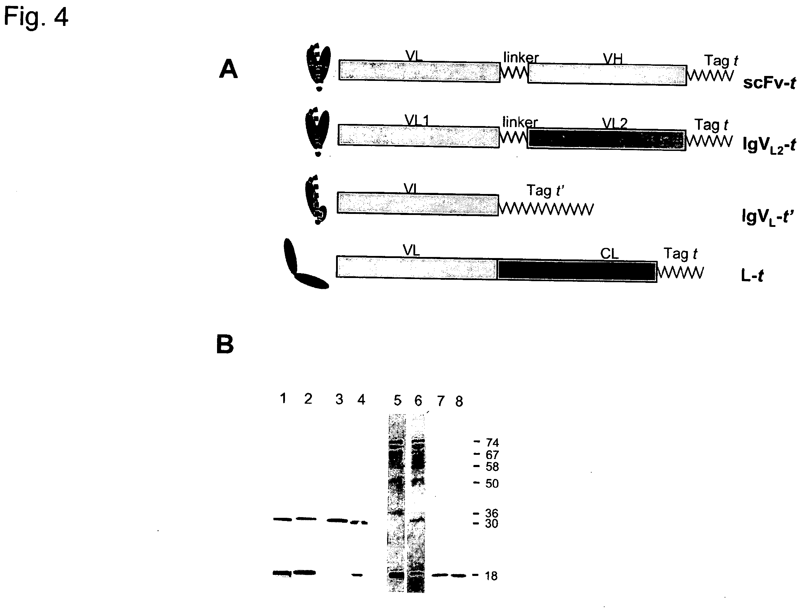

**Fig. 4** **Schematic representations *(A)* and purity of Aβ-hydrotyzing IgVs *(B)*.** scFv-*t*, VL domain and VH domains connected by a peptide linker; *t*, His6/c-myc tag located at the C-terminus; IgV_{L2}-*t*, heterodimeric construct composed of two different VL domains; IgV_{L}-*t'*, the single domain IgV containing a VL domain. *t'* corresponds to the linker, an aberrant polypeptide in place of the VH domain at the C terminus of the linker and the His6-c-myc tag; L-*t*, full-length light chain containing the VL domain, the light chain constant domain (κ) and the terminal tag *t.* In *(B),* lanes 1 and 2, respectively, are silver stained and anti-c-myc antibody stained gels of IgV_{L2}-*t* 2E6 after two cycles of metal affinity chromatography. Lanes 3 and 4, respectively, are silver stained and anti-c-myc antibody stained gels of IgV_{L2}-*t* 2E6 after further chromatography on the FPLC Mono Q column (fractions corresponding to retention times 10-11 min). Lanes 5 and 6, respectively, are silver stained and anti-c-myc antibody stained gels of IgV_{L}-*t'* 5D3 after two cycles of metal affinity chromatography. Lanes 7 and 8, respectively, are silver stained and anti-c-myc antibody stained gels of IgV_{L}-*t'* 5D3 after further chromatography on the Mono Q column (fractions corresponding to retention times 23-23.5 min).

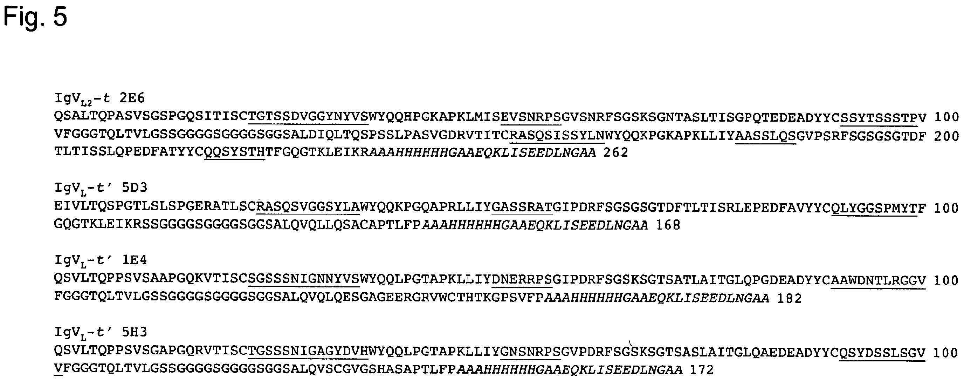

**Fig. 5** **Deduced IgV amino acid sequences.** VH and VL CDRs are underlined. The linker is shadowed. Tag *t*, the his6-c-myc tag is in italics. The polypeptide at the C terminus of the VL domain in IgV_{L}-*t'* clone 5D3, 1E4 and 5H3 is designated tag *t'*. Tag *t'* in each of these clones is composed of the expected N terminal 17 residue linker, an aberrant 15-28 residue intervening peptide sequence and the expected C terminal 26 residue his6-c-myc tag *t*. The aberrant peptide sequences in the IgV_{L}-*t'* clones are composed of: clone 5D3, VH FR1 residues 1-7 linked to CH1 residues 116-123; clone 1E4, VH FR1 residues 1-9, an unidentified 17 residue peptide and CH1 residues 122-123; clone 5H3, VH FR1 residues 1-2, an unidentified 9 residue peptide and CH1 residues 118-123. The unidentified peptide regions were not derived from any known protein as determined by Blast homology searches.

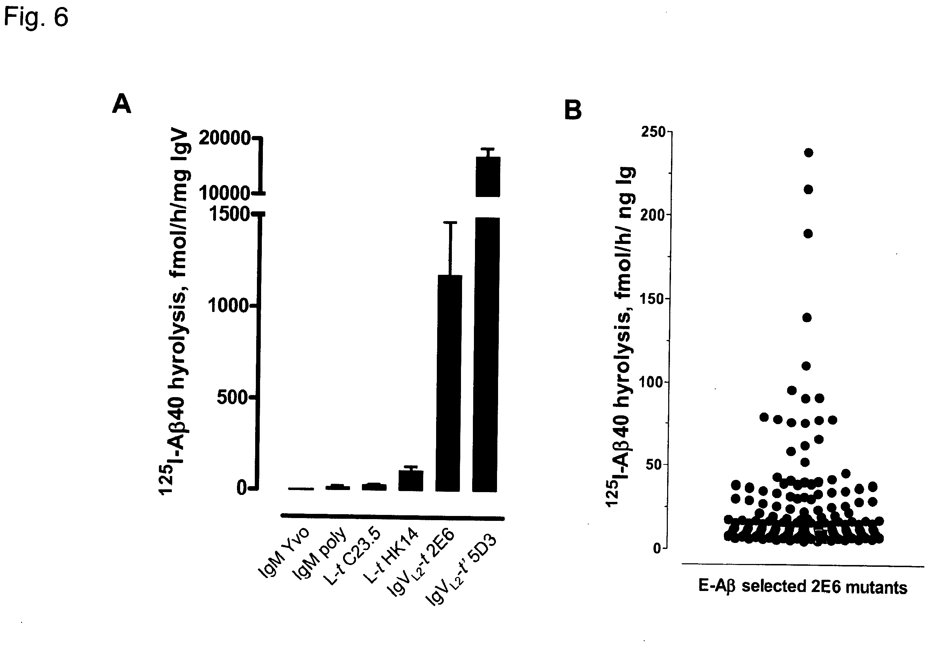

**Fig. 6 (A)** **Exceptional catalytic activity of IgV_{L2}-*t* 2E6 and IgV_{L}-*t'* 5D3 compared to previously known Aβ hydrolyzing IgMs and Ig light chain subunits.** Shown are the Aβ40 hydrolysis activity (fmol/h/mg Ig; means of duplicates) of monoclonal IgM Yvo (405 µg/ml), a pooled polyclonal IgM preparation (90 µg/ml), L-*t* C23.5 (15 µg/ml), L-*t* HK14 (3.4 µg/ml), IgV_{L2}-*t* 2E6 (0.55µg/ml) and IgV_{L}-*t'* 5D3 (0.075 µg/ml). ¹²⁵I-Aβ40, ∼30000 cpm (∼0.1 nM). The monoclonal and polyclonal IgM preparations are described in Taguchi H, et al, J Biol Chem 2008, 283, 4714; L-*t* HK14 is described in Liu R, et al, Biochemistry 2004, 43, 9999. **(B) Isolation of catalytically improved IgV_{L2}-*t* 2E6 mutants.** A random mutant library containing ∼4 amino acid mutations/IgV_{L2}-*t* molecule was generated by error-prone mutagenesis. Following covalent selection of mutants expressed on phages using E-Aβ, secreted IgV_{L2}-*t* mutants in culture supernatants of individual bacterial colonies (1:75 diluted) were screened for Aβ hydrolysis. Values are corrected for background activity observed in supernatants from empty vector control colonies. Ig concentrations were estimated by c-myc dot blots. Shown are values of ¹²⁵I-Aβ40 (0.1 nM) hydrolysis (means of duplicates) incubated (18 h) with culture supernatants of 143 clones obtained by covalent selection. The red data point corresponds to wildtype IgV_{L2}-*t* 2E6 hydrolytic activity.

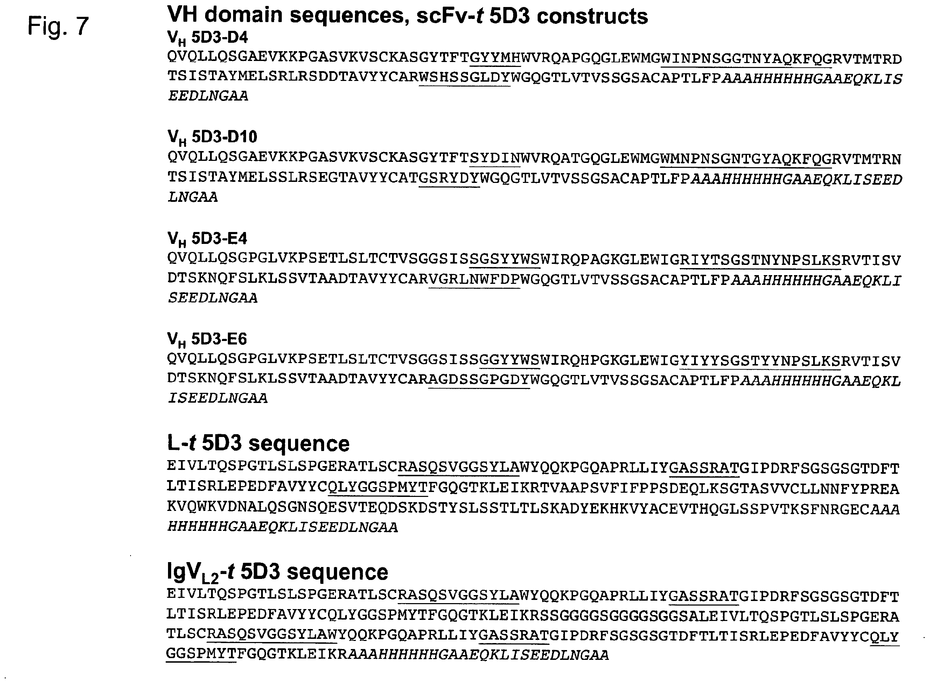

**Fig. 7** **Deduced amino acid sequences of various derivatives of IgV_{L}-*t'* 5D3, i.e., the repaired scFv-*t*, full-length L-*t* and homodimeric IgV_{L2}-*t*.** For the 4 scFv-*t* constructs, only VH domain sequences are shown. The scFv-*t* constructs contain an identical VL domain derived from IgV_{L}-*t'* 5D3 (see sequence in Fig 5). CDRs are underlined, linker is shadowed and the His6-c-myc tag is in italics.

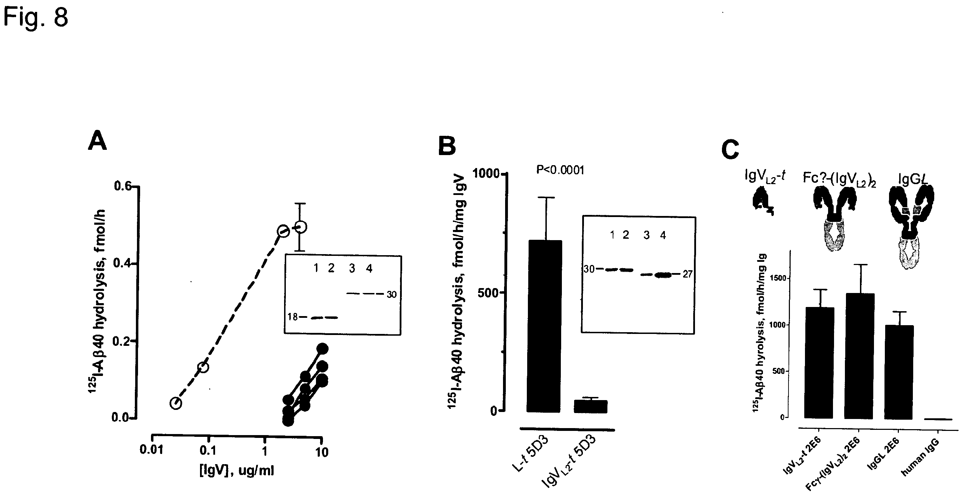

**Fig. 8** **¹²⁵I-Aβ40 hydrolyzing activity of various derivatives of IgV_{L}-*t'* 5D3, i.e., the repaired scFv-*t*, full-length L-*t* and homodimeric IgV_{L2}-*t*. *(A)*** ¹²⁵I-Aβ40 (∼30000 cpm, -0.1 nM) incubated in duplicate for 18 h with IgV_{L}-*t'* 5D3 (0.075-1.9 µg/ml) and four scFv-*t* 5D3 variants (2.5-10 µg/ml). *Inset,* SDS-PAGE gels showing purified IgV_{L}-*t'* (lane 1) and scFv-*t* (lane 3) stained with silver. Lanes 2 and 4 show, respectively, the IgV_{L}-*t'* and scFv-*t* stained with anti c-myc antibody. The bands at 18 kDa and 30 kDa represent, respectively, the IgV_{L}-*t'* and scFv-*t*. ***(B)*** Shown are ¹²⁵I-Aβ40 hydrolysis activities of L-*t* 5D3 and IgV_{L2}-*t* 5D3 assayed as in Panel A (means of 3 independent assays). *Inset,* SDS-PAGE gels showing purified L-*t* (lane 1) and IgV_{L2}-*t* (lane 3) stained with silver. Lane 2 and 4 show, respectively, the L-*t* and IgV_{L2}-*t* stained with anti c-myc antibody. The bands at 30 kDa and 27 kDa represent, respectively, the L-*t* and IgV_{L2}-*t*. **(C) IgG*L* and Fcγ1-(V_{L2})₂ versions of clone 2E6. *Inset,*** Schematic representations of the starting IgV_{L2}-*t* structure and its IgG*L* and Fcγ1-(V_{L2})₂ versions; No extraneous polypeptide tags are present in the IgG*L* or Fcγ1-(V_{L2})₂. Aβ hydrolysis by IgG*L* and Fcγ1-(V_{L2})₂ versions of clone 2E6. IgG*L* and Fcγ1-(IgV_{L2})₂ purified by Protein G-Sepharose chromatography from culture supernatants of NS0 cells transfected with the appropriate heavy and light chain expressing vectors. Identically purified control IgG from human serum was without activity.

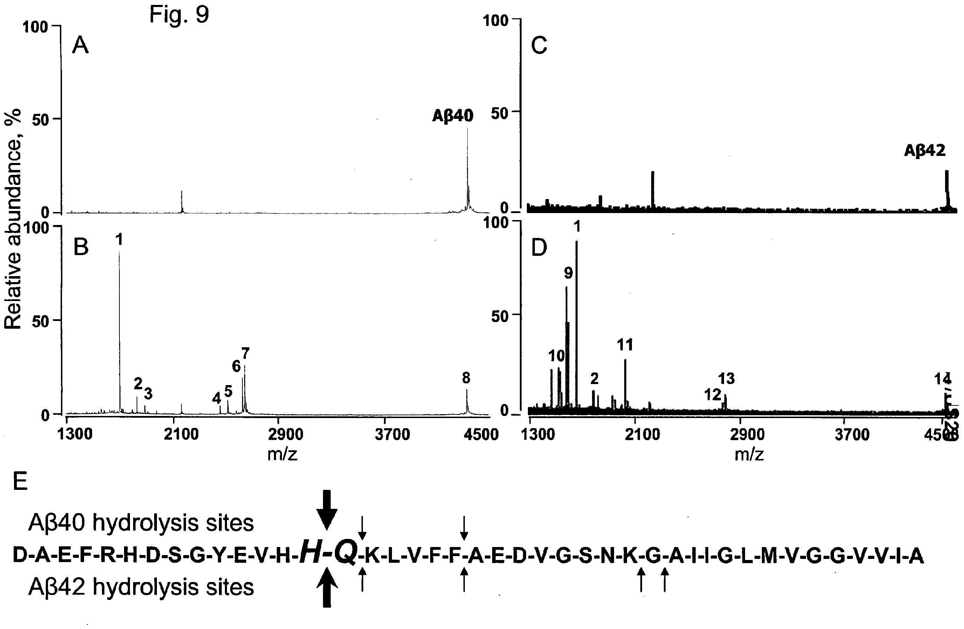

**Fig. 9** **Aβ40 and Aβ42 peptide bonds hydrolyzed by IgV_{L2}-*t* 2E6.** MALDI-TOF mass spectra of Aβ40 incubated for 89 h in diluent (89 h) ***(A)*** or with IgV_{L2}-*t* 2E6; ***(B)*** and Aβ42 incubated similarly in diluents ***(C)*** or IgV_{L2}-*t* 2E6 ***(D).*** Aβ40 or Aβ42, 100 µM; IgV_{L2}-*t* 2E6, 1 µM. Spectra were collected using α-cyano-4-hydroxy cinnamic acid as matrix (positive ion mode, 20,000 volts). Peak numbers in the spectra indicate: **1,** Aβ 1-14 (calculated (M+H)⁺ = 1698.7, observed (M+H)⁺ = 1698.8); **2,** Aβ 1-15 (calculated (M+H)⁺ = 1826.8, observed (M+H)⁺ = 1826.9); **3,** Aβ 21-40 (calculated (M+H)⁺ = 1886.0, observed (M+H)⁺ = 1886.1); **4,** Aβ 1-20 (calculated (M+H)⁺ = 2461.2, observed (M+H)⁺ = 2461.2); **5,** Aβ 16-40 (calculated (M+H)⁺ = 2520.4, observed (M+H)⁺ = 2520.4); **6,** pGluAβ 15-40 (calculated (M+H)⁺ = 2631.4, observed (M+H)⁺ = 2631.5); **7,** Aβ 15-40 (calculated (M+H)⁺ = 2648.5, observed (M+H)⁺ = 2648.5); **8,** Aβ40 (calculated (M+H)⁺ = 4328.2, observed (M+H)⁺ = 4328.1); **9,** Aβ 15-29 (calculated (M+H)⁺ = 1638.9, observed (M+H)⁺ = 1638.9); **10,** Aβ 15-28 (calculated (M+H)⁺ = 1581.8, observed (M+H)⁺ = 1581.8); **11,** Aβ 21-42 (calculated (M+H)⁺ = 2070.1, observed (M+H)⁺ = 2070.2); **12,** pGluAβ 15-42 (calculated (M+H)⁺ = 2814.6, observed (M+H)⁺ = 2814.8); **13,** Aβ 15-42 (calculated (M+H)⁺ = 2832.6, observed (M+H)⁺ = 2832.6); **14,** Aβ42 (calculated (M+H)⁺ = 4512.3, observed (M+H)⁺ = 4512.1). The hydrolytic sites in Aβ40 and 42 are shown with arrows in ***(E).***

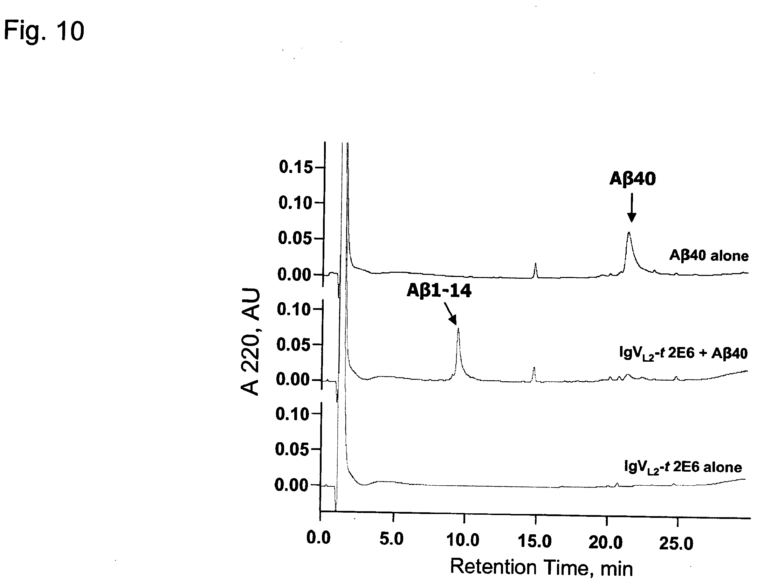

**Fig. 10** **HPLC detection of Aβ40 fragments generated by IgV_{L2}-*t* 2E6.** Aβ40 (100 µM) incubated with IgV_{L2}-*t* 2E6 (40 µg/ml) for 72 h (middle chromatogram). Top and bottom chromatograms show, respectively, the control IgV_{L2}-*t* 2E6 alone (40 µg/ml) and the control Aβ40 alone (100 µM) incubated in diluent. The peptide peaks were identified by ESI-mass spectroscopy as in Taguchi H, et al, J Biol Chem 2008, 283, 4714.

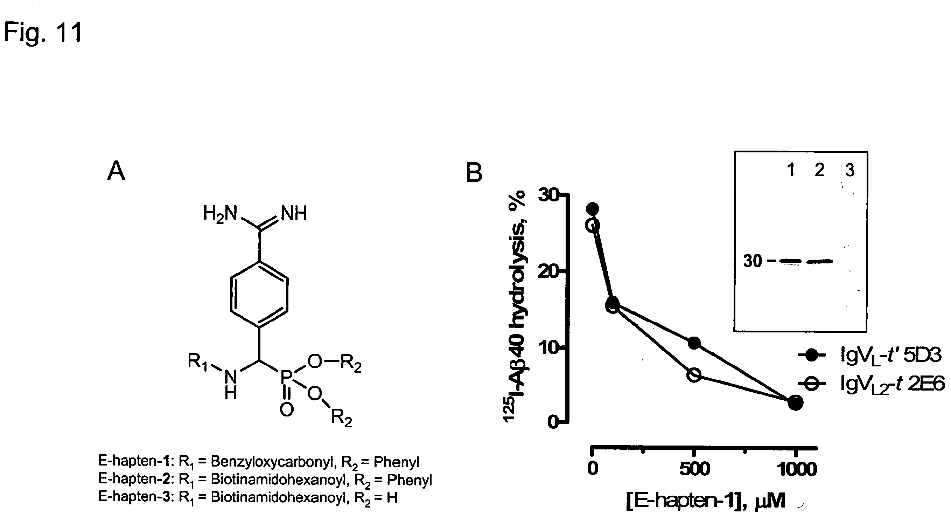

**Fig. 11** **Inhibition of IgV hydrolytic activity by E-hapten-1. *(A)*** E-hapten-**1** is an active site-directed inhibitor of trypsin-like serine proteases. E-hapten-**2** is a biotinylated analog of E-hapten-**1** employed to detect covalent IgV adducts by streptavidin-peroxidase staining of SDS-electrophoresis gels. E-hapten-**3** is the unesterified phosphonic acid analog of E-hapten-**1** devoid of covalent reactivity. ***(B)*** Shown is inhibition of IgVL2-*t* 2E6 and IgV_{L}-*t'* catalyzed ¹²⁵I-Aβ40 hydrolysis by E-hapten**-1**. IgV_{L2}-*t* 2E6 (0.55 µg/ml) or IgV_{L}-*t'* 5D3 (0.075 µg/ml) was preincubated with E-hapten-**1** (0.01, 0.5 and 1 mM) for 2 h with followed by determination of 125I-Aβ hydrolytic activity. *Inset,* Streptavidin-peroxidase stained blots of SDS electrophoresis gels showing IgV_{L2}-*t* 2E6 (20 µg/ml) incubated for 18 h with E-hapten **2** (0.1 mM, lane 2) or E-hapten-**3** (lane 3). Lane 1, silver-stained SDS-gel of IgV_{L2}-*t* 2E6

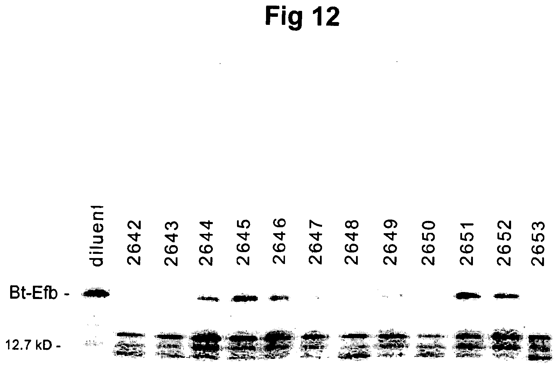

**Fig 12****. Efb hydrolysis by IgG.** Shown is a streptavidin-peroxidase-stained blot of a reducing SDS-electrophoresis gel showing cleavage of biotinylated Efb (0.1 µM) by affinity-purified IgG from 12 healthy adult subjects (0.35 µM) after a 20 h incubation (reaction volume 20 µl). Diluent lane represents biotinylated Efb incubated in the absence of IgG under identical conditions.

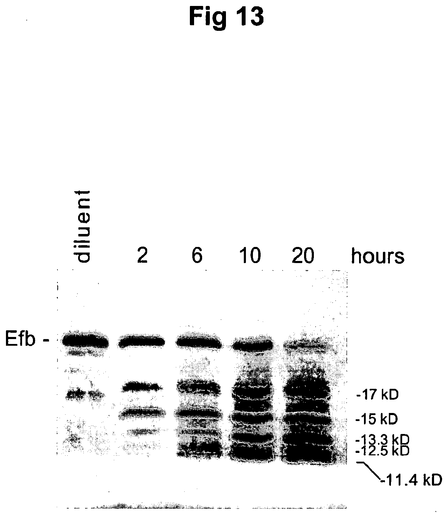

**Fig 13****. Time-dependent cleavage of biotinylated Efb by pooled human IgG.** Shown is a silver stained SDS-electrophoresis gel (15%; run under reducing conditions) demonstrating Efb (0.1 µM) cleavage by affinity purified IgG (45 µg/ml). Product masses are indicated on the right. Diluent lane, Efb incubated in the absence of IgG.

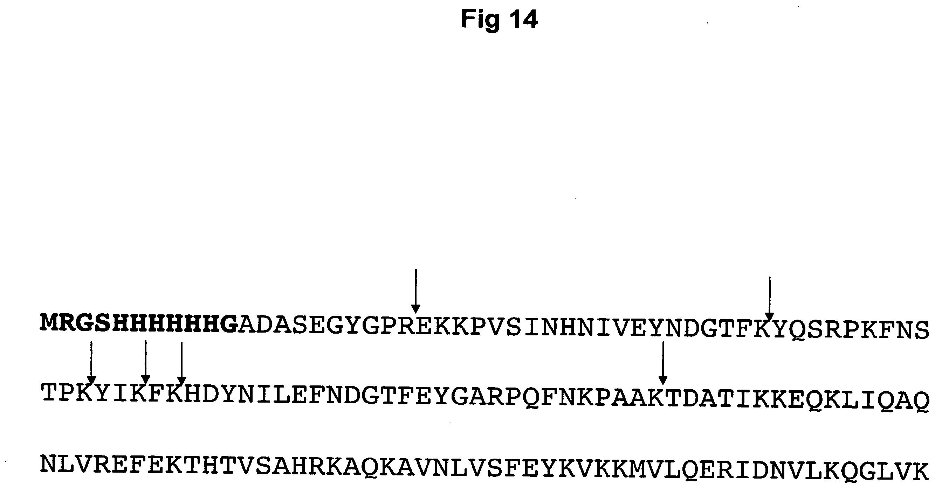

**Fig 14****. Identification of peptide bonds cleaved by pooled polyclonal IgG.** Efb (12 µg/ml) was incubated for 20 h with pooled polyclonal serum IgG (N=12 humans, 57 µg/ml). The reaction mixture was subjected to reducing SDS-PAGE (20% gels) and transferred onto a PVDF membrane. Coomassie stained product bands were sequenced by Edman's degradation. Shown is the Efb amino acid sequence with scissile bonds indicated by arrows. Bold region represents the N-terminal His tag sequence.

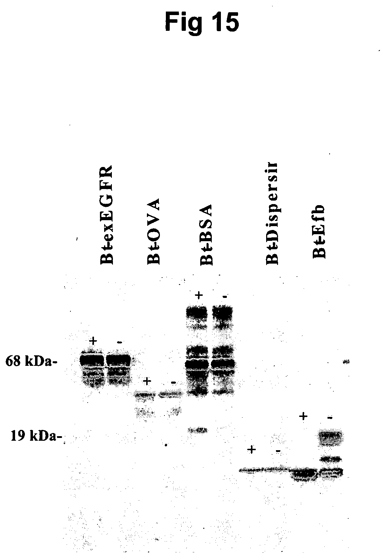

**Fig 15****. Bt-Efb is cleaved selectively by IgG.** Shown is a streptavidin-peroxidase stained blot of a reducing SDS-electrophoresis gel of biotinylated extracellular domain of epidermal growth factor receptor (Bt-exEGFR; 0.25 µM), ovalbumin (Bt-OVA; 0.25 µM), bovine serum albumin (BSA), Dispersin (0.25 µM) and Efb (1.1 µM) incubated in the presence (+) or absence (-) of pooled IgG (0.5 µM) for 20 h.

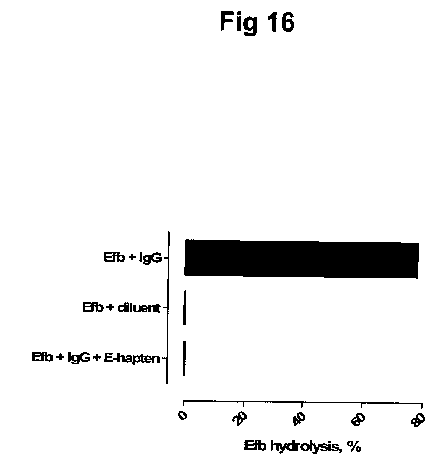

**Fig 16****. Serine-protease inhibitor blocks IgG-mediated catalytic activity.** Efb (0.7 µM) was incubated with pooled polyclonal IgG (1 µM) in the presence and absence of the serine protease inhibitor E-hapten **2** (shown **in** **Fig 11A****;** 100 µM).

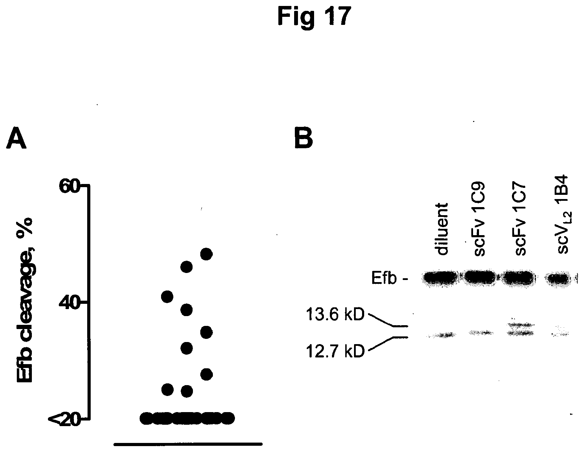

**Fig 17****. Identification of Efb-hydrolyzing antibody fragments. *(A)*** Bt-Efb (0.1 µM) was incubated in the presence of purified antibody fragments at 37°C for 20 h. Shown are %Bt-Efb cleavage determined as %decrease of Bt-Efb band intensity compared to the diluent control. ***(B)*** Shown are streptavidin-peroxidase-stained blots of reducing SDS-PAGE gels demonstrating cleavage of Bt-Efb following incubation with 2 of 46 clones tested (scFv-*t* 1C7 and scV_{L2}-*t* 1B4). The scFv-*t* 1C9 clone is a representative non-catalytic clone. Reaction volume, 0.02 ml. Diluent lane, Efb incubated in the absence of antibody fragment.

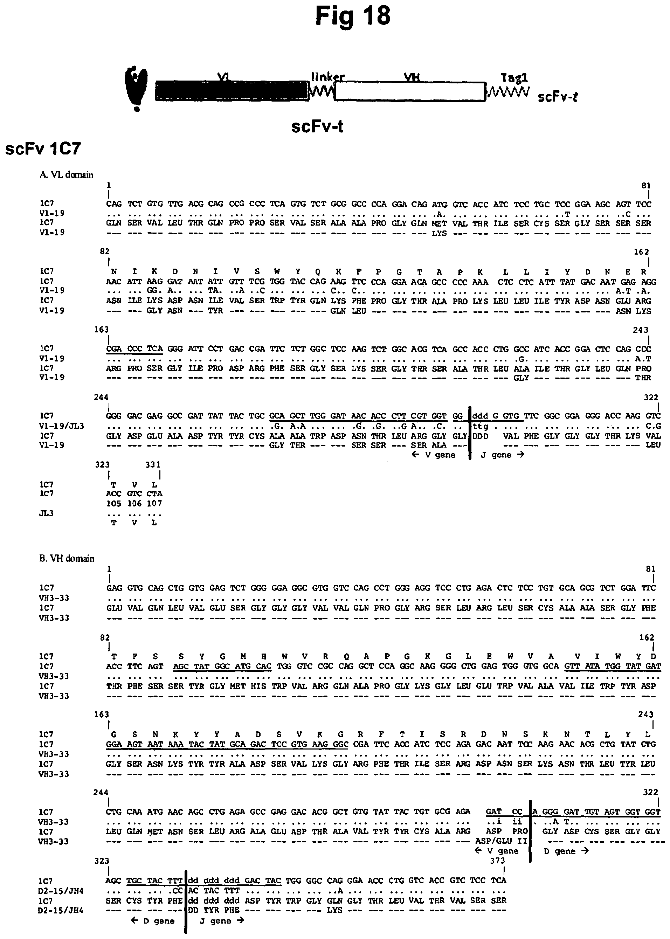

**Fig 18****. Nucleotide and amino acid sequences of scFv-*t* 1C9.** *Illustration:* Schematic representation of the domain organization of scFV-t 1C9; V_{L} and V_{H} domains are linked by the peptide linker, and the tag containing a His6 motif and c-myc peptide is located at the C-terminus of the molecule. In sequences, "•" and "-" indicate, respectively, a 1C9 nucleotide and amino acid identical to the corresponding position of the closest germline sequence.

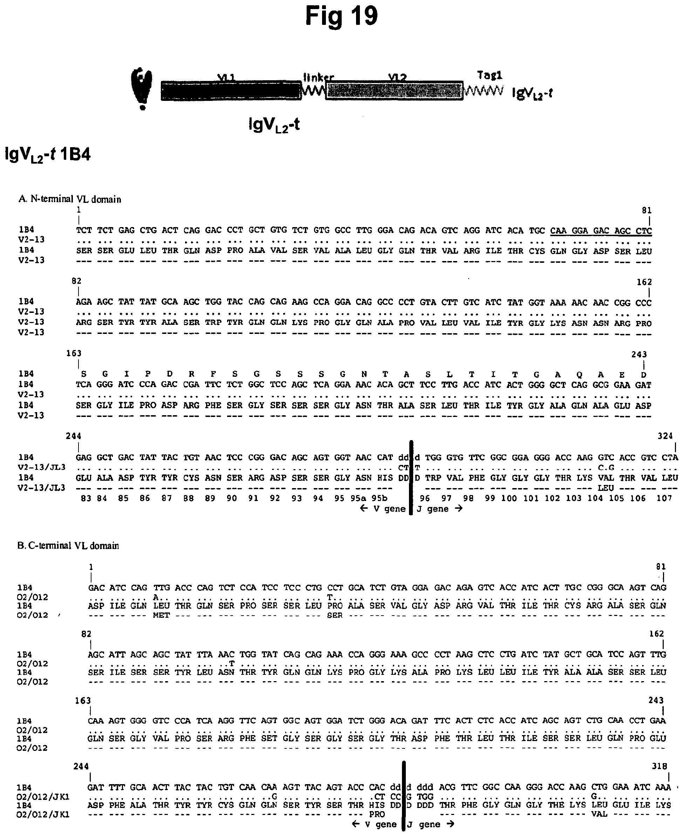

**Fig 19****. Nucleotide and amino acid sequences of IgV_{L2}-*t* 1B4.** *Illustration:* Schematic representation of the domain organization of IgV_{L2}-*t* 1B4; Two different V_{L} domains are linked by the peptide linker, and the tag containing a His6 motif and c-myc peptide is located at the C-terminus of the molecule. In sequences, "•" and "-" indicate, respectively, a 1B4 nucleotide and amino acid identical to the corresponding position of the closest germline sequence.

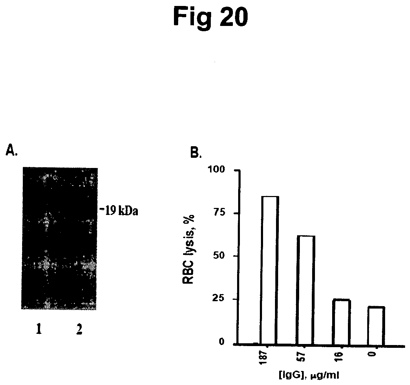

**Fig 20****. Effect of catalytic IgG on C3b binding and Efb-mediated complement inhibition. (A)** Western-C3b ligand blot analysis of CIg-treated Efb. Pooled IgG from twelve healthy adult subjects was incubated with Efb and subjected to SDS-PAGE (10% tricine gel), transferred onto nitrocellulose paper and probed with digoxigenin-labeled C3b as described, followed by a second incubation with alkaline phosphatase-labeled anti-digoxigenin Fab fragments. **(B)** Efb (0.1 µM) was preincubated in absence or presence of varying concentrations of pooled IgG for 20 h at 37°C in 50 mM Tris•HCl, 100 mM glycine, 0.1 mM CHAPS, pH 8.0, containing 67 µg/ml gelatin. Reaction volume, 20 µl. The "high reference serum" (5 µl) provided as part of the CH50 complement assay (Diamedix) was added to each Efb-IgG mixture for 1 h. Positive control was 5 µl of high reference serum added to 20 µl PBS and preincubated as above. Each reaction mixture was subsequently added to tubes containing antibody-coated RBCs, vortexted and incubated at room temperature for 1 h as instructed by manufacturer. A tube with no high reference serum served as a negative control. RBC lysis via the classical pathway was quantified by measuring absorbance (A₄₁₅) after the tubes were centrifuged. The data are plotted relative to the observed lysis in the positive control "high reference serum" tubes (no Efb, no IgG; 100% value) using the equation 100 x (A₄₁₅ of Sample (Efb alone or Efb+IgG)/ A₄₁₅ of positive control.

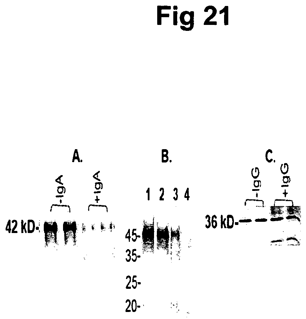

**Fig 21****. Hydrolysis of Bt-SdrE, Bt-I-Protein A, and ClfA by human Ig. (A)** Bt-SdrE (0.1 µM) was incubated in the presence of purified pooled IgA (1 µM, 15 h) from healthy adult volunteers. **(B)** Bt-I-Protein A (0.1 µM, 17 hours) was incubated with increasing concentrations of human serum IgA pooled from healthy individuals (Lane 1, 0 µg/ml; Lane 2, 32 µg/ml; Lane 3, 160 µg/ml; Lane 4, 600 µg/ml). Reaction mixtures were then subjected to SDS-PAGE electrophoresis visualized by streptavidin-peroxidase staining. **(C)** ClfA (0.1 µM) was incubated in the presence of 1 µM pooled IgG from healthy individuals for 15 h and then run on a 15% tricine gel and silver stained.

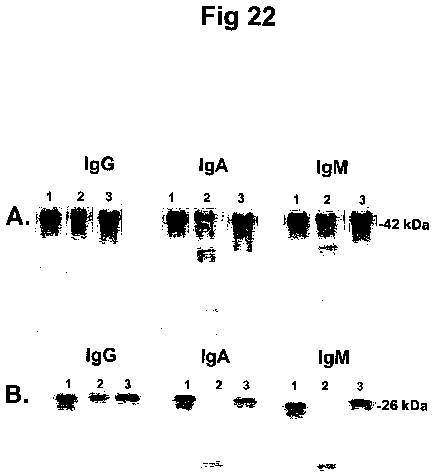

**Fig 22****. Hydrolysis of (A) Bt-SdrE and (B) Bt-Mapl9.** Bt-proteins (0.1 µM) were incubated 15 h at 37°C in diluent (Lane 1) or Igs (1 µM) purified from pediatric subjects without (Lane 2), or with deep-bone *S. aureus* infections (Lane 3). Reaction mixtures were then subjected to SDS-PAGE electrophoresis and Bt-proteins were visualized by streptavidin-peroxidase staining.

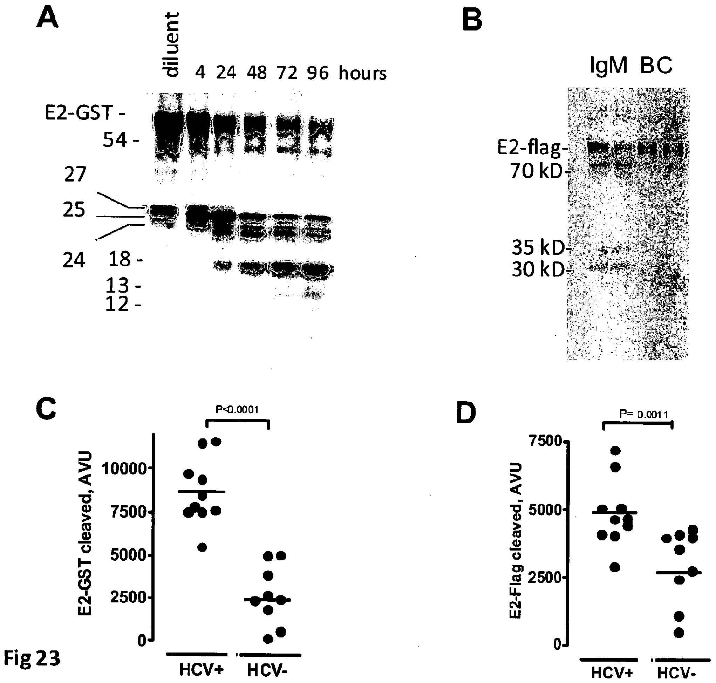

**Fig 23****. Hydrolysis of E2 by IgM from HCV infected humans. (A) Time-dependent degradation of E2-GST by IgM.** E2-GST (10 µg/mL) was incubated with a pooled IgM preparation (25 µg/mL) from sera of HCV infected subjects. Shown are anti-GST-peroxidase stained blots of SDS-gels revealing the reduction of the intact E2-GST band intensity as a function of incubation time. Multiple cleavage products were detected as anti-GST stainable bands with mass values smaller than the 70kD E2-GST band. **(B) E2-Flag hydrolysis by IgM.** Shown are anti-E2 stained blots of SDS-gel of E2-Flag incubated as in panel A in the presence (left two lanes) or absence (right two lanes) of IgM. **(C) E2-GST cleaving activity of IgM from humans with and without HCV infection.** E2-GST incubated with IgM as in panel A (48 h) was subjected to SDS-electrophoresis and anti-GST staining as in panel A. E2-GST hydrolysis was quantified by measuring the reduction of the intact E2-GST band by densitometry in reference to the diluent control. Each point represents one study subject. **(D) E2-flag cleaving activity of IgM from humans with and without HCV infection.** E2-flag was incubated with IgM and E2-flag cleavage determined as in panel C. Each point represents one study subject.

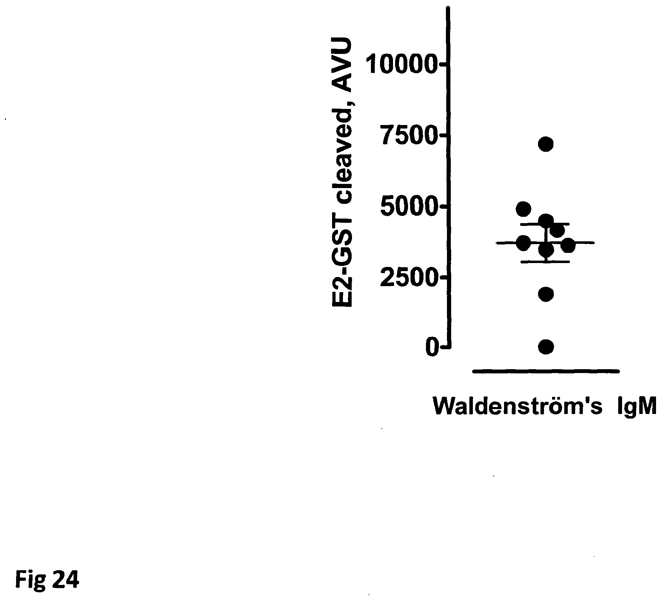

**Fig 24****. Hydrolysis of E2-GST by monoclonal IgM preparations.** E2-GST (75.5 µg/mL) incubated with Waldenstrom's IgM (75.5 µg/mL) for 48 h was subjected to SDS-electrophoresis, and E2-GST hydrolysis was quantified as in **Fig 23****.** Each point represents one study subject.

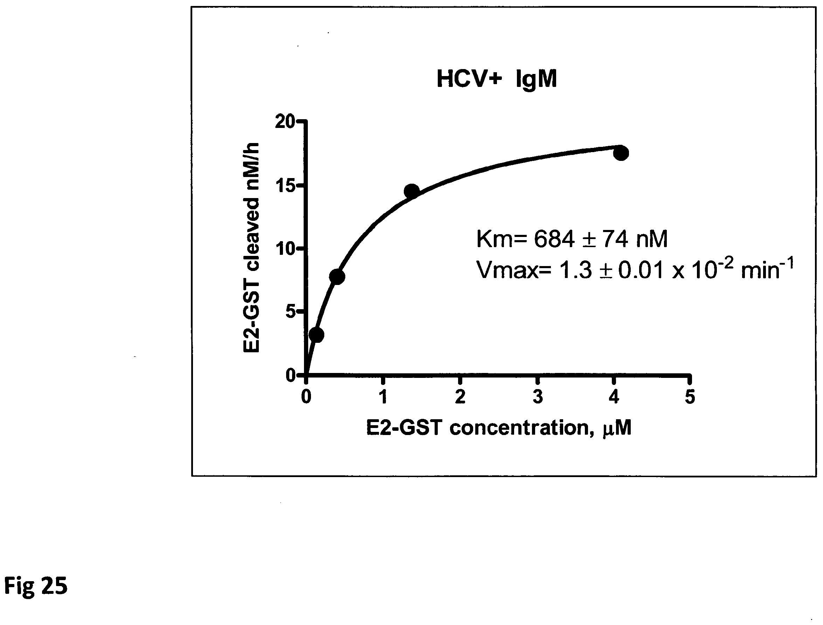

**Fig 25****. Apparent kinetic parameters for E2-GST hydrolysis by IgM from HCV infected humans.** A pooled IgM preparation from HCV infected humans (n= 10; 25 µg/mL) was incubated with increasing concentrations of E2-GST (0.14-4.1 µM), and E2-GST hydrolysis was determined as described in **Fig 23****.** Km and Vmax values were obtained from the least-square-fit of the velocity (V) vs E2-GST concentration data to the Michaelis-Menten equation, V = Vmax•[E2-GST]/(Km + [E2-GST]) (r² 0.992); Vmax, [E2-GST], and Km denote, respectively, the maximum velocity, initial concentration of E2-GST, and Michaelis constant.

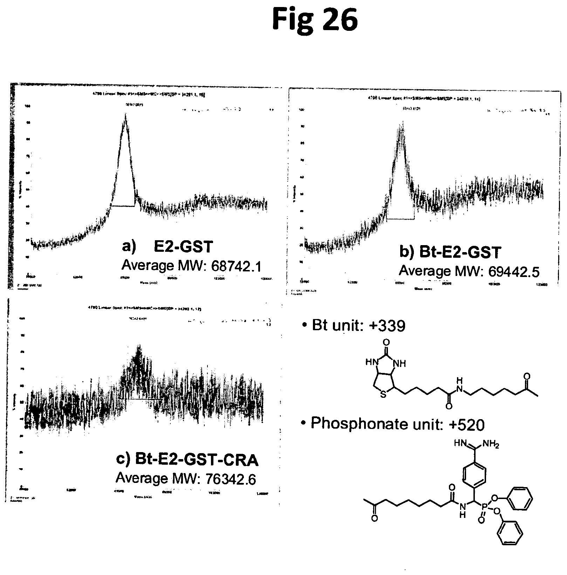

**Fig 26****. MALDI-TOF mass spectra of E2-GST (a), biotinylated E2-GST (b), and E2-E-GST (c).** Also shown are structures and expected mass increments for biotinamidohexanoyl (Bt unit) and phosphonate groups.

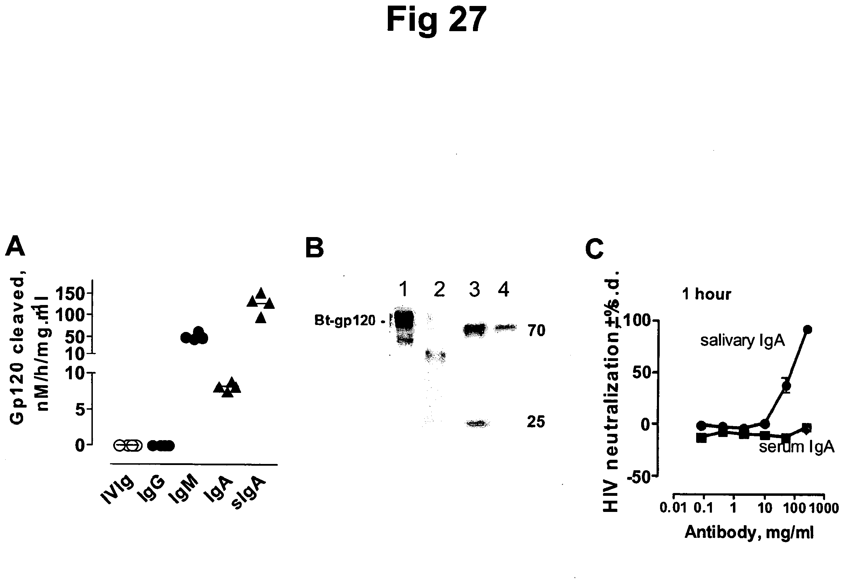

**Fig 27****. *(A)*** i**gp120 hydrolysis by IgAs from HIV negative subjects.** Plot shows proteolytic activity per unit time and per unit Ig mass. Shown are data from serum Igs and salivary IgA (sIgA) from 4 humans and 4 commercial IVIG preparations. ***(B)* Streptavidin-peroxidase stained reducing SDS-gels showing biotinylated gp120 treated with diluent (lane 1) or salivary IgA (lane 2).** Lanes 3 and 4 show, respectively, covalent adducts of E-421-433 and E-hapten formed by a monoclonal human IgM, code 1801. ***(C)* Neutralizing potency of IgA and IgG Abs purified from pooled serum or saliva.** HIV-1 strain, 97ZA009; host cells, PBMCs. Values are relative to p24 concentrations in test cultures receiving diluent instead of Igs. Serum IgA showed neutralizing activity after 24 h incubation with the virus (not shown).

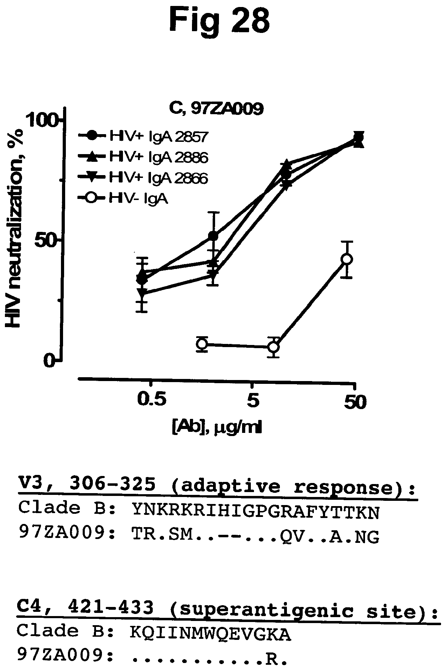

**Fig 28****. Heterologous HIV strain neutralization by serum IgA from presumptive clade B infected S₁₈ subjects. *Top,*** Neutralization of clade C strain ZA009 (R5 coreceptor) by IgA from 3 S₁₈ subjects. IgA incubated 1b with HIV before assaying infectivity using human PBMCs. Means of 4 replicates. ***Bottom,*** Consensus V3 region 306-325 and 421-433 region sequences compared to corresponding strain ZA009 sequences. Dot, identity; dash, gap.

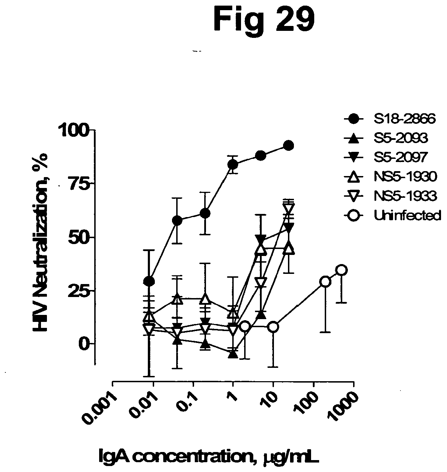

**Fig 29****. Superior neutralizing potency of serum IgA from a S₁₈ subject.** Neutralization of clade C strain ZA009 (R5 coreceptor) by IgA isolated from sera of an uninfected subjects, an S₁₈ subject (i.d. 2866), two S₅ subjects (i.d. 2093 and 2097) and two NS₅ subjects (i.d. 1930 and 1933). HIV (100 TCID₅₀) was incubated with IgA for 1h, then allowed to infect phytohemagglutinin-stimulated human PBMCs. Values are expressed as percent reduction of p24 concentrations in test cultures compared to cultures that received diluent instead of IgA (means ± s.d. of 4 replicates).

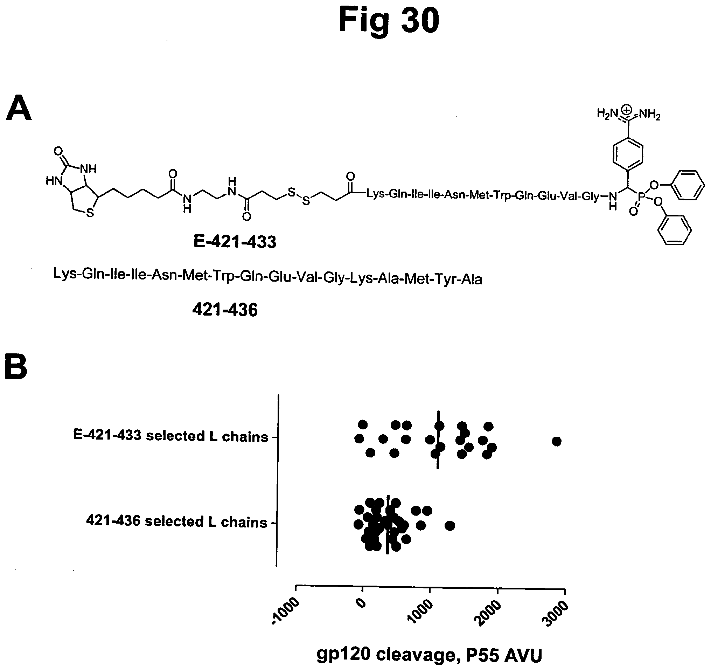

**Fig 30****. Isolation of nucleophilic antibody L chain subunits that recognize gp120 residues 421-433 from a phage-displayed human L chain library.** (A) E421-433 and 421-436 structures. ***(B)*** gp120 hydrolyzing activity of L chains selected using E-421-433 or 421-436. For selection methods, see text. The activity was measured using biotinylated gp120 (100 nM) as substrate and expressed as the intensity of the 55kD product band intensity (P55; in arbitrary volume unit, AVU) determined by densitometry of streptavidin-peroxidase stained blots of SDS-electrophoresis gels.

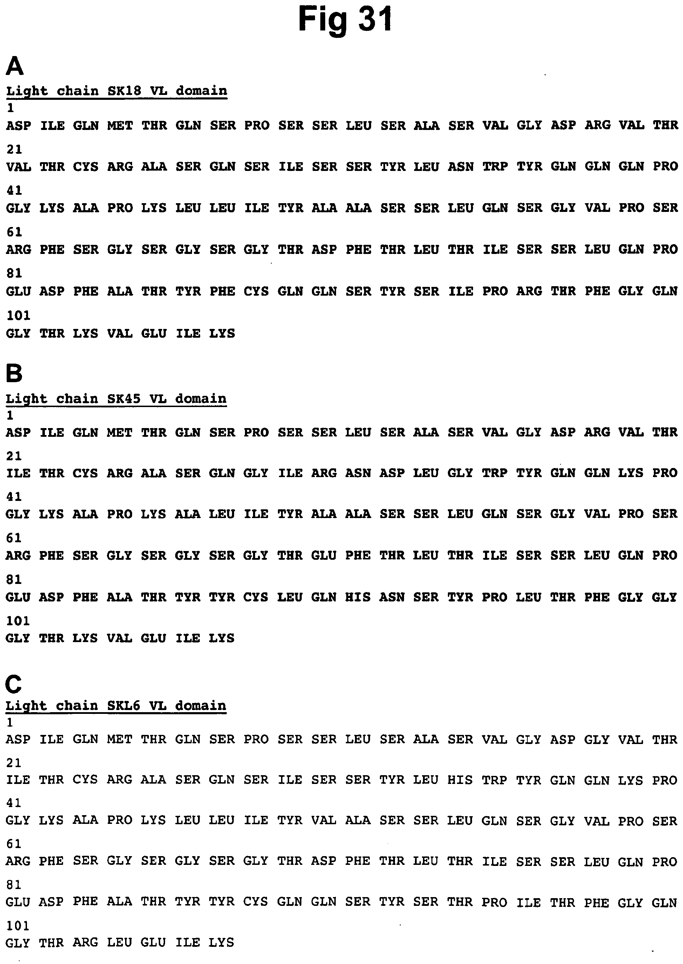

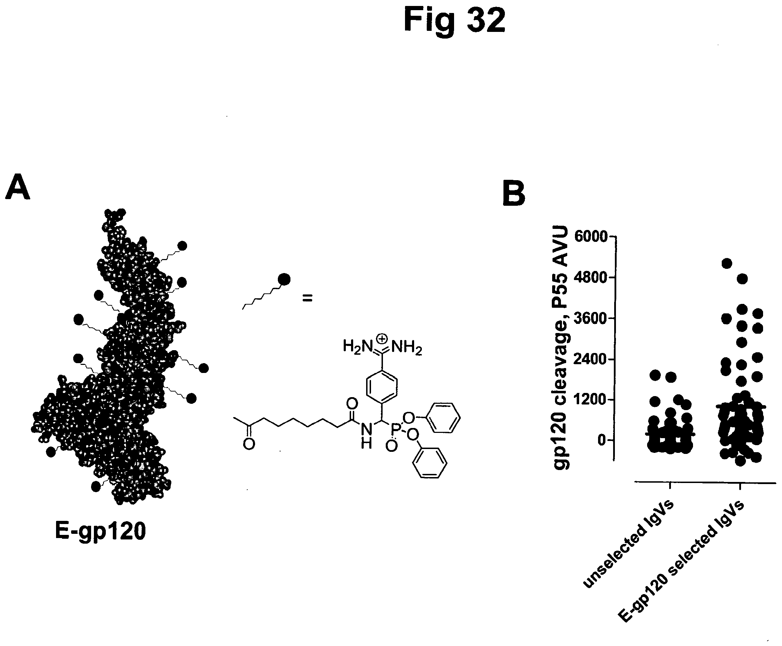

**Fig 31****. Amino acid sequences of VL domains of L chains SK18, SK45 and SKL6.** **Fig 32****. Isolation of nucleophilic antibody IgVs by covalent phage selection with E-gp120. *(A)*** E-gp120 structure. ***(B)*** gp120 hydrolyzing activity of IgVs. For selection methods, see text. The activity was measured using biotinylated gp120 (100 nM) as substrate and expressed as the 55 kD product band intensity (P55; in arbitrary volume unit, AVU) determined by densitometry of streptavidin-peroxidase stained blots of SDS-electrophoresis gels.

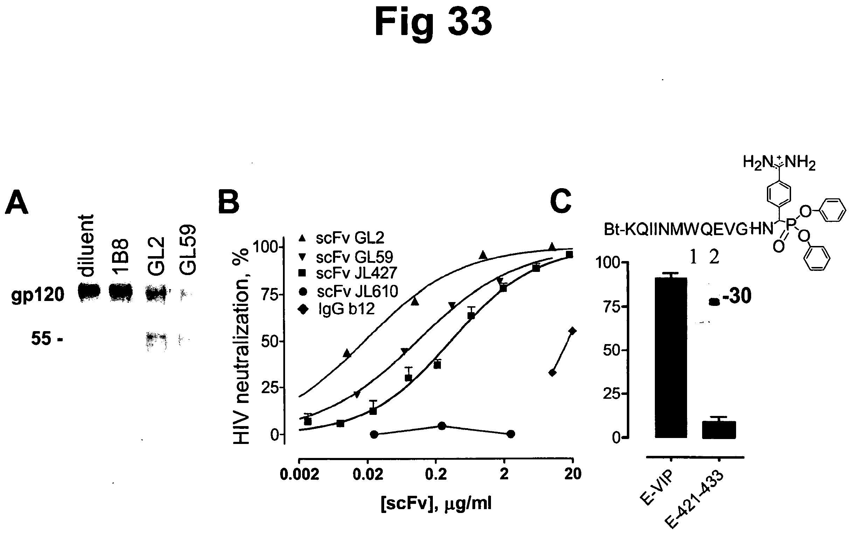

**Fig 33****. Catalytic and neutralizing activities of lupus scFv-*t* clones. *(A)*** Streptavidin-peroxidase stained blots of SDS-gels showing cleavage of Bt-gp120 (0.1 µM) by purified scFv-*t* clones GL2 and GL59 (20 µg/ml). scFv-*t* 1B8 analyzed in parallel is devoid of catalytic activity. ***(B)*** HIV neutralization (strain ZA009) by scFv-*t* clones. Host cells: PBMCs. ***(C)*** Inhibition of scFv-*t* neutralizing activity by E-421-433. scFv-*t* JL427 (0.24 µg/ml) preincubated for 24 h with E-421-433 (100 µM, see structure in inset) or the control electrophilic peptide E-VIP before assay of neutralizing activity and PBMC. *Inset,* streptavidin stained blot of SDS-gel showing formation of scFv-*t* JL427 adducts with E-421-433 (lane 2; 30 kD) and absence of adducts with E-VIP (lane 1).

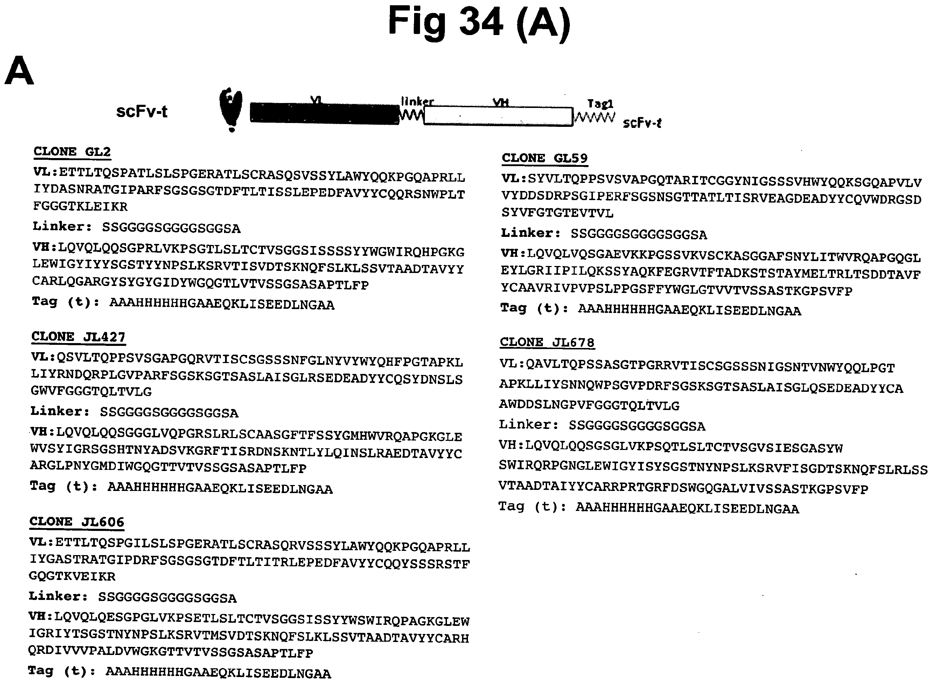

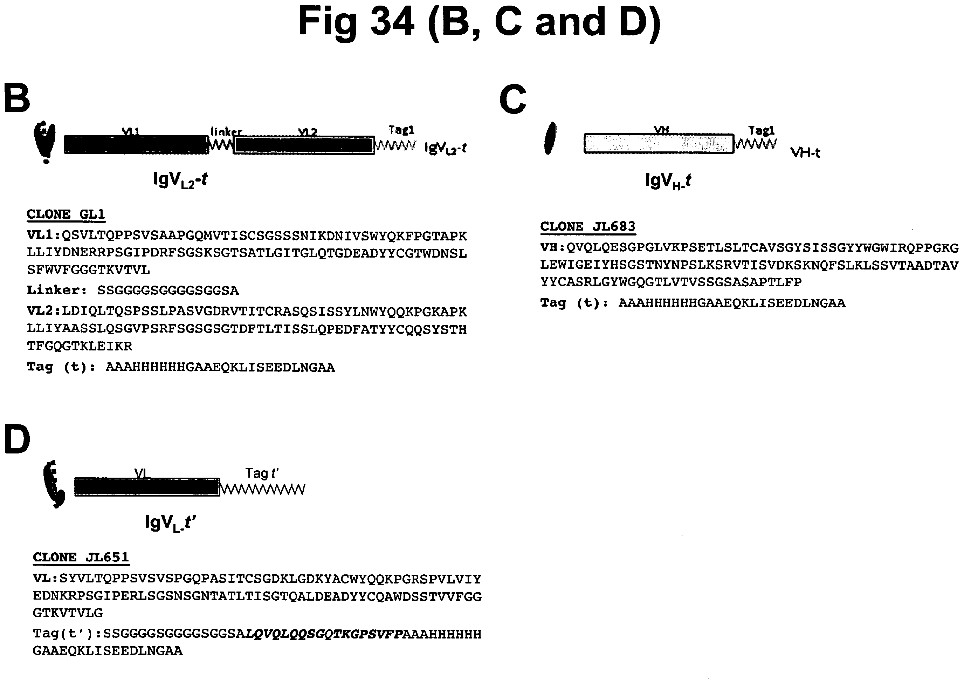

**Fig 34****. Schematic representations of the structures and amino acid sequences of IgVs. *(A)*** scFv-*t* clones GL2, GL59, JL427, JL606 and JL678. ***(B)*** IgV_{L2}-*t* clone GL1. **(C)** IgV_{H}-*t* clone JL683. **(D)** IgV_{L}-*t'* clone JL651. In JL651, tag *t'* corresponds to the linker, an aberrant polypeptide in place of the VH domain at the C terminus of the linker and the His6-c-myc tag.

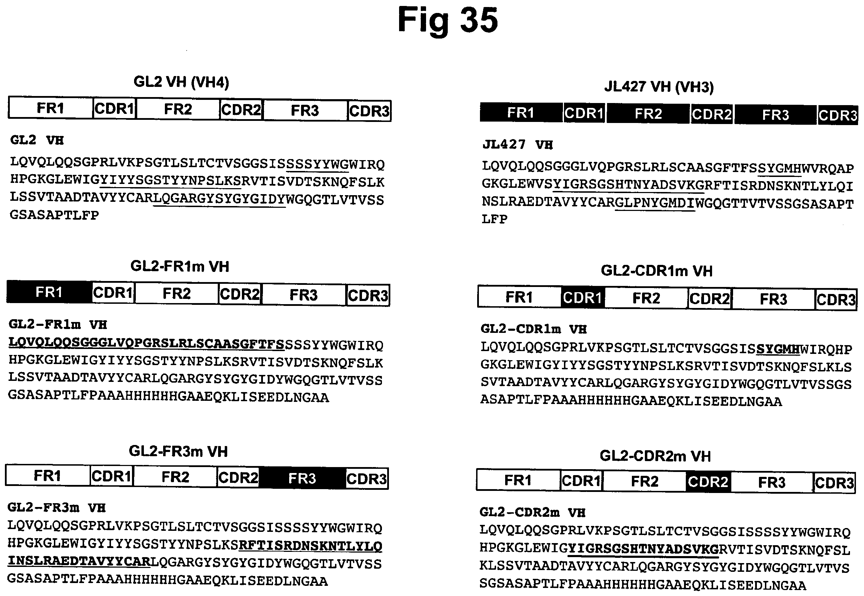

**Fig 35****. Schematic representations of the VH domain structures and amino acid sequences of FR-swapped mutants of scFv-*t* GL2.** Black and white boxes, respectively, are regions derived from scFv-*t* JL427 and scFv-*t* GL2. All mutants tested contained the VL domain of scFv-*t* GL2.

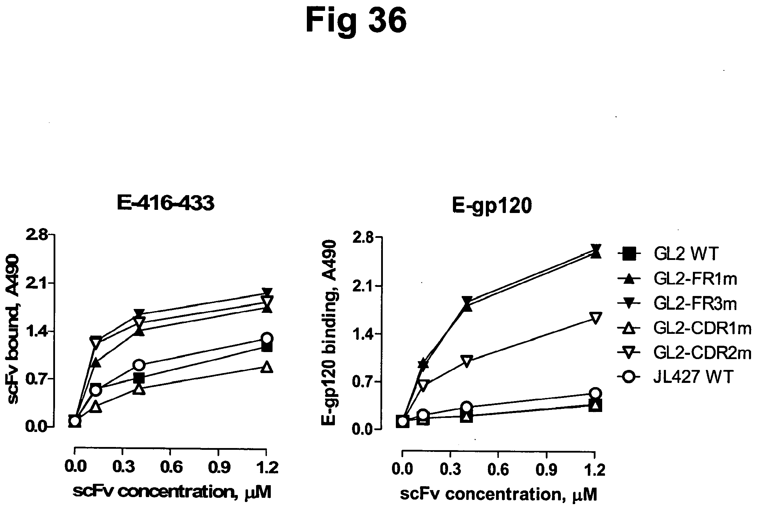

**Fig 36****. E-416-433 and E-gp120 binding by scFv GL2 mutants.** Shown are ELISA data with plates coated with E-416-433 (KLH conjugate; 70 ng peptide equiv/well) or E-gp120 (100 ng/well). Bound scFv was detected with anti-c-myc antibodies and peroxidase-conjugated anti-mouse IgG (Fc specific).

### DETAILED DESCRIPTION OF THE INVENTION

As used herein, the term "a" or "an", when used in conjunction with the term "comprising" in the claims and/or the specification, may refer to "one", but it is also consistent with the meaning of "one or more", "at least one", and "one or more than one". Some embodiments of the invention may consist of or consist essentially of one or more elements, method steps, and/or methods of the invention. It is contemplated that any compound, composition, or method described herein can be implemented with respect to any other device, compound, composition, or method described herein.

As used herein, the term "or" in the claims refers to "and/or" unless explicitly indicated to refer to alternatives only or the alternatives are mutually exclusive, although the disclosure supports a definition that refers to only alternatives and "and/or".

As used herein, the term "Ig" in the claims refers to any immunoglobulin of the IgM, IgG and classes.

As used herein, the term "IgV" in the claims refers to any variable domain of the light cand heavy chain Ig subunits with and without incorporation of additional sequences at the termini of the V domains.

As used herein, the term "tag" in the claims refers to any polypeptide or non-peptide incorporated at the terminus or within the V domain or combination of V domains.

As used herein, the term "single domain IgV" refers to a V domain that can fulfill the antigen recognition function in the absence of a second full-length V domain.

As used herein, the term "two domain IgV" in the claims refers to a combination of two V domains that can recognize the antigen.

As used herein, the term "antigen recognition" refers to the ability to bind the antigen by noncovalent means or covalent means or the ability to catalyze the chemical transformation of the antigen.

In one embodiment of the present invention, there are provided classes of IgVs with high level catalytic and binding activities and the desired bioactivity profiles. The structure and properties of the IgVs are exemplified by their reactivities with various antigens of medical interest. Optionally, the IgVs can be isolated from humans with the autoimmune disease systemic lupus erythematosus. Other suitable sources of the IgVs are humans without disease and humans with Alzheimer disease, *S. aureus* infection, HIV infection or HCV infection. For example, humans with Alzheimer disease produce Igs with specificity for Aβ peptide. Consequently, such humans are suitable for isolating IgVs directed to Aβ.

The invention discloses several IgVs that catalyze the hydrolysis of Aβ isolated from a library of IgVs derived from lupus patients. The library consists mostly of two domain IgVs containing paired VL-VH antigen combining sites. However, a minority of clones are structurally aberrant because of imprecision of cloning methods used for generating the library. The aberrant structures include IgVs containing two VL domains (designated IgVL2-t constructs wherein *t* denotes a short peptide tag included in the IgVs to help identify and purify the recombinant proteins) and IgVs with a single VL domain linked to non-natural polypeptides at the C terminus, e.g., short VH domain sequences containing large internal deletions (designated IgV_{L}-*t'* wherein *t'* denotes the tag region encompassing the aberrant structure at the terminus the VL domain). Random screening methods along with the use of an electrophilic Aβ analog to isolate IgVs with greatest nucleophilic reactivity permitted isolation of catalytic IgVs. Unexpectedly, IgVs with rare IgVL2-t and IgV_{L}-*t'* structures expressed the greatest catalytic activity directed to Aβ. The invention also discloses single chain Fv constructs (scFvs) obtained by repairing the aberrant VH domain contained in the IgV_{L}-*t'* construct. Such repaired scFvs displayed reduced catalytic activity, indicating that the VH domain generally suppresses VL domain catalytic activity.

The use of electrophilic antigen analogs in the present invention is based on the principle that nucleophilic sites located in the V domains imparts to some Igs the ability to catalyze chemical reactions. The nucleophilicity derives from activation of certain amino acid side chains. In serine proteases, precise spatial positioning of the Ser-His-Asp triad allows formation of a hydrogen bonded network that imparts nucleophilic reactivity to the Ser oxygen. Similar sites are present in catalytic V domains [1]. Previous studies have shown that V domain nucleophilic sites bind covalently to phosphonate esters incorporated within antigenic epitopes [2,3].

The electrophilic antigen analogs are designed based on the split site model of catalytic Igs, in which the Ig paratope and nucleophilic sites are treated as two distinct subsites. The analogs are derivatives of polypeptides in which one or more amino acid side chains are linked to the electrophilic phosphonate group as described in US Patent application 20070105092. Examples of other suitable electrophiles are the carbon atom in carbonyl esters, carbonyl amides, carbonates, aldehydes, ketones and aliphatic and aromatic carbonyl compounds; the boron atom in boronates and the vanadium atom in vanadates. Electron withdrawing and donating groups are linked directly to the electrophilic atom or via spacer groups to enhance and decrease the covalent reactivity with IgV nucleophiles. Optionally, a positive charge or a negative charge is placed in the vicinity of the electrophilic atom to mimic the basic residue and acidic residue specificity of catalytic Igs.

The catalytic sites of the IgVs disclosed in the present invention can be produced as a result of innate as well as adaptive immune processes. Previous studies have indicated that Ig nucleophilic and proteolytic activities are heritable traits, encoded by germline V domains [4]. Because the catalytic activity is germline-encoded, in principle, the immune system is capable of mounting catalytic Ig V domains directed to any polypeptide antigen. Adaptive specialization for recognition and cleavage of the polypeptide antigen can occur by processes such as V-D-J/V-J junctional diversification and somatic hypermutation of the V domains.

In another embodiment, the invention discloses improved catalytic mutants of an IgVL2-*t* obtained by random mutagenesis of the VL domains, display of the mutant proteins on phage surface and isolated mutant IgV_{L2}-*t* clones by covalent phage selection using the electrophilic Aβ analog.

Additional embodiments of the inventions disclose IgVs directed to proteins belonging to infectious microbes. One such embodiment is isolation of catalytic IgVs to *S. aureus* virulence factors, for example, the virulence factor Efb, obtained by random screening of the lupus IgV library described above. A catalytic scFv and an IgV_{L2}-*t* that hydrolyzed Efb was identified by the by electrophoresis screening methods. Catalytic IgVs with improved catalytic activity can readily be obtained by conducting phage selection procedures as described, for example, using electrophilic analogs of Efb.

The invention also discloses the composition of catalytic IgVs directed to the HIV coat protein gp120. These IgVs can be obtained from human IgV libraries as described above. Selection of the IgVs displayed on phage surface can be accomplished using gp120, an electrophilic analog of gp120 or peptides corresponding to the antigenic epitope recognized by the IgVs. HIV gp120 is a B cell superantigen recognized by IgVs from humans without infection. The invention discloses IgVs that recognize the superantigenic peptide epitope composed of gp120 amino acid residues 421-433 or 416-433 and neutralize HIV infection of lymphocytes. Certain IgVs that catalyze the hydrolysis of gp120 are described. As the IgVs neutralize diverse HIV strains they can be applied for immunotherapy of HIV infection. In addition, they can be used as topical microbicides for prevention of heterosexual HIV transmission.

Another embodiment of the invention concerns recognition of B cell superantigens by the V domains, which is thought to occur mainly at conserved V domain residues. IgVs obtained by mutagenesis methods with improved recognition of the HIV gp120 superantigenic site are disclosed. The approach for obtaining the mutants consists of replacing entire FRs or CDRs of the VH domains by corresponding FRs or CDRs drawn from other V domains. In addition, the ability of the VL domain to recognize the HIV gp120 superantigenic site is disclosed. Improved superantigen recognition can also be readily attained by pairing of two V domains that can independently recognize the superantigenic site. Similarly, mutations introduced at individual amino acids in the FRs or CDRs can be employed to improve IgV recognition of the superantigens. Also disclosed are the novel B cell superantigenic properties of the *S. aureus* virulence factors, Map19, ClfA, LukF and SdrE. The disclosed IgV engineering methods can readily be applied to obtain improved recognition of the *S. aureus* virulence factors.

The IgVs can be engineered further to improve their stability in vascular circulation and other anatomic sites, for example by linkage to a polyethylene glycol molecule or the Fc region of Igs. Also disclosed are novel full-length catalytic IgG*L* and IgM*L* molecules containing antigen combining sites composed of 2 VL domains instead of one VL domain and one VH domain. Also disclosed are two novel bivalent Fc-containing derivatives of an IgV_{L2}-*t*: ***(a)*** an IgG*L* construct in which one of the V_{L} domain was cloned into the light chain subunit (κ) and the second V_{L} domain into the heavy chain subunit (in place of the V_{H} domain); and ***(b)*** an Fcγ-(IgV_{L2})₂ construct containing two IgV_{L2} components attached to the N terminii of the Fc fragment. The novel IgG*L* and Fcγ-(IgV_{L2})₂ expressed catalytic activity, indicating that the integrity of catalytic site is unaffected by inclusion of the Fc fragment. Such engineering strategies can be used to prepare long-lived Ig catalysts as immunotherapeutic agents.

The invention discloses that variations in the structure of the terminal *t'* region that do not influence of the single domain IgV_{L}-*t'* function. The *t'* region interferes with noncovalent V domain association and maintains the IgV_{L}-*t'* in monomeric form with high level catalytic activity. Optionally, the *t'* region can contain peptide or non-peptide structures that direct the IgV to the desired target anatomic site. For example, inclusion of the polyanionic compound putrescine or the TAT peptide in the *t'* region can facilitate passage of the IgV across the blood-brain-barrier. Similarly, the *t'* region can include an scFv directed to an antigen expressed at the blood-brain-barrier to facilitate transport across the blood-rain-barrier, for example an scFv to the insulin receptor known in the art to transport drugs across the barrier.

Another aspect of the present invention consists of identifying polyclonal Igs from blood and mucosal secretions with enriched catalytic activity. This can be accomplished by screening Ig preparations from individual human donors and identifying those immunoglobulin preparations that express catalytic activity greater than the average catalytic activity of Ig preparations. Optionally, the Igs can be obtained from human blood. Alternatively, the present invention also discloses IgG, IgA and IgM preparations obtained from mucosal fluids such as saliva. The catalytic activity is directed to one or more protein, e.g., an *S. aureus* virulence factor, the HCV coat protein E2, the HIV coat protein gp120 or Aβ. For example, the invention discloses findings of exceptionally potent HIV neutralization by IgA preparations from long-term survivors of HIV infection. Such Ig preparations are a suitable source of pooled Igs for treatment and prevention of HIV infection. Similarly, screening of human donors has revealed variable levels of catalytic Igs directed to *S aureus* virulence factors protein A, Map19, ClfA, LukF and SdrE. Screening of human donors with HCV infection has revealed enhanced levels of catalytic IgMs to the HCV coat protein E2, with activity levels varying widely from one donor to another. Screening of human donors has revealed similar variations in catalytic IgMs directed to Aβ. The present invention conceives pooled Igs from donors expressing enriched levels of catalytic activities to the appropriate antigens to be useful in various medical treatments, including treatment of antibiotic-resistant *S. aureus infection,* HCV infection and Alzheimer disease.

To one skilled in the art, it is evident that protective monoclonal Abs can readily be cloned from the catalytic Ig producing subjects by procedures such as lymphocyte immortalization by Epstein-Barr virus or antibody repertoire cloning and phage display by molecular biology methods. Similarly, the present invention conceives preparation of IgVs from the catalytic Ig producing subjects.

The monoclonal and recombinant Igs disclosed in the present invention can be readily improved by various protein engineering methods familiar to persons skilled in the art. Examples of the engineering techniques are as follows.

The IgVs can be recloned as full-length IgG, IgM and IgA to provide for increased half-life in vivo and increased avidity of protein recognition. When administered to animals, Fv constructs display half-lives in blood on the order of hours. In comparison, the half-life of full-length Abs in blood can be as large as 2-3 weeks. Therefore, to achieve persistent neutralization of the antigen, the preferred reagents are the full-length Igs. On the other hand, the smaller IgV constructs may offer tissue penetration capabilities superior to full-length Igs.

Recloning monovalent IgVs as IgG, IgM and IgA provides for increased antigen binding valencies, respectively. This is useful in some instances. Multivalent binding improves the apparent antigen binding strength, known in the art by the term avidity. In addition, the constant domains imparts important effector functions to Igs, for example, the ability to fix complement, mediate Ig-dependent cellular cytotoxicity and bind Fc receptors. Full-length Igs are readily obtained from IgVs by cloning the V domains into appropriate mammalian cell expression vectors. The vectors contain cDNA encoding the constant domains of the desired Ig class and subclass. The vectors are available commercially, for example, from Lonza. The vectors contain human Ig constant domains flanked by restriction sites for insertion of foreign V domains.

Increased avidity of antigen recognition can also be obtained by forming IgV multimers. For example, tetravalent antibody fragments are generated by placing a 33-amino acid self-aggregating peptide derived from the GNC4 protein at the C terminus of an scFv construct. The peptide associates noncovalently into a 4-helix bundle, permitting expression of multiple valencies.

The sequences of VL, VH and linker domains can be varied by mutagenesis to improve their biological activity. The mutants expressed on the surface of a display vector as described above are allowed to bind the target antigen. This allows separation of the mutants with the highest antigen recognition capability, which in turn can be anticipated to result in improved neutralization capacity. Mutagenesis of the linker peptide that joins the VL and VH domains is designed to improve the interfacial contacts of the VL and VH domains, which allows these domains to form superior antigen binding cavities. To obtain IgVs with improved catalytic activity, mutations are introduced into the FRs or CDRs using mutagenic primers, the mutant molecules are expressed on the surface of phages, and the phages are allowed to bind covalently to the electrophilic antigen analogs. The process is repeated several times, with additional mutations introduced at each cycle followed by the phage separation by antigen binding.

In addition to the strategy described above, favorable mutations can also be introduced in the V domains on a rational basis to improve the catalytic activity. For instance, candidate amino acids suitable for mutagenesis can be identified by molecular modeling or X-ray crystallography information. The ligand can be positioned in the hypothetical binding site to identify candidate residues suitable for rational mutagenesis. For instance, replacement of a small neutral amino acid with a similarly sized charged residue can be attempted as a means to introduce an additional electrostatic stabilizing interaction.

The catalytic VL domain can be paired with the VH domain of other Igs with specific target antigen binding activity. This can improve the binding strength to the target antigen and also result in changes in epitope specificity that can improve antigen neutralizing activity.

The catalytic Igs described herein are generally administered to a patient as a pharmaceutical preparation. The pharmaceutical preparations of the invention are conveniently formulated for administration with a acceptable medium such as water, buffered saline, ethanol, polyol (for example, glycerol, propylene glycol, liquid polyethylene glycol and the like), dimethyl sulfoxide (DMSO), oils, detergents, suspending agents or suitable mixtures thereof. The concentration of the Abs in the chosen medium will depend on the hydrophobic or hydrophilic nature of the medium, as well as the other properties of the catalytic antibodies. Solubility limits may be easily determined by one skilled in the art.

As used herein, "biologically acceptable medium" includes any and all solvents, dispersion media and the like which may be appropriate for the desired route of administration of the pharmaceutical preparation, as exemplified in the preceding paragraph. The use of such media for pharmaceutically active substances is known in the art. Except insofar as any conventional media or agent is incompatible with the catalytic antibody to be administered, its use in the pharmaceutical preparation is contemplated.

In one preferred embodiment, the catalytic Igs can be infused intravenously into the patient. For treatment of certain medical disorders, steps must be taken to ensure that sufficient amounts of the molecules reach their target cells to exert a biological effect. The lipophilicity of the molecules, or the pharmaceutical preparation in which they are delivered may have to be increased so that the molecules can arrive at their target locations. Furthermore, the Igs of the invention may have to be delivered in a cell-targeted carrier so that sufficient numbers of molecules will reach the target cells. Methods for increasing the lipophilicity and targeting of therapeutic molecules, which include capsulation of the Igs of the invention into liposomes, are known in the art.

The IgVs and Igs of the present invention can also be used as topical microbicides, for example as a vaginal cream, foam or film.

The pharmaceutical IgV or Ig preparation is formulated in dosage unit form for ease of administration and uniformity of dosage. Dosage unit form, as used herein, refers to a physically discrete unit of the pharmaceutical preparation appropriate for the patient undergoing treatment. Each dosage should contain a quantity of active ingredient calculated to produce the desired effect in association with the selected pharmaceutical carrier. Procedures for determining the appropriate dosage unit are well known to those skilled in the art. For example, the half-life of syngeneic IgG in the human is about 20 days. Over this period, 60,480 antigen molecules will be cleaved by one molecule of an antibody with a turnover of 2.1/min. It can be seen, therefore, that the peptidase antibodies can express considerably more potent antigen neutralizing activity than stoichiometric, reversibly-binding molecules.

The pharmaceutical preparation comprising the catalytic Igs may be administered at appropriate intervals, for example, twice a week until the pathological symptoms are reduced or alleviated, after which the dosage may be reduced to a maintenance level. The appropriate interval in a particular case would normally depend on the condition and the pathogenic state sought to be treated in the patient.

Standard criteria for acceptable prophylactic or therapeutic agents are employed as follows: (1) Discussions of how such criteria are established for the acceptability of prophylactic or therapeutic agents are common in the art can can be found in such texts as Guide to Clinical Trials by Bert Spilker, Raven Press, New York, 1991. Acceptable criteria for demonstration of efficacy include, for example, measuring clearance of bacterial infection. Conventional monoclonal Igs that act to inhibit the function of particular target molecules are among the most common type of therapeutic agent under development for clinical use by biotechnology and pharmaceutical companies. Accordingly, methods of administration of monoclonal Igs are well known to clinicians of ordinary skill in the art. The Igs contemplated in the present invention will constitute a major improvement over such conventional monoclonal Igs because of their superior potency, resulting in dramatic decrease in the cost of treatment.

While certain of the preferred embodiments of the present invention have been described and specifically exemplified above, it is not intended that the invention be limited to such embodiments. Various modifications may be made thereto without departing from the scope and spirit of the present invention. The following examples are provided to facilitate an understanding of the present invention.

### References

1. Gao QS, Sun M, Rees AR, Paul S: Site-directed mutagenesis of proteolytic antibody light chain. J Mol Biol 1995, 253:658-664.
2. Planque S, Taguchi H, Burr G, Bhatia G, Karle S, Zhou YX, Nishiyama Y, Paul S: Broadly distributed chemical reactivity of natural antibodies expressed in coordination with specific antigen binding activity. J Biol Chem 2003, 278:20436-20443.
3. Nishiyama Y, Bhatia G, Bangale Y, Planque S, Mitsuda Y, Taguchi H, Karle S, Paul S: Toward selective covalent inactivation of pathogenic antibodies: a phosphate diester analog of vasoactive intestinal peptide that inactivates catalytic autoantibodies. J Biol Chem 2004, 279:7877-7883.
4. Gololobov G, Sun M, Paul S: Innate antibody catalysis. Mol Immunol 1999, 36:1215-1222.

### EXAMPLE 1: Catalytic Igs to amyloid β peptide

***Aβ hydrolyzing polyclonal IgMs.*** Accumulation of amyloid β peptide (Aβ) aggregates in the brain is thought to be a central event leading to neurodegenerative changes observed in Alzheimer disease (AD). There is consensus that immunoglobulins (Igs) with specific Aβ binding activity are viable candidates for AD therapy. Monoclonal and polyclonal candidate Igs are under consideration for this purpose. The polyclonal Igs consist of pooled IgG class antibodies from normal humans that contain a small amount of Aβ binding IgGs (commonly referred to as intravenously infused IgG, IVIG [1]. The monoclonal Igs were raised by immunization of mice with Aβ and 'humanized' by protein engineering to reduce undesirable human anti-mouse Ig responses. Studies using murine AD models have indicated that peripherally administered monoclonal Igs and polyclonal IVIG with Aβ binding activity can clear Aβ from the brain [2-4]. Phase III trials of a monoclonal Aβ binding IgG for AD therapyare ongoing (Wyeth-Elan). Baxter has announced its intent to start a Phase III IVIG trial.

We observed that electrophoretically homogeneous IgMs from the sera of elderly healthy subjects without dementia (>65 years) hydrolyzed Aβ at levels greater than non-elderly adults (<35 years; P=0.035; Student's t-test for unpaired observations[5]. To evaluate disease association, we compared the hydrolysis of ¹²⁵I-Aβ40 by IgM preparations from 25 non-demented elderly individuals and 23 AD patients. Excess albumin was present as an alternate substrate in the assays, minimizing the contribution of promiscuous antigen recognition. Intact Aβ was separated from product peptides by 5% trichloroacetic acid (TCA) precipitation. Alternatively, reversed-phase high performance liquid chromatography was employed to separate intact and fragmented Aβ using a Novapak C18 column with 0.05 % trifluoroacetic acid in acetonitrile for elution. Observed values of hydrolysis measured by the TCA precipitation method and RP-HPLC were highly correlated (r² 0.96). Twenty two of the 25 IgM preparations from undemented elderly humans studied displayed detectable ¹²⁵I-Aβ40 hydrolytic activity varying over a 118-fold range. This suggests that the catalytic IgM response is polymorphic and varies in different individuals. IgMs from the AD group displayed superior hydrolytic activity (P<0.0001; two-tailed Mann-Whitney U test and Student's t-test; Fig. 1). HPLC and ESI-MS studies indicated using pooled IgM indicated hydrolysis mainly at the Lys28-Gly29 bond, and at lesser levels, at the Lys16-Leu17 bond. The IgMs from AD patients did not hydrolyze irrelevant polypeptides determined by an electrophoresis assay (biotinylated soluble epidermal growth factor receptor, albumin or ovalbumin [5]. It may be concluded that increased Aβ40 hydrolysis by IgM preparations from AD patients is not due to an increase of non-specific catalytic activity.

To exclude the possibility of trace protease contaminants, pooled polyclonal IgM previously purified by affinity chromatography using anti-IgM antibody was subjected to 2 cycles of sequential gel filtration in a solvent that dissociates noncovalently associated protein-protein complexes (6 mol/L guanidine hydrochloride). The highly purified 900 kDa IgM fraction from the second chromatography cycle displayed detectable Aβ40 hydrolytic activity determined by RP-HPLC (68 pmol/h/mg IgM), and the product profile was essentially identical to the starting IgM preparation. To our knowledge, there are no known conventional proteases with mass 900 kDa. The denaturing column conditions preclude the possibility of smaller adventitious proteases.

***Aβ40 hydrolyzing IgVs.*** We searched for Aβ40 hydrolyzing recombinant IgVs in a human library composed of ∼10⁷ clones. The library was constructed as described in (6). A majority of the clones in the library are scFv-*t* constructs with the domain organization V_{L}*-Li-*V_{H}*-t,* where *Li* denotes the 16-residue peptide SS(GGGGS)₂GGSA joining the V_{L} domain C terminus to the V_{H} domain N terminus and *t* denotes the 26 residues C terminal peptide containing the *c-myc* peptide and his6 tags [6]. The library also contains a minority of IgV clones with unnatural structures generated by cloning errors (see below). Sixty three IgVs purified from the periplasmic extracts of randomly picked clones by his6 tag binding to Ni-affinity columns were tested for ¹²⁵I-Aβ40 hydrolyzing activity. Concentrations of the IgV in these extracts are variable and depend on the expression level from bacteria. Generally, the concentration vary from 1-10 µg/ml in the hydrolysis assay. Two IgVs displayed with activity distinctly greater than the remaining clones were identified (Fig 2A). The activity of the empty vector control extract (pHEN2 devoid of an IgV insert) was within the range of assay errors (mean of background TCA soluble radioactivity+3 S.D. values, 32.4 fmol/h/mg/IgV). The phagemid DNA of the high activity IgV clone 2E6 was re-expressed in 15 individual bacterial colonies. All recloned colonies secreted IgV with robust ¹²⁵I-Aβ40 hydrolyzing activity (Fig 2B), ruling out trivial sample preparation differences as the cause of proteolytic activity. As before, purified extract of the control empty vector clone did not hydrolyze ¹²⁵I-Aβ40.

Our previous studies have suggested catalytic Ig nucleophilic sites recognize polypeptide antigens containing electrophilic phosphonates by noncovalent peptide epitope binding coordinated with covalent bonding to the phosphonate group [7]. To isolate Aβ40-selective nucleophilic IgVs, we prepared a biotinylated Aβ40 analog (Bt-E-Aβ40; Fig 3A) containing phosphonates located at the side chains of Lys16 and Lys25 residues. Bt-Aβ40 (6 mg) was reacted with diphenyl-*N*-[*O*-(3-sulfosuccinimidyl)suberoyl]-amino(4amidinophenyl)methane phosphonate in DMSO. The reaction mixture was purified by reversed-phase HPLC, and lyophilization. Its identity and purity were confirmed by HPLC [retention time 36.36 min, >99.9%; 0.05% TFA in water:0.05% TFA in acetonitrile:10-40:60 in 50 min, 1.0 ml/min; 220 nm absorbance], electrospray ionization mass spectrometry [observed *m*/*z*, 1427.6, 1142.6 and 952.4; calculated (*M*+4H)⁴⁺, (*M*+ 5H)⁵⁺ and (M+ 6H)⁶⁺ for C₂₆₆H₃₈₀N₆₂O₇₁P₂S_{2;} 1427.1, 1141.9 and 951.8, respectively].

Phages displaying the IgVs as p3-fusion proteins were packaged from TG1 *E.coli* cells harboring the recombinant vector using helper phages, and the phages were subjected to a covalent selection procedure. Briefly, the phages were incubated (1 ml; 10 min, 25°C) with Bt-E-Aβ40 immobilized on anti-biotin gel (Sigma) in 10mM sodium phosphate, 0.137mM NaCl, 2.7mM KCl, pH 7.4, (PBS) containing 1% skimmed milk. The phage-gel mixture was packed in a column and washed with PBS until A280 was <0.01. Noncovalently bound phages were removed by successive washes with Aβ1-40 solution (5 column volumes) and covalently bound phages were eluted with 0.1M glycine-HCl, pH 2.7. Soluble IgVs were obtained by infecting HB2151 cells with the phages. Periplasmic extracts were prepared following induction with isopropyl-d-thiogalactoside. For initial screening of catalytic activity, the IgVs were purified by a single round of metal affinity chromatography by means of the His6 tag at the C terminus. For further characterization, the extract was subjected to two sequential rounds of metal affinity chromatography. SDS-gel electrophoresis was on 4-20% gels. Total protein was determined by the microBCA kit (Pierce). To separate monomer from aggregates and eliminate degradation products, an FPLC anion exchange column (MonoQ HR 5/5, GE healthcare) equilibrated with 50mM Tris buffer, pH 7.4,containing 0.1mM CHAPS was employed. Ni-purified IgVs (10 ml) were dialyzed against equilibration buffer and subjected to chromatography. Bound protein was eluted with a linear gradient of NaCl from 0 to 1M. Fractions (500 µl each) were analyzed by SDS-PAGE as described above.

Phage IgVs obtained by treatment with excess Aβ40 were designated noncovalently selected IgVs. Residual irreversible phage IgV immune complexes eluted by acid disruption of the biotin-anti-biotin antibody complexes were designated covalently selected IgVs. The noncovalently selected IgVs displayed no or minimal ¹²⁵I-Aβ40 hydrolyzing activity (Fig 3B). Several covalently selected IgVs expressed robust hydrolytic activity that increased increased as a function of decreasing E-Aβ40 concentrations employed for phage selection (Fig 3C). This is consistent with the prediction of more efficient proteolysis by IgVs with the greatest Aβ40-directed nucleophilic and noncovalent binding reactivities.

***IgV primary structure and activity validation.*** IgV clone 2E6 obtained without phage selection and 3 covalently selected IgVs with the greatest Aβ40-hydrolyzing activity (clones 5D3, 1E4 and 5H3) were studied further. Identical cDNA sequences were obtained for each clone by sequencing from the 5' to 3' direction and the 3' to 5' direction. IgV 2E6 is a single chain heterodimer of two different V_{L} domains (designated heterodimeric IgV_{L2}-*t*, Fig 4A; full sequences shown in Fig 5). IgVs 5D3, 1E4 and 5H3 are single domain V_{L} clones with unexpected C terminal polypeptide segments, designated IgV_{L}-*t'* clones, where *t'* denotes *(a)* the expected linker peptide, *(b)* an unexpected 15-28 residue aberrant peptide sequence in place of the customary V_{H} domain composed of ∼115 residues, and (c) the expected 26 residue peptide sequence containing *c-myc* and his6 tags (Fig 4A and Fig 5).

Two clones were studied further, IgV_{L2}-*t* 2E6 and IgV_{L}-*t'* 5D3. Their deduced protein masses predicted from the cDNA sequences, are respectively, 27 and 17 kD. Denaturing electrophoresis and silver staining of the proteins purified by 2 cycles of his₆ binding to Ni columns revealed proteins close to the predicted mass of the monomer IgVs (IgV_{L2}-*t* 2E6, 30 kD; IgV_{L}-*t'* 5D3, 18 kD; Fig 4B). However, additional protein bands were visible (low mass IgV_{L2}-*t* 2E6 band at 18 kD; high mass IgV_{L}-*t'* 5D3 bands at 36, 50, 58, 67 and 74 kD). All of the additional bands were stained with anti-c-*myc* antibody (Fig 4B). As irrelevant proteins are not stained nonspecifically by the anti-c-*myc* antibody (phosphorylase b, BSA, ovalbumin, carbonic anhydrase, trypsin inhibitor, α-lactalbumin), there is no evidence of non-IgV contaminants. We concluded that the anomalous low mass IgV_{L2}-*t* band is a self-degradation product and the high mass IgV_{L}-*t'* band are aggregates. The IgV_{L2}-*t* and IgV_{L}-*t'* were purified by anion exchange FPLC to electrophoretically homogeneous proteins that migrated as monomers in the denaturing electrophoresis gels (Fig 4B). The identity of the 5D3 monomer band was confirmed by peptide tryptic digestion and mass spectroscopic analysis. The IgV ¹²⁵I-Aβ40 hydrolyzing activities were essentially identical after one and two cycles of chromatography on Ni columns, and they were increased noticeably following FPLC purification (Table 1; by 3.1-fold and 31.2-fold, respectively, for the IgV_{L2}-*t* and IgV_{L}-*t'*). These observations indicate that the Aβ hydrolyzing activity is attributable to the IgVs.

**Table. 1 ¹²⁵I-Aβ1-40 hydrolyzing activity of different IgV preparations. The recombinant IgV preparations purified by one round of metal-affinity chromatography on Ni agarose (preparation "MA1") were subjected to either another round of Ni chromatography ("MA2") or anion exchange chromatography on MonoQ ("AEQ"). Hydrolysis assay conditions are as in Fig. 3. Shown are specific activities expressed in fmol/h/mg protein unit (mean ± SD).**

| Ig | Specific activity, fmol/h/mg protein IgV (mean ± SD) | | |
|---|---|---|---|
| | MA1 | MA2 | AEQ |
| IgV_{L2}-*t* 2E6 | 380 ± 73 | 368 ± 40 | 1158 ± 285 |
| IgV_{L}-*t'* 5D3 | 543 ± 270 | 552 ± 127 | 17239 ± 1481 |

Sequencing of 26 randomly picked clones from the library indicated that the majority of the IgVs in the library are scFv constructs (83.3%) and a minority are IgV_{L2}-*t* constructs (12.5%) or IgV_{L}-*t'* (4.2%) structures. The cumulative probability of identifying 4 high activity Aβ40-hydrolyzing clones from the library with the rarely represented IgV_{L2}-*t* or IgV_{L}-*t'* structures by random chance alone is very small (P=0.92x10⁻⁵; computed as 0.125 x 0.042³). As none of the high activity clones contain the archetypal V_{L}-V_{H} paired structure of physiological Ab combining sites, it may be concluded that expression of Aβ hydrolyzing activity by the rare V_{L} domain IgV structures is a favored event.

We reported recently the Aβ40 hydrolyzing activity of polyclonal human IgM preparations, a monoclonal IgM from a patient with Waldenstrom's macroglobulinemia [5], and the cross-reactive light chain subunit of an Ig directed to the antigen VIP [8]. IgV_{L2}-*t* 2E6 and IgV_{L}-*t'* 5 D3 hydrolyzed ¹²⁵I-Aβ40 with potency superior to the previously identified IgM and light chain preparations by 1-4 orders of magnitude (Fig 6A).

To further improve the catalytic activity, we prepared a phage library of randomly mutated IgV_{L2}-*t* 2E6 clones (∼4 mutations/molecule; 7x10⁶ clones) and isolated mutant IgV_{L2}-*t* clones by covalent phage selection. The library was produced by a standard error-prone PCR method known to a person skilled in the art using a commercially available kit (Mutazyme II, Stratagene; template clone 2E6 in pHEN2; concentration adjusted to yield the desired number of mutations; verified by sequencing of 15 clones). Mutant IgV_{L2}-*t* phages were selected by covalent binding to E-Bt-Aβ40 (0.1 nM) as described above. Soluble IgV_{L2}-*t* mutants secreted into culture supernatants were screened for Aβ hydrolytic activity along with empty vector controls and wildtype supernatants. Several mutants with substantially improved Aβ hydrolyzing activity have been isolated (Fig 6B). The mutant with greatest activity, designated clone 2E6m, has been selected for further development in the present proposal.

***Repaired IgV 5D3.*** The aberrant *t'* region of IgV_{L}-*t'* 5D3 contains 7 residue V_{H} FR1 and 8 residue CH1 peptides with deletion of V_{H} residues 8-115 (Kabat numbering; Fig 7). Repaired scFv-*t* molecules were generated by replacing the *t'* region of IgV_{L}-*t'* 5D3 by four randomly picked full-length V_{H} domains (sequences are available in Fig 7). The deleted V_{H} segment was filled in by PCR amplification of V_{H} domains from the IgV library. For VH amplification, back and forward primers were designed to anneal the partial FR1 and CH1 sequences. To facilitate cloning, *Apa*LI and *Not*I sites were incorporated into the back (GGTAGTGCACTTCAGGTG CAGCTGTTGCAGTCT) and forward (ATGTGCGGCCGCGGGGAAAAGGGTTGGGGGCA TGC) primers, respectively. Plasmid DNA encoding the IgV library was used as the template. PCR was performed using *Taq* DNA polymerase (Invitrogen) as follows: an initial denaturation step for 5 min at 95°C, followed by 35 cycles each of amplification (1 min at 95°C, 1 min at 40°C and 1 min at 72°C) and a final extension step of 10 min at 72°C. The PCR product was digested with *Apa*LI and *Not*I and ligated into the IgV vector similarly digested using T4 DNA ligase (Invitrogen) according to manufacturer's protocols. Electrocompetent HB2151 *E. coli* were transformed with the ligation mixture and transformants were selected on 2YT ampicillin plates. To prepare the full-length L chain counterpart of clone 5D3, the V_{L} domain was amplified by PCR with primers contaning *Nco*I and *Not*I restriction sites and cloned into similarly digested pHEN2. The construct also contained the constant κ domain with the His6-c-myc tag at the C terminus. The sequences of the constructs were verified and soluble scFv antibodies were expressed in HB2151 *E. coli* as mentioned above.Purification of secreted scFv-*t* and L-*t* on Ni columns afforded electrophoretically homogeneous scFv-*t* proteins at the expected mass (30 kD, example shown in Fig 8A, inset).

The ¹²⁵I-Aβ40-hydrolyzing activity of the scFv-*t* constructs was consistently lower than the parent IgV_{L}-*t'* (by ∼82-167 fold; Fig 8A), suggesting an autonomous catalytic site located in the V_{L} domain that is suppressed by pairing with the V_{H} domains. The V_{L} domains of Igs tend to form noncovalent aggregates [9,10], introducing uncertainty as to whether the catalytic species is a monomer or an aggregate. To address this, we prepared the homodimeric IgV_{L2}-*t* molecule containing the two 5D3 V_{L} domains connected by the peptide linker. Purified IgV_{L2}-*t* 5D3 hydrolyzed ¹²⁵I-Aβ40 poorly (Fig 8B). On the other hand, the V_{L} domain linked to the κ constant domain displayed readily detected hydrolytic activity (this molecule corresponds to the light chain subunit, designated L-t). In denaturing electrophoresis gels, the homodimeric IgV_{L2}-*t* 5D3 migrated at the predicted 27 kD position with no evidence of degradation into monomers (Fig 8B inset). L-*t*, on the other hand, migrated as a mixture of 30 kD monomers and 60 kD S-S bonded dimers. These observations suggest that the monomeric, unpaired V_{L} domain state favors maintenance of catalytic site integrity.

IgVL2-*t* 2E6 was recloned as an IgG*L* using immunoglobulin expression vector cassettes containing human γ1 constant and λ constant domains described in (McLean et al.; Molec. Immunol. 37, 837-845, 2000). The two VL domains were PCR amplified using primers containing appropriate restriction sites (taagatctCAGTCTGCCCTGACTCAGCCT, tagcggccgcgggctgaccTAAAACGGTGAG; contain *Nco*I/*Xho*I sites and *Bgl*II/*Not*I sites, respectively; taGAATTCCAGTTGACCCAGTCTCC and taaagcttgcACGTT TGATTTCCAGCT T; contain *Apa*LI/*Not*I sites and *Eco*RI/*Hind*III)*,* followed by cloning into similarly digested pHC-huCγ1 and pLC-huCλ vectors. Essentially similar methods were used to prepare the Fcγ-(IgV_{L2})₂ 2E6 construct containing two IgV_{L2} components attached to the N terminus of the γ1 Fc fragment using the pHC-huCγ1 vector in which the CH1 domain of the heavy chain subunit had been removed. After sequence verification, the recombinant proteins were expressed in HEK293 cells by transient transfection using Lipofectamine (Invitrogen) using standard protocols. The proteins were purified by protein G affinity chromatography as described previously.

The three versions of the 2E6 clone [IgG*L*, Fcγ-(IgV_{L2})₂ and IgV_{L2}-*t*] hydrolyzed Aβ with comparable potency (Fig 8C). Identically purified irrelevant IgGs did not hydrolyze Aβ. These observations indicate that the integrity of catalytic site is not compromised by inclusion of the Fc fragment and that it is possible to prepare long-lived versions of the Ig catalysts as candidate immunotherapeutic agents.

***Catalytic properties.*** The hydrolytic activity of both IgV clones was saturable with increasing Aβ40 concentration (1 nM-100 µM nonradioactive Aβ40 mixed with 0.1 nM ¹²⁵I-Aβ40). Kinetic parameters are reported in Table 2.

**Table 2. Apparent kinetic parameters for Aβ40 hydrolysis by recombinant antibody fragments. Reaction rates were measured by TCA precipitation following incubation of increasing Aβ40 (0.001-40 µM) containing constant amount of ¹²⁵I-Aβ40 (∼300000 cpm) with IgV_{L2}-t 2E6 (3.75 µg/ml) or IgV_{L}-t' 5D3 (0.15 µg/ml). Kinetic constants were obtained from non-linear regression fits to the Michaelis-Menten equation (r² for IgV_{L2}-t 2E6 and IgV_{L}-t' 5D3, respectively, 0.99 and 0.99.**

| Catalyst | *Km* (M) | *k_{cat}* (min⁻¹) | *k_{cat}*/*Km* (M⁻¹ min⁻¹) |
|---|---|---|---|
| IgV_{L2}-*t* 2E6 | 8.0 x 10⁻⁵ | 3.0 x 10⁻¹ | 3.7 x 10³ |
| IgV_{L}-*t'* 5D3 | 1.7 x 10⁻⁵ | 1.0 | 5.9 x 10⁴ |

MALDI-MS of nonradioactive Aβ40 treated with IgV_{L2}-*t* 2E6 or IgV_{L}-*t'* 5D3 indicated similar product profiles. The deduced peptide that are hydrolyzed are shown in Fig 9A and the product profiles with the IgV_{L2}-*t* as catalyst are in Fig 9B,C. The prominent products were Aβ1-14 and Aβ15-40 (see Fig 6 legend for observed and calculated m/z values), suggesting hydrolysis of the His14-Gln15 peptide bond. Smaller signals for the following peptide products were also detected: Aβ1-15, Aβ1-20, Aβ16-40 and Aβ21-40. The corresponding scissile bonds in Aβ40 are Gln15-Lys16 and Phe20-Ala21. Similar product profiles were evident in reaction mixtures of the longer Aβ42 peptide treated with the IgV_{L2}-*t* 2E6 (Fig 9D,E), except that additional minor peptide products suggesting cleavage at the Lys27-Gly28 and Gly28-Ala29 bonds were evident.

As MALDI-MS does not enable accurate quantification of the reaction products, we also conducted RP-HPLC of Aβ40 treated with IgV_{L2}-*t* 2E6. This indicated depletion of the intact Aβ40 peak, accompanied by appearance of a major peptide product absent in control chromatograms of the IgVs alone or Aβ40 alone (Fig 10). The product peak was identified as Aβ1-14 by ESI-MS, suggesting the His14-Gln15 bond as the major cleavage site.

Previous studies have indicated that naturally occurring proteolytic Igs frequently utilize a serine protease catalytic mechanism entailing nucleophilic attack on the electrophilic carbonyl of peptide bonds [11]. This was the basis for covalent phage IgV selection with the electrophilic Aβ40 analog reported in Fig 3. To confirm the reaction mechanism, we studied the reactivity of IgV_{L2}-*t* 2E6 and IgV_{L}-*t'* 5D3 with the electrophilic phosphonate diester E-hapten-**1** (Fig 11A) developed originally as an active site-directed covalent inhibitor of serine proteases [12]. E-hapten-**1** inhibited ¹²⁵I-Aβ40 hydrolysis by both clones (Fig 11B). The biotin-containing version of the phosphonate diester, E-hapten-**2**, formed 30 kD covalent adducts with IgV_{L2}-*t* 2E6 that were stable to boiling and SDS treatment (Fig 11B. inset). The control hapten-**3**, a poorly electrophilic analog of E-hapten-**2**, did not form detectable adducts with the IgV_{L2}-*t*. The results support a nucleophilic catalytic mechanism.

### References for Example 1

1. Dodel RC, Du Y, Depboylu C, Hampel H, Frolich L, Haag A, Hemmeter U, Paulsen S, Teipel SJ, Brettschneider S, et al.: Intravenous immunoglobulins containing antibodies against beta-amyloid for the treatment of Alzheimer's disease. J Neurol Neurosurg Psychiatry 2004, 75:1472-1474.
2. White AR, Hawke SH: Immunotherapy as a therapeutic treatment for neurodegenerative disorders. J Neurochem 2003, 87:801-808.
3. Dodel R, Hampel H, Depboylu C, Lin S, Gao F, Schock S, Jackel S, Wei X, Buerger K, Hoft C, et al.: Human antibodies against amyloid beta peptide: a potential treatment for Alzheimer's disease. Ann Neurol 2002, 52:253-256.
4. Bard F, Cannon C, Barbour R, Burke RL, Games D, Grajeda H, Guido T, Hu K, Huang J, Johnson-Wood K, et al.: Peripherally administered antibodies against amyloid beta-peptide enter the central nervous system and reduce pathology in a mouse model of Alzheimer disease. Nat Med 2000, 6:916-919.
5. Taguchi H, Planque S, Nishiyama Y, Symersky J, Boivin S, Szabo P, Friedland RP, Ramsland PA, Edmundson AB, Weksler ME, et al.: Autoantibody-catalyzed Hydrolysis of Amyloid {beta} Peptide. J Biol Chem 2008, 283:4714-4722.
6. Paul S, Tramontano A, Gololobov G, Zhou YX, Taguchi H, Karle S, Nishiyama Y, Planque S, George S: Phosphonate ester probes for proteolytic antibodies. J Biol Chem 2001, 276:28314-28320.
7. Nishiyama Y, Bhatia G, Bangale Y, Planque S, Mitsuda Y, Taguchi H, Karle S, Paul S: Toward selective covalent inactivation of pathogenic antibodies: a phosphate diester analog of vasoactive intestinal peptide that inactivates catalytic autoantibodies. J Biol Chem 2004, 279:7877-7883.
8. Liu R, McAllister C, Lyubchenko Y, Sierks MR: Proteolytic antibody light chains alter beta-amyloid aggregation and prevent cytotoxicity. Biochemistry 2004, 43:9999-10007.
9. Maeda H, Steffen E, Engel J: Kinetics of dimerization of the Bence-Jones protein Au. Biophys Chem 1978, 9:57-64.
10. Ely KR, Herron JN, Harker M, Edmundson AB: Three-dimensional structure of a light chain dimer crystallized in water. Conformational flexibility of a molecule in two crystal forms. J Mol Biol 1989, 210:601-615.
11. Planque S, Bangale Y, Song XT, Karle S, Taguchi H, Poindexter B, Bick R, Edmundson A, Nishiyama Y, Paul S: Ontogeny of proteolytic immunity: IgM serine proteases. J Biol Chem 2004, 279:14024-14032.
12. Oleksyszyn J, Powers JC: Amino acid and peptide phosphonate derivatives as specific inhibitors of serine peptidases. Methods Enzymol 1994, 244:423-441.

### EXAMPLE 2: Catalytic Igs to Staphylococcus aureus antigens

***Polyclonal IgG-catalyzed hydrolysis of Efb.** S. aureus* possesses an arsenal of proteins dedicated to subverting mammalian adaptive and innate immune responses [1-3]. The potent immunmodulatory effects of these proteins facilitate evasion of host immune responses by the bacteria. Efb, a secreted protein interferes with complement function (complement-mediated lysis and opsonophagocytosis) [4,5], platelet function [6,7] and delays wound healing as tested in animal models [8]. Efb has also been implicated in bacterial adhesion to host cell surfaces, and conventional Igs that block the binding of Efb to fibrinogen and prevented Efb-mediated inhibition of platelet aggregation have been described [9].

In the present invention, polyclonal Ig preparations were purified from serum of 12 adult humans without clinical symptom of *S. aureus* infection at the time of blood donation (6 males and 6 females). Sera were subjected to affinity chromatography on immobilized anti-human IgM antibodies (for preparation of IgM) or immobilized anti-human IgA antibodies (for the preparation of IgA) as described [10,11]. This procedure yielded electrophoretically homogeneous IgM and IgA as determined by SDS-electrophoresis of Coomassie-stained gels and via Western blotting using antibodies specific to µ or α chain as described [10,11]. IgG was purified using immobilized Protein G as described previously [12]. Efb hydrolysis by individual Ig preparations was studied using biotinylated Efb (Bt-Efb; prepared from recombinant Efb expressed in *E. coli*; [4,13]) as substrate. All of the IgG, IgA and IgM from the 12 subjects degraded Bt-Efb, evident from disappearance of the parent 19 kDa band and appearance of three biotin-containing bands with nominal mass values of 14, 10 and 7 kDa, respectively (note that Bt-Efb contains only 1-2 moles biotin/mole protein, and the absence of biotin in certain degradation fragments may preclude their detection). IgG possessed the most efficient catalytic activity **(****Fig 12****).** Efb hydrolysis was observed at IgG concentrations as low as 0.1 µM. The activity levels varied widely in different human subjects (Efb hydrolysis ranging between 20-100% of the available Efb). Non-biotinylated Efb was also cleaved by IgG; The hydrolysis was time-dependent and readily noticeable as early as 2 hours after initiating the incubation **(****Fig 13****).** To identify the Efb peptide bonds susceptible to cleavage by IgG, we subjected the reaction products separated by electrophoresis to N-terminal sequencing. All of the bonds hydrolyzed by IgG contained a basic residue (Lys/Arg) at the PI position **(****Fig 14****).** One of the IgG-susceptible bonds is located in the C3b-binding region of Efb (Lys₁₀₀-Thr₁₀₁).

Under identical conditions, several non-*S. aureus* control proteins were not cleaved by IgG **(****Fig 15****).** Hydrolysis of the control proteins by the promiscuous serine protease trypsin was readily detectable. The absence of indiscriminate proteolytic activities in the IgG preparations suggests that trace protease contamination do not explain the observed Efb cleavage reaction. Moreover, we have subjected the IgG, IgA and IgM preparations to FPLC gel filtration in denaturing solvent (6 M guanidine hydrochloride; to remove any non-covalently-associated proteases) followed by dialysis against PBS (to renature the antibodies). The IgM (900 kDa), IgG (150 kDa) and IgA fractions (160 kDa) recovered from the column displayed the proteolytic activity using several model tripeptide substrates [10,11]. Fab fragments prepared from IgG and IgM have also been shown to retain the proteolytic activity, indicating that the catalytic site is located in the V domains.

Four classes of proteases are known: serine proteases, thiol proteases, acid proteases and metalloproteases. E-hapten **2** (structure shown in **Fig 11A**) contains an electrophilic phosphonate group that binds irreversibly to the catalytic site of serine proteases and thereby inhibits their catalytic activity. This compound was a potent inhibitor of IgG-catalyzed Efb hydrolysis **(****Fig 16****).** Inhibitors of metalloproteases (EDTA and 1,10-phenanthroline), cysteine-proteases (iodoacetamide), and acid-proteases (pepstatin) were not effective or marginally effective. Like other naturally occurring catalytic Igs, these results suggest that catalytic lgs to Efb use a serine protease-like mechanism of catalysis.

***Identification of Efb-hydrolysing IgVs.*** We tested Efb hydrolysis by purified IgV constructs from 42 randomly picked clones from our human IgV library (∼10⁷ clones). The majority of clones in this library (83 %) are scFv constructs mimicking the physiological structures of Ig combining sites. A minority (17%) of clones are IgVs with aberrant structures including IgV_{L2}-*t* and IgV_{L}-*t'* (see EXAMPLE 1). Efb-hydrolysis was studied by the SDS-electrophoresis method using the Bt-Efb substrate (0.1 µM) incubated with IgVs for 20 hours as described above. Blots of the gels were stained with streptavidin-peroxidase. Substrate consumption was measured by densitometry of the intact Bt-Efb band compared to the control incubation conducted in the absence of IgV under otherwise the identical conditions. IgVs displayed >20% cleavage were considered positive **(****Fig 17****).** The catalytic IgV clones identified were 1B4, 1B10, 1G2, 1G5, 2B4, 2B6, C6, 2C7, and 2E6 (range, 25-49%; median, 35%; mean, 35%; SD, 9%). The finding of recombinant IgVs with Efb hydrolyzing activity further validates the existence of the catalytic Igs to this protein in the expressed human repertoire.

Two catalytic clones (1C7, 1B4) were subjected to di-deoxy nucleotide sequencing, revealing one to be an scFv construct (clone 1C7) and the other to be a IgV_{L2}-*t* construct (1B4) **(****Figs 18** **and** **19****).** Comparison of the cDNA sequences of scFv 1C7 and IgV_{L2}-*t* 1B4 with their closest germline V gene counterparts revealed the presence of mutations in the V domains of both constructs.

***Catalytic neutralization of the S. aureus virulence factor Efb.*** Complement activation and deposition of C3b on the surface of *S. aureus* is important in the opsonophagocytic clearance of the bacteria. Efb interferes strongly with complement activation, impeding bacterial phagocytosis and aiding bacterial survival in the early stages of the infection before a protective capsule has been formed. In addition, Efb is an inhibitor of Ig-dependent complement-mediated lysis [4,13]. We determined the binding of complement component C3b by Efb-IgG reaction mixtures. Blots of the reaction mixtures were probed with digoxigenin-labeled C3b as described previously [13]. Efb incubated with purified catalytic IgG lost all C3b binding activity **(****Fig 20A****).** Moreover, Efb treated with a catalytic IgG preparation lost its ability to inhibit complement-dependent RBC lysis **(****Fig 20B****).** The data suggest that catalytic Igs neutralize Efb.

***Catalytic Igs to additional S. aureus virulence factors.** S. aureus* has evolved numerous polypeptide virulence factors that are important in establishment and progression of infection. The known virulence factors include proteins that facilitate bacterial adhere to extracellular matrix components and host cell surfaces; exert toxic effects on host cells and help the bacterium evade host immune defenses [1,2,14-18]. Of particular interest are the factors expressed by the USA300 isolate, which is responsible for most community-acquired infections in the U.S. [19].

The virulence factors can be classified as adhesions, toxic factors and immunomodulators. Adhesins are attractive targets of Igs for the purpose of impeding bacterial colonization [18]. Several bacterial adhesions are characterized by an LPXTG motif, such as Sdr family adhesins. The transpeptidases Sortase A and B covalently link the threonine of this sequence to cell wall-associated pentaglycine, anchoring the proteins to the cell wall. The important role of these adhesins is supported by evidence that *S. aureus* sortase mutants are impaired in the ability to cause acute lethal disease, abscess formation in internal organs, infectious arthritis and infectious endocarditis in mouse and rat models [20-22]. Additional important adhesins are Clumping factor A and B (ClfA, ClfB), which are structurally-related fibrinogen (Fg) binding proteins [23,24]. ClfB also binds cytokeratin 10 [25,26]. The fibronectin (Fn)-binding adhesins, FnbpA and FnbpB, also have a Fg-binding domain and compete with ClfA for Fg binding [27]. FnbpA overexpression in ClfA and ClfB deficient *S. aureus* permits bacterial-Fg interactions [27].

Panton Valentine Leukocidin (PVL), a bi-component exotoxin assembled from two polypeptides (LukS-PV and LukF-PV) is an important *S. aureus* toxin that forms pores in membranes and lyses various host cell types, including neutrophils and macrophages. PVL expressing isolates are thought to cause more severe infections compared to PVL-negative strains. A virulence factor role for PVL was described in a mouse pneumonia model [28]. *S. aureus* alpha-toxin (Hla) is a potent hemolytic, cytotoxic, and dermonecrotic toxin. It lyses erythrocytes, mononuclear cells, platelets, epithelial and endothelial cells [29]. Cell death occurs because of membrane damage [30,31]. Most *S. aureus* strains express alpha-toxin, but the level of expression can vary.

*S. aureus* possesses an arsenal of immunomodulatory proteins. One example is Efb described in the preceding section. Another is Map (also named Eap), a secreted protein that binds host surfaces and can also re-bind to the bacteria. It has profound effects on T cell function *in vitro* and in animal models [32,33]. It also reduces neutrophil migration [34] and induces proinflammatory cytokine production from PBMCs [35]. Protein A is secreted protein and is also found linked covalently to the cell wall. It binds the V domains of certain VH3 family Igs and is the prototypical B cell superantigen. Protein A acts directly on B cells, and by inducing apoptosis of VH3+ B cells, it causes dramatic changes in the expressed Ig repertoire [15,17]. Protein A also binds the Fc region of certain IgG subclasses.

We studied that the ability of Igs from adult humans with no evidence of clinical *S. aureus* infection to catalyze the hydrolysis of the following *S. aureus* virulence factors: Protein A, Map 19, ClfA, LukF and SdrE. Distinct levels of hydrolysis of these proteins by the IgM, IgA and IgG preparations was observed. IgA preparations tended to show greatest activity for all substrates. Example data are shown in **Fig 21****.** No noticeable hydrolysis of ClfB or LukS was evident at the Ig concentrations tested (150 µg/ml). Previous studies have reported the superantigenic character of Protein A with respect to Ig binding activity [16,17]. For our studies, the Fc-binding activity of Protein A was removed by treatment with iodine monochloride as described previously [15,36]. The resultant I-Protein A preparation is known to bind V_{H}3 family immunoglobulins via interactions at the V domains.

We also compared the hydrolysis of Map19 and SdrE by Igs from pooled sera from pediatric subjects without *S. aureus* disease and children diagnosed with deep-bone *S. aureus* infections **(****Fig 22****).** The Igs from children with deep-bone infections were poorly hydrolytic compared to disease-free children. For example, the Map19 hydrolysis activity levels of Igs from disease-free vs deep-bone infection were: IgG, 1.0 ± 0.2 vs <0.4; IgM, 6.9 ± 0.1 vs 0.5 ± 0.2; IgA, 6.7 ± 0.3 vs <0.4 nM/h/µg Ig.

These observations suggest that catalytic Igs to *S. aureus* virulence factors are widely distributed in humans. The reduction in catalytic Igs observed in individuals with bacterial disease is interesting, suggesting that an impaired defense due to the catalytic Igs may be a factor in progression of infection and development of clinical disease.

These results also suggest that Efb, Map19 and SdrE may have B cell superantigenic characters, i.e., they are recognized by catalytic Igs without requirement for antigen-specific stimulation. The reduction of catalytic Igs in children with clinical *S. aureus* is consistent with findings that synthesis of Igs to superantigens is decreased following exposure to the superantigen. In the present inventions, we obtained additional support for the superantigenicity of certain *S. aureus* proteins by examination of pooled Igs from mice maintained in a pathogen-free facility. Efb was cleaved at readily detectable levels by all class of Igs from the mice (IgG 5.6 ± 1.1, IgM 5.9 ± 1.3, IgA 32.7 ± 1.3 nM/h/µg Ig). Following experimental *S. aureus* infection, IgG preparations from the mice displayed undetectable Efb-cleaving activity (<3.7 nM/h/µg Ig) and IgA displayed reduced Efb-cleaving activity (6.9 ± 1.5 nM/h/µg Ig). The IgM activity was maintained at nearly unchanged levels (5.1 ± 0.1 nM/h/µg IgM).

### Reference for EXAMPLE 2

1. Lowy FD: Staphylococcus aureus infections. N Engl J Med 1998, 339:520-532.
2. Rooijakkers SH, van Kessel KP, van Strijp JA: Staphylococcal innate immune evasion. Trends Microbiol 2005, 13:596-601.
3. Foster TJ: Immune evasion by staphylococci. Nat Rev Microbiol 2005, 3:948-958.
4. Lee LY, Hook M, Haviland D, Wetsel RA, Yonter EO, Syribeys P, Vernachio J, Brown EL: Inhibition of complement activation by a secreted Staphylococcus aureus protein. J Infect Dis 2004, 190:571-579.
5. Rooijakkers SH, van Strijp JA: Bacterial complement evasion. Mol Immunol 2007, 44:23-32.
6. Shannon O, Flock JI: Extracellular fibrinogen binding protein, Efb, from Staphylococcus aureus binds to platelets and inhibits platelet aggregation. Thromb Haemost 2004, 91:779-789.
7. Palma M, Shannon O, Quezada HC, Berg A, Flock JI: Extracellular fibrinogen-binding protein, Efb, from Staphylococcus aureus blocks platelet aggregation due to its binding to the alpha-chain. J Biol Chem 2001, 276:31691-31697.
8. Shannon O, Uekotter A, Flock JI: Extracellular fibrinogen binding protein, Efb, from Staphylococcus aureus as an antiplatelet agent in vivo. Thromb Haemost 2005, 93:927-931.
9. Shannon O, Uekotter A, Flock JI: The neutralizing effects of hyperimmune antibodies against extracellular fibrinogen-binding protein, Efb, from Staphylococcus aureus. Scand J Immunol 2006, 63:184-190.
10. Planque S, Bangale Y, Song XT, Karle S, Taguchi H, Poindexter B, Bick R, Edmundson A, Nishiyama Y, Paul S: Ontogeny of proteolytic immunity: IgM serine proteases. J Biol Chem 2004, 279:14024-14032.
11. Mitsuda Y, Planque S, Hara M, Kyle R, Taguchi H, Nishiyama Y, Paul S: Naturally occurring catalytic antibodies: evidence for preferred development of the catalytic function in IgA class antibodies. Mol Biotechnol 2007, 36:113-122.
12. Kalaga R, Li L, O'Dell JR, Paul S: Unexpected presence of polyreactive catalytic antibodies in IgG from unimmunized donors and decreased levels in rheumatoid arthritis. J Immunol 1995, 155:2695-2702.
13. Lee LY, Liang X, Hook M, Brown EL: Identification and characterization of the C3 binding domain of the Staphylococcus aureus extracellular fibrinogen-binding protein (Efb). J Biol Chem 2004, 279:50710-50716.
14. Patti JM, Allen BL, McGavin MJ, Hook M: MSCRAMM-mediated adherence of microorganisms to host tissues. Annu Rev Microbiol 1994, 48:585-617.
15. Silverman GJ, Sasano M, Wormsley SB: Age-associated changes in binding of human B lymphocytes to a VH3-restricted unconventional bacterial antigen. J Immunol 1993, 151:5840-5855.
16. Goodyear CS, Silverman GJ: Staphylococcal toxin induced preferential and prolonged in vivo deletion of innate-like B lymphocytes. Proc Natl Acad Sci U S A 2004, 101:11392-11397.
17. Goodyear CS, Silverman GJ: Death by a B cell superantigen: In vivo VH-targeted apoptotic supraclonal B cell deletion by a Staphylococcal Toxin. J Exp Med 2003, 197:1125-1139.
18. Rivas JM, Speziale P, Patti JM, Hook M: MSCRAMM--targeted vaccines and immunotherapy for staphylococcal infection. Curr Opin Drug Discov Devel 2004, 7:223-227.
19. Klevens RM, Morrison MA, Nadle J, Petit S, Gershman K, Ray S, Harrison LH, Lynfield R, Dumyati G, Townes JM, et al.: Invasive methicillin-resistant Staphylococcus aureus infections in the United States. Jama 2007, 298:1763-1771.
20. Weiss WJ, Lenoy E, Murphy T, Tardio L, Burgio P, Projan SJ, Schneewind O, Alksne L: Effect of srtA and srtB gene expression on the virulence of Staphylococcus aureus in animal models of infection. J Antimicrob Chemother 2004, 53:480-486.
21. Mazmanian SK, Liu G, Jensen ER, Lenoy E, Schneewind O: Staphylococcus aureus sortase mutants defective in the display of surface proteins and in the pathogenesis of animal infections. Proc Natl Acad Sci USA 2000, 97:5510-5515.
22. Jonsson IM, Mazmanian SK, Schneewind O, Bremell T, Tarkowski A: The role of Staphylococcus aureus sortase A and sortase B in murine arthritis. Microbes Infect 2003, 5:775-780.
23. Walsh EJ, Miajlovic H, Gorkun OV, Foster TJ: Identification of the Staphylococcus aureus MSCRAMM clumping factor B (ClfB) binding site in the alphaC-domain of human fibrinogen. Microbiology 2008, 154:550-558.
24. McDevitt D, Nanavaty T, House-Pompeo K, Bell E, Turner N, McIntire L, Foster T, Hook M: Characterization of the interaction between the Staphylococcus aureus clumping factor (ClfA) and fibrinogen. Eur J Biochem 1997, 247:416-424.
25. Walsh EJ, O'Brien LM, Liang X, Hook M, Foster TJ: Clumping factor B, a fibrinogen-binding MSCRAMM (microbial surface components recognizing adhesive matrix molecules) adhesin of Staphylococcus aureus, also binds to the tail region of type I cytokeratin 10. J Biol Chem 2004, 279:50691-50699.
26. O'Brien LM, Walsh EJ, Massey RC, Peacock SJ, Foster TJ: Staphylococcus aureus clumping factor B (ClfB) promotes adherence to human type I cytokeratin 10: implications for nasal colonization. Cell Microbiol 2002, 4:759-770.
27. Wann ER, Gurusiddappa S, Hook M: The fibronectin-binding MSCRAMM FnbpA of Staphylococcus aureus is a bifunctional protein that also binds to fibrinogen. J Biol Chem 2000, 275:13863-13871.
28. Labandeira-Rey M, Couzon F, Boisset S, Brown EL, Bes M, Benito Y, Barbu EM, Vazquez V, Hook M, Etienne J, et al.: Staphylococcus aureus Panton-Valentine leukocidin causes necrotizing pneumonia. Science 2007, 315:1130-1133.
29. Ward PD, Adlam C, McCartney AC, Arbuthnott JP, Thorley CM: A histopathological study of the effects of highly purified staphlococcal alpha and beta toxins on the lactating mammary gland and skin of the rabbit. J Comp Pathol 1979, 89:169-177.
30. Essmann F, Bantel H, Totzke G, Engels IH, Sinha B, Schulze-Osthoff K, Janicke RU: Staphylococcus aureus alpha-toxin-induced cell death: predominant necrosis despite apoptotic caspase activation. Cell Death Differ 2003, 10:1260-1272.
31. Jonas D, Walev I, Berger T, Liebetrau M, Palmer M, Bhakdi S: Novel path to apoptosis: small transmembrane pores created by staphylococcal alpha-toxin in T lymphocytes evoke internucleosomal DNA degradation. Infect Immun 1994, 62: 1304-1312.
32. Lee LY, Miyamoto YJ, McIntyre BW, Hook M, McCrea KW, McDevitt D, Brown EL: The Staphylococcus aureus Map protein is an immunomodulator that interferes with T cell-mediated responses. J Clin Invest 2002, 110:1461-1471.
33. Xie C, Alcaide P, Geisbrecht BV, Schneider D, Herrmann M, Preissner KT, Luscinskas FW, Chavakis T: Suppression of experimental autoimmune encephalomyelitis by extracellular adherence protein of Staphylococcus aureus. J Exp Med 2006, 203:985-994.
34. Chavakis T, Hussain M, Kanse SM, Peters G, Bretzel RG, Flock JI, Herrmann M, Preissner KT: Staphylococcus aureus extracellular adherence protein serves as anti-inflammatory factor by inhibiting the recruitment of host leukocytes. Nat Med 2002, 8:687-693.
35. Scriba TJ, Sierro S, Brown EL, Phillips RE, Sewell AK, Massey RC: The Staphyloccous aureus Eap protein activates expression of proinflammatory cytokines. Infect Immun 2008, 76:2164-2168.
36. Paul S, Karle S, Planque S, Taguchi H, Salas M, Nishiyama Y, Handy B, Hunter R, Edmundson A, Hanson C: Naturally occurring proteolytic antibodies: selective immunoglobulin M-catalyzed hydrolysis of HIV gp120. J Biol Chem 2004, 279:39611-39619.

### EXAMPLE 3: Polyclonal catalytic IgMs to hepatitis C virus coat protein E2

*Hepatitis C virus (HCV).* The E2 coat protein expressed by HCV is thought to be essential for viral infection by virtue of its role in host cell binding [1]. Proposed cellular receptors for HCV E2 are CD81 and the LDL receptor. E2 contains hypervariable regions and comparatively conserved regions. Igs to the hypervariable regions are frequent in infected individuals [2]. Conventional non-catalytic Igs to E2 have been suggested to be important in control of virus infection [2]. Certain monoclonal Igs to E2 neutralize the virus and are under consideration for therapy of HCV infection [3].

A pooled Ig preparation consisting of IgM class antibody was obtained from serum of 10 human subjects with asymptomatic HCV infection. These subjects were positive for the NS3, NS4 HCV antigen determined by a commercially available kit (Abbott laboratories). Sera were subjected to affinity chromatography on immobilized anti-human IgM antibody as described previously [4]. The Ig preparation obtained was electrophoretically homogeneous as determined by SDS-electrophoresis of Coomassie-stained gels and immunoblotting using anti-µ antibodies. HCV E2 hydrolysis by the Ig preparation was studied using two recombinant E2 preparations as substrates: commercially available E2 containing the 26 kD glutathione-S-transferase tag (E2-GST; baculovirus expression system), and recombinant E2 containing the 8-residue Flag tag (E2-FL; expressed in HEK293 cells; see ref [5]). The latter substrate was purified partially by affinity chromatography using immobilized anti-FL antibody by methods known to one skilled in the art. Hydrolysis of these substrates was determined by SDS-electrophoresis followed by staining of the gels with commercially available anti-GST antibody, anti-FL antibody or anti-E2 antibody.

The pooled IgM from HCV+ subjects hydrolyzed E2-GST, evident from time-dependent disappearance of the intact substrate band and appearance of the multiple anti-GST stainable bands with mass values smaller than E2-GST **(****Fig 23A****).** IgM cleaved E2-FL also, determined by anti-FL staining or anti-E2 staining of the gels **(****Fig 23B****).**

Next, the E2 hydrolyzing activities of IgM preparations obtained from individual serum samples from HCV-infected (n=10) and uninfected (n=9) subjects were determined. The hydrolytic activity was expressed as the decrease in the intact E2-GST or E2-FL band intensity determined by densitometry using the diluent control as reference (E2-GST or E2-FL incubated in the absence of IgM). The E2 hydrolyzing activity of IgM preparations from the HCV+ subjects was greater than the HCV negative subjects using E2-GST or E2-FL as the substrates **(****Fig 23C****).** Widely varying levels of catalytic activity were evident in different donors within the two groups of subjects.

To validate the catalytic activity, we tested the hydrolysis of E2-GST by 9 monoclonal IgM preparations obtained from humans with Waldenström's macroglobulinemia as described in our previous publication [6]. Several IgMs displayed detectable E2-GST hydrolyzing activity **(****Fig 24****).**

Apparent kinetic constants for the pooled IgM from HCV+ subjects were determined at varying concentrations of E2-GST **(****Fig 25****).** From the fit of the rate data to the Michaelis-Menten equation, values of Km and maximal velocity (Vmax) were, respectively, 684 nM and 1.3 x 10⁻² moles E2-GST/mole IgM/min.

Homogeneous recombinant IgVs to E2 can be readily obtained by covalent selection of the phage displayed IgV libraries using an electrophilic E2 analog as demonstrated for other antigens in EXAMPLES 1 and 4. For this purpose, we have prepared an electrophilic analog of biotinylated E2-GST (E-E2-GST). The E-E2-GST was obtained by successive acylation of exposed amino groups with N-hydroxysuccinimide esters of 6-biotinamidohexanoic acid and diphenyl N-suberoylamino(4-amidinophenyl)methane-phosphonate, followed by gel filtration purification at each step by methods described in refs [7,8]. MALDI-TOF MS of the biotinylated E2-GST, E-E2-GST product and the starting E2-GST protein indicated a mass increase of 700.4 for the biotin containing protein and 6900.1 for the phosphonate containing protein, corresponding to 2 biotin and 13 phosphonate groups per E2-GST molecule **(****Fig 26****).**

### References for EXAMPLE 3

1. Barth H, Liang TJ, Baumert TF: Hepatitis C virus entry: molecular biology and clinical implications. Hepatology 2006, 44:527-535.
2. Zeisel MB, Fafi-Kremer S, Fofana I, Barth H, Stoll-Keller F, Doffoel M, Baumert TF: Neutralizing antibodies in hepatitis C virus infection. World J Gastroenterol 2007, 13:4824-4830.
3. Galun E, Terrault NA, Eren R, Zauberman A, Nussbaum O, Terkieltaub D, Zohar M, Buchnik R, Ackerman Z, Safadi R, et al.: Clinical evaluation (Phase I) of a human monoclonal antibody against hepatitis C virus: safety and antiviral activity. J Hepatol 2007, 46:37-44.
4. Planque S, Bangale Y, Song XT, Karle S, Taguchi H, Poindexter B, Bick R, Edmundson A, Nishiyama Y, Paul S: Ontogeny of proteolytic immunity: IgM serine proteases. J Biol Chem 2004, 279:14024-14032.
5. Cocquerel L, Kuo CC, Dubuisson J, Levy S: CD81-dependent binding of hepatitis C virus E1E2 heterodimers. J Virol 2003, 77:10677-10683.
6. Paul S, Karle S, Planque S, Taguchi H, Salas M, Nishiyama Y, Handy B, Hunter R, Edmundson A, Hanson C: Naturally occurring proteolytic antibodies: selective immunoglobulin M-catalyzed hydrolysis of HIV gp120. J Biol Chem 2004, 279:39611-39619.
7. Planque S, Taguchi H, Burr G, Bhatia G, Karle S, Zhou YX, Nishiyama Y, Paul S: Broadly distributed chemical reactivity of natural antibodies expressed in coordination with specific antigen binding activity. J Biol Chem 2003, 278:20436-20443.
8. Paul S, Planque S, Zhou YX, Taguchi H, Bhatia G, Karle S, Hanson C, Nishiyama Y: Specific HIV gp120-cleaving antibodies induced by covalently reactive analog of gp120. J Biol Chem 2003, 278:20429-20435.

### EXAMPLE 4: Catalytic and neutralizing Igs to HIV gp120

***Neutralizing Igs from non-infected humans.*** Over 33 million humans are infected with HIV. No effective vaccine for HIV is available. Progression to AIDS occurs at variable rates in infected subjects. Without treatment, -50% of infected subjects die in 10 years [1]. There is consensus that the variable rates of disease progression derive at least in part from discrete immunological factors. Innate and adaptive immune responses help protect against the virus (e.g., [2-4]). Knowledge of resistance factors to HIV can help guide improved treatment and development of a preventive vaccine.

Effective control of HIV by the immune system is thwarted by: ***(a)*** rapid sequence diversification of the immunogenic epitopes in its coat protein gp120; and ***(b)*** the lack of robust adaptive responses to conserved epitopes of viral protein important in virus-host cell interactions. The immune system generally fails to produce Igs to conserved gp120 epitopes with sufficient neutralization potency and breadth to control HIV fully. Ig epitope specificity is an important property governing neutralization. Although rare, neutralizing monoclonal antibodies (MAbs) to gp120 and gp41 have been identified. The best known are: MAb b12 directed to a gp120 determinant overlapping the CD4bs [5]; MAb 2G12 to a mannose-dependent gp120 epitope [6,7]; and MAb 2F5 to a gp41 epitope involved in forming the fusogenic gp41 intermediate [8]. These MAbs neutralize many clade B strains and they protect macaques against challenge with clade B SHIV strains (SIV engineered to express HIV *env*) [9-11]. However, they are often ineffective against HIV belonging to other clades, *e.g.,* clade C strains responsible for >50% of all infections [9]. Igs with epitope specificity similar to MAbs b12, 2G12 or 2F5 are usually not detected in chronically infected HIV subjects. Most Igs in infected individuals are directed to epitopes that mutate rapidly or are inaccessible sterically, allowing infection to progress. Often, the Igs recognize the immunodominant, hypermutable epitopes located within V3 residues 306-325 [12,13]. These Igs usually do not neutralize CCR5-dependent strains or strains with divergent V3 sequences.

gp120 contains a B cell superantigen site recognized by preimmune Igs without requirement for adaptive B cell maturation [14]. B cell superantigen recognition is mediated mostly by contacts at Ig V domain FR residues [15,16]. Most conventional Ig-antigen contacts, in contrast, occur at the CDRs. Peptide mapping studies suggest that the gp120 superantigen site is discontinuous determinant, composed of residues 241-250, 341-350 and 421-440 [17].

We observed that IgMs [18] and IgAs [19] from non-infected humans catalyze the hydrolysis of gp120. From initial rate data, the average turnover number (*k*_{cat}) for a polyclonal IgM preparation was 2.8/min. Salivary IgAs, and to a lesser extent, serum IgAs from uninfected humans hydrolyzed gp120 **(****Fig 27A,B****).** IgGs did not. Neutralization potency was modest, determined by p24 enzymeimmunoassay using peripheral blood mononuclear host cells. HIV strain ZA009 was neutralized by treatment with salivary IgA for 1 h **(****Fig 27C****).** Neutralization of the virus by treatment with serum IgA was evident only after prolonged incubation (24 h; not shown), and there was little or no neutralization at the 1 h time point [19]. IgG was ineffective at both time points studies. Thus, the neutralizing activity follows the pattern of the catalytic activity: salivary IgA>serum IgA>>serum IgG.

Specificity was evident from lack of hydrolysis of albumin, soluble epidermal growth factor receptor, FVIII C2 domain and Tat. The catalytic reactions were inhibited completely by an electrophilic analog of gp120 residues 421-433 (E-421-433) [18,19]. IgMs and IgAs formed covalent adducts with E-421-433 stable to boiling and SDS **(****Fig 27B****).** Similarly, viral neutralization was inhibited by E-421-433 [19]. By N-terminal aa sequencing, the major cleavage site was the 432-433 bond in the SAg site [18,19]. Salivary sIgA also hydrolyzed the V83-E84 and Y321-T322 bonds. These observations indicate a nucleophilic catalytic mechanism in which noncovalent 421-433 recognition permits selective gp120 hydrolysis. We and others have previously reported hydrolysis of multiple bonds in polypeptide Ags by Igs, some distant from the binding epitope [20,21]. The observations are consistent with a split-site model involving distinct subsites responsible for noncovalent binding and catalysis [22]. The catalytic subsite fails to make stable contacts with the Ag in the noncovalent immune complex. As the transition state develops, different peptide bonds become positioned in register with the catalytic site. When the Ab recognizes a conformational epitope, the alternate cleavage sites must be spatial neighbors, but they can be distant in the linear sequence, producing a complex fragmentation pattern.

***Neutralizing Igs from long-term survivors of HIV infection.*** Adaptive synthesis of specific Igs to microbial antigens usually entails sequence diversification at the CDRs of the B cell receptor (BCR), guided by selection pressures enabling proliferation of B cells with the highest affinity BCRs. Adaptive improvement of Igs to superantigenic epitopes is thought to be proscribed by B cell down-regulatory signals generated upon superantigen interactions at BCR FRs [23].

We studied IgA preparations from the blood of three subjects who have survived for 18-20 years following infection by presumptive clade B HIV strains despite little or no anti-retroviral therapy (designated survivor "S₁₈" subjects; CD4 cell counts 178-426/µl). Serum IgA preparations from the S₁₈ subjects displayed readily detectable gp120 hydrolyzing activity. Identical fragmentation profiles were observed by treating biotinylated gp120 with IgA from HIV infected and non-infected humans. Diverse clade B and heterologous clade C strains were neutralized with exceptional potency by the S₁₈ IgAs (**Fig 28** and **Table 1;** 1 h incubation with virus; n=11 R5-dependent strains). The immunodominant V3 306-325 epitope is highly variable in these strains and the 421-433 epitope is mostly conserved (see example sequences, **Fig 28**). In comparison, the reference MAb commonly cited as a broad neutralizing Ig, clone b12, did not neutralize many strains, and when neutralization was evident, the potency was substantially lower **(Table 1).** The neutralizing potency of S₁₈ IgAs was about 3 orders of magnitude superior to IgAs from uninfected subjects and IgAs obtained 1-5 years after seroconversion from subjects who succumbed to AIDS (designated non-survivors "NS₅" subjects; CD4⁺ T cell counts <200/µl; n=10) or survived without development of AIDS (S₅ subjects, CD4⁺ T cell counts >200/µl; n=10) **(****Fig 29****).** gp120 binding by IgAs from S₁₈, NS₅ and S₅ subjects was comparable, indicating similar conventional antibody activity. The restricted presence of potently neutralizing IgAs in very late infection suggests an unconventional adaptive response. In addition to binding to the immunodominant V3 epitope, increased binding of a non-hydrolyzable electrophilic analog of gp120 residues 421-433 by S₁₈ IgAs was evident. These observations suggest that survivors with prolonged HIV infection mount a late adaptive IgA response to gp120 with properties that can slow progression to AIDS.

**Table 1. Heterologous clade C HIV and diverse clade B strain neutralization by IgAs from 3 S₁₈ subjects. Clade C strains: 97ZA009, 98TZ013, 98TZ017, Du123, Du156, Du172, Du422. Clade B strains: ADA, PAVO and QH0692. All CCR5-dependent. PBMC hosts, p24 assays. Dose-dependent HIV neutralization was evident in all assays.**

| | Number of strains neutralized/Number of strains tested (IC₅₀ range; mean ± S.D, µg/ml) | |
|---|---|---|
| | clade B | clade C |
| IgA 2857 | 3/3 (0.08-0.20; 0.12 ± 0.07) | 6/6 (0.01-2.70; 0.9 ± 1.3) |
| IgA 2866 | 3/3 (0.002-1.000; 0.37 ± 0.55) | 7/7 (0.008-1.960; 0.5 ± 1.0) |
| IgA 2886 | 3/3 (0.20-0.80; 0.53 ± 0.33) | 7/7 (0.10-10.70; 2.4 ± 3.8) |
| IgG B12 | 3/4 (0.90-10.00; 3.96 ± 5.22) | 3/8 (4.10-10.50; 6.1 ± 3.5) |

***Ig L chain subunits directed to gp120 residues 421-433.*** Previously, increased polyclonal Igs that bind the 421-436 region were observed in lupus sera [24]. We and others reported increased catalytic Igs in autoimmune diseases. Several reports have noted that HIV infection is infrequent in lupus patients [25-29]. The mechanism of increased Igs to the 421-433 region in lupus is not clear. One possibility is Ig synthesis driven by a homologous antigen. By searching the sequence databases, we found nucleotide sequence homology between the 421-433 region and a human endogenous retroviral sequence (HERV) [30]. HERV expression is increased in lupus [31].

To isolate nucleophilic antibody L chain subunits that recognize gp120 residues 421-433, we prepared a phage-displayed light chain library from lupus patients. The library was fractionated by the following two methods: (a) a two step procedure entailing covalent selection using an electrophilic hapten as described in [32] followed by non-covalent selection using the peptide corresponding to gp120 residues 421-436; and (b) a one step, affinity-driven covalent selection using the electrophilic analog of gp120 421-433 containing the phosphonate group at the C-terminus (E-421-433; **Fig 30A**). Procedures for preparation of the phage light chain library and E-421-433 were described previously [32,33]. Selection procedures were essentially as in [32] and [30]. For example, E-421-433 complexed phages were captured with streptavidin-agarose, the noncovalently bound phages were removed by acid washing (pH 2.7), and covalently bound phages were eluted by 2-mercaptoethanol reduction of the S-S group in the E-421-433 linker. Covalently selected light chains were expressed in soluble form and purified by metal affinity chromatography.

Thirty one L chains selected using the 421-436 peptide and 22 L chains selected using E-421-433 were studied for gp120 hydrolyzing activity. The proteolysis assay was conducted using biotinylated gp120 (Bt-gp120; prepared from recombinant gp120 of MN strain; baculovirus expression system) as substrate followed by SDS-electrophoresis and densitometry of streptavidin-peroxidase stained blots. The group of L chains selected using E-421-433 displayed significantly greater proteolytic activity than the 421-436 selected L chains (**Fig 30B****;** mean ± SE values, respectively, 1098 ± 157 vs 369 ± 56 AVU; P<0.0001, unpaired two-tailed t-test). This result indicates that, although both groups contain 421-433-directed nucleophilic antibody fragments, the E-421-433 selection can afford more efficient enrichment of the L chains compared to the two-step selection.

Two L chains each from the 421-436 selected group (clones SK18 and SK45) and the E-421-433 selected group (clones SK2C2 and SK2F5) were assessed for their ability to neutralize a clade C HIV primary isolate (97ZA009) using peripheral blood mononuclear cells (PBMCs) as hosts. All four light chains neutralized this HIV isolate (IC₅₀/IC₈₀ values: SK18, 0.6/2.1; SK45, 0.6/3.9; SK2C2, <0.01/0.13; SK2F5, <0.01/0.07 µg/mL). The cDNAs encoding the 421-436-selected L chains SK18 and SK45 and E-421-433 selected L chain SKL6 were sequenced. Their deduced V_{L} domain amino acid sequences are shown in **Fig 31****.**

***IgV fragments isolated by covalent phage selection with gp120 and E-gp120.*** To isolate nucleophilic IgV fragments, the phage-displayed IgV library derived from lupus patients was fractionated for binding to gp120 or E-gp120 **(****Fig 32A****).** Procedures for preparation of the phage IgV library and E-gp120 were described previously [32,34]. As described in EXAMPLE 1, a majority of the clones in this library are scFv constructs mimicking the physiological structures of Ig combining sites. The remaining clones are IgVs with unnatural structures, including IgV_{L2}-*t* and IgV_{L}-*t'* structures. IgV-displaying phages were packaged from the library and subjected to noncovalent selection by affinity chromatography on recombinant gp120 (immobilized on Biorad Affigel-10). Phages from the acid eluate were then subjected to covalent selection with biotinylated E-gp120. E-gp120 complexed phages were captured with immobilized anti-biotin IgG, and following extensive washing with a neutral buffer, bound phages were allowed to elute using the pH 2.7 buffer. The gp120 selected and E-gp120 selected IgVs were expressed in soluble form and purified by metal affinity chromatography.

Sixty two IgVs obtained by E-gp120 selection were assayed for gp120 hydrolysis activity along with a random collection of 61 IgVs from the source library obtained without selection. The proteolysis assay was conducted using biotinylated gp120 as in the preceding section. The E-gp120 selected group displayed significantly greater proteolytic activity than the unselected group (**Fig 32B****;** mean ± SE intensity of 55kD product band generated by the E-gp120 selected group vs unselected group, 1027 ± 167 vs 196 ± 58 AVU; P<0.0001, unpaired two-tailed t-test). Examples of gp120 hydrolyzing IgVs obtained by E-gp120 selection (clones GL2 and GL59) are shown in **Fig 33A****.**

About 50% of the IgV clones selected using gp120 and 100% of the clones selected using E-gp120 neutralized the clade C strain ZA009 using PBMC hosts **(Table 2).** The neutralizing potency of the E-gp120 selected clones was markedly superior to the gp120 selected clones, suggesting the functional advantage gained due to greater nucleophilicity **(Table 2).** The neutralizing potency of example IgV clones GL2, GL59 and JL427 are shown in **Fig 33B****.** scFv 610 in this figure is a control non-neutralizing clone, and IgG b12 is a leading monoclonal IgG commonly cited in the literature as a broadly neutralizing IgG. Neutralization by the IgV clones was inhibited by treatment with E-421-433 but not the irrelevant E-VIP **(****Fig 33C****).**

**Table 2. HIV neutralization (clade C, R5, ZA009) by lupus scFv fragments selected using gp120 or E-gp120. The characteristics of the lupus library are as in ref [32]. The scFv were purified by Ni-NTA chromatography and tested for neutralization using ZA009 (clade C, R5) as in ref [35].**

| *Selecting Antigen* | # *of clones, tested* | # *of clones, neutralized* | *Potency (EC_{50,} µg*/*ml)* | | |
|---|---|---|---|---|---|
| | | | 0.25-2.5 | 0.025-0.25 | 0.0025-0.025 |
| gp120 | 52 | 25 | 17 | 8 | 0 |
| E-gp120 | 14 | 14 | 0 | 6 | 8 |

By nucleotide sequencing, the E-gp120 selected IgVs GL2 and GL59 were identified as scFv constructs that mimic the structure of physiological Ig combining sites. The gp120 selected IgV clones JL606, JL678 and JL427 were also scFvs. As in the E-Aβ selection studies (EXAMPLE 1), certain selected IgV clones contained aberrant structures. These are the heterodimeric IgV_{L2} clone GL1, the single domain IgV_{H}-*t* clone JL683 without a VL domain partner but with the expected tag at the C terminus, and the single domain IgV_{L}-*t'* clone JL651 containing a VH domain with a large internal deletion. Tag *t* refers to the 27 residue c-myc/his6 sequence at the C terminus, and the designation *t'* is used to denote the unexpected VH peptide with tag *t* at the C terminus in IgV_{L}-*t'* JL651. Schematic representations of the structures and amino acid sequences of these clones are in **Fig 34****.** The HIV neutralizing potency of these clones is shown in **Table 3.**

**Table 3. HIV neutralization potency of E-gp120-selected and gp120-selected IgVs. HIV, 97ZA009; host, PBMC. IC₅₀ values are in ng/mL units, and mean ± SEM are shown for clones tested in multiple independent assays (numbers in parenthesis represents the number of assays).**

| | E-gp120 selected | | | gp120 selected | | | | |
|---|---|---|---|---|---|---|---|---|
| | scFv GL2 | scFv GL59 | IgV_{L2}-*t* GL1 | IgV_{H-t} JL683 | IgV_{L-t'} JL651 | scFv JL606 | scFv JL678 | scFv JL427 |
| IC₅₀, ng/mL | 8.8 ± 5.3 (3) | 2.5 ± 0.9 (6) | 8.1 ± 5.8 (2) | 3.7 | 29.3 | 82.5 | 141.0 | 300 ± 350 (24) |

scFv clones GL2 and JL427 neutralized diverse R5-dependent HIV strains (clades A, B, C; **Table 4**). Another intriguing finding is that scFv clone JL427 neutralized primary HIV strains in the PBMC assay strains but not the corresponding pseudovirions assayed in reporter cell lines. This implies that the 421-433 region may be expressed on native virions and pseudovirions in Ig-recognizable and non-recognizable conformations, respectively. Another Ig that neutralizes primary HIV isolates but not pseudovirions is reported [36]. Importantly, a recent study indicates that neutralization of primary HIV strains by Igs from certain infected subjects correlates with lack of progression whereas neutralization of pseudovirions does not [37]. For these reasons, it is appropriate to rely on infection assays using primary HIV isolates. In these assays, the 421-433 recognizing Igs surpass the potency and breadth of neutralization of other known Igs.

**Table 4: Cross-clade HIV neutralization by lupus scFvs GL2 and JL427. PBMC assays. HIV strains (clade/coreceptor strain names): A/R5 92RW008; A/X4 92USNG17; B/R5 SF162, 92BR014, BAL, PVO, TRO, ADA; B/X4 92HT599, Tybe; B/R5X4 BZ167, 92BR014; C/R5 98BR004, 97ZA009, 98TZ013, 98TZ017, Du123, Du422, Du172;D/R5 92UG266; D/X4 92UG001, 92UG266, 92UG046. Dose dependent HIV neutralization was evident in all cases.**

| HIV clade | Number of strains neutralized/Number of strains tested (IC50 range, mean ± S.D.; µg/ml) | |
|---|---|---|
| | scFv GL2 | scFv JL427 |
| A | 2/2 (0.8-4.2; 2.5 ± 2.4) | 1/1 (0.2; N/A) |
| B | 5/6 (0.007-3.7; 1.5 ± 1.9) | 6/8 (0.03-5.1; 1.3 ± 2.0) |
| C | 5/5 (0.015-2.7; 0.9 ± 1.3) | 6/6 (0.007-4.8; 0.9 ± 1.9) |
| D | 0/3 | 0/4 |

***IgV FR*/*CDR swapping.*** Antigen-specific Igs are usually synthesized adaptively by mutations occurring in the CDRs. In comparison, the superantigenic site of gp120 is thought to be recognized by Igs via contacts at conserved residues located mainly in VH3 domain FR1 and FR3 with additional contributions provided by CDR 1 and CDR2 [15,16]. Conventional Igs that bind the gp120 superantigenic site predominantly utilize VH3 family genes [14], suggesting that conserved VH3 family gene elements preferentially recognize the superantigenic site by noncovalent means. Replacement of the individual FRs and CDRs in a gp120 binding VH3 family Fab by the corresponding FRs and CDRs of a non-VH3 family, non-gp120 binding Fab resulted in loss of the gp120 binding activity of the former Fab [15].

The present invention discloses FR and CDR swapping as a novel means to improve recognition of the gp120 superantigenic site by Igs. The neutralizing scFv GL2 contains a VH4 family VH domain and the neutralizing scFv JL427 contains a VH3 family VH domain. FR1, FR3, CDR1 or CDR2 of the scFv GL2 VH domain were replaced individually by the corresponding regions of the VH3 family scFv JL427. This was done by a PCR-based substitution method using mutagenic primers coding for the aforementioned scFv JL427 regions and scFv GL2 in pHEN2 plasmid as template. PCR products were treated with T4 polynucleotide kinase, *Dpn*I to remove the methylated template DNA, and the purified mutant DNA was circularized by ligation. The DNA was sequenced to confirm the presence of the desired mutations, and mutant scFv mutants were expressed and purified by metal affinity chromatography as described [32]. **Fig 35** shows a schematic representation of the mutants and their sequences. The mutants are designated GL2-FR1m, GL2-FR3m, GL2-CDR1m and GL2-CDR2m, wherein GL2 denotes the scFv GL2 containing FR1, FR3, CDR1 and CDR3 from scFv JL427.

ELISAs using plates coated with E-gp120 or KLH conjugated to E-416-433 were employed to determine recognition of the gp120 superantigenic site. Preparation of E-gp120 and study of its binding by the scFv constructs was as in [34]. Preparation of E-416-433 was as in [38]. This peptide analog displays improved reactivity to Igs that recognize the gp120 superantigenic site compared to E-421-433, a finding consistent with the report that inclusion of the N terminal 416-420 residues induces a conformational change in the 421-433 region [39]. E-416-433 contains phosphonates at Lys422 and Lys432. Its identity was verified by electrospray ionization-mass spectrometry. Conjugation of the peptide analog to KLH was as in [40]. The nonhydrolyzable E-antigen probes were employed (instead of unmodified gp120 and unmodified 416-433 peptide) to preclude possible hydrolysis by scFv GL2, which has the ability to catalyze gp120 degradation.

The mutant scFv constructs GL2-FR1m, GL2-FR3m displayed markedly increased binding to E-gp120 and E-416-433 compared to wildtype (unmutated) scFv GL2 (Fig 36). These mutants also displayed increased binding to the two antigenic probes compared to the scFv JL427. scFv JL427 contains the full-length complement of gp120 superantigen binding expressed by a full-length VH3 domain, whereas the mutants contain only small fragments derived from the VH3 domain of JL427. The likely explanation is that the scFv GL2 VL domain provides an important contribution in recognition of the superantigenic epitope of gp120. The obvious alternative route to improving gp120 superantigen site binding, therefore, is to screen and select for optimally paired VL and VH domains from diverse combinatorial libraries of the two domains.

### References in EXAMPLE 4

1. Time from HIV-1 seroconversion to AIDS and death before widespread use of highly-active antiretroviral therapy: a collaborative re-analysis. Collaborative Group on AIDS Incubation and HIV Survival including the CASCADE EU Concerted Action. Concerted Action on SeroConversion to AIDS and Death in Europe. Lancet 2000, 355:1131-1137.
2. Cecilia D, Kleeberger C, Munoz A, Giorgi JV, Zolla-Pazner S: A longitudinal study of neutralizing antibodies and disease progression in HIV-1-infected subjects. J Infect Dis 1999, 179:1365-1374.
3. Townsley-Fuchs J, Kam L, Fairhurst R, Gange SJ, Goodglick L, Giorgi JV, Sidell N, Detels R, Braun J: Human immunodeficiency virus-1 (HIV-1) gp120 superantigen-binding serum antibodies. A host factor in homosexual HIV-1 transmission. J Clin Invest 1996, 98:1794-1801.
4. Gaudieri S, Nolan D, McKinnon E, Witt CS, Mallal S, Christiansen FT: Associations between KIR epitope combinations expressed by HLA-B/-C haplotypes found in an HIV-1 infected study population may influence NK mediated immune responses. Mol Immunol 2005, 42:557-560.
5. Burton DR, Pyati J, Koduri R, Sharp SJ, Thornton GB, Parren PW, Sawyer LS, Hendry RM, Dunlop N, Nara PL, et al.: Efficient neutralization of primary isolates of HIV-1 by a recombinant human monoclonal antibody. Science 1994, 266:1024-1027.
6. Sanders RW, Venturi M, Schiffner L, Kalyanaraman R, Katinger H, Lloyd KO, Kwong PD, Moore JP: The mannose-dependent epitope for neutralizing antibody 2G12 on human immunodeficiency virus type 1 glycoprotein gp120. J Virol 2002, 76:7293-7305.
7. Scanlan CN, Pantophlet R, Wormald MR, Ollmann Saphire E, Stanfield R, Wilson IA, Katinger H, Dwek RA, Rudd PM, Burton DR: The broadly neutralizing anti-human immunodeficiency virus type 1 antibody 2G12 recognizes a cluster of alpha1-->2 mannose residues on the outer face of gp120. J Virol 2002, 76:7306-7321.
8. Ofek G, Tang M, Sambor A, Katinger H, Mascola JR, Wyatt R, Kwong PD: Structure and mechanistic analysis of the anti-human immunodeficiency virus type 1 antibody 2F5 in complex with its gp41 epitope. J Virol 2004, 78:10724-10737.
9. Binley JM, Wrin T, Korber B, Zwick MB, Wang M, Chappey C, Stiegler G, Kunert R, Zolla-Pazner S, Katinger H, et al.: Comprehensive cross-clade neutralization analysis of a panel of anti-human immunodeficiency virus type 1 monoclonal antibodies. J Virol 2004, 78:13232-13252.
10. Mascola JR, Louder MK, VanCott TC, Sapan CV, Lambert JS, Muenz LR, Bunow B, Birx DL, Robb ML: Potent and synergistic neutralization of human immunodeficiency virus (HIV) type 1 primary isolates by hyperimmune anti-HIV immunoglobulin combined with monoclonal antibodies 2F5 and 2G12. J Virol 1997, 71:7198-7206.
11. Veazey RS, Shattock RJ, Pope M, Kirijan JC, Jones J, Hu Q, Ketas T, Marx PA, Klasse PJ, Burton DR, et al.: Prevention of virus transmission to macaque monkeys by a vaginally applied monoclonal antibody to HIV-1 gp120. Nat Med 2003, 9:343-346.
12. Gorny MK, Xu JY, Karwowska S, Buchbinder A, Zolla-Pazner S: Repertoire of neutralizing human monoclonal antibodies specific for the V3 domain of HIV-1 gp120. J Immunol 1993, 150:635-643.
13. Profy AT, Salinas PA, Eckler LI, Dunlop NM, Nara PL, Putney SD: Epitopes recognized by the neutralizing antibodies of an HIV-1-infected individual. J Immunol 1990, 144:4641-4647.
14. Berberian L, Goodglick L, Kipps TJ, Braun J: Immunoglobulin VH3 gene products: natural ligands for HIV gp120. Science 1993, 261:1588-1591.
15. Neshat MN, Goodglick L, Lim K, Braun J: Mapping the B cell superantigen binding site for HIV-1 gp120 on a V(H)3 Ig. Int Immunol 2000, 12:305-312.
16. Karray S, Juompan L, Maroun RC, Isenberg D, Silverman GJ, Zouali M: Structural basis of the gp120 superantigen-binding site on human immunoglobulins. J Immunol 1998, 161:6681-6688.
17. Goodglick L, Zevit N, Neshat MS, Braun J: Mapping the Ig superantigen-binding site of HIV-1 gp120. J Immunol 1995, 155:5151-5159.
18. Paul S, Karle S, Planque S, Taguchi H, Salas M, Nishiyama Y, Handy B, Hunter R, Edmundson A, Hanson C: Naturally occurring proteolytic antibodies: selective immunoglobulin M-catalyzed hydrolysis of HIV gp120. J Biol Chem 2004, 279:39611-39619.
19. Planque S, Mitsuda Y, Taguchi H, Salas M, Morris MK, Nishiyama Y, Kyle R, Okhuysen P, Escobar M, Hunter R, et al.: Characterization of gp120 Hydrolysis by IgA Antibodies from Humans without HIV Infection. AIDS Res Hum Retroviruses 2007, 23:1541-1554.
20. Hifumi E, Mitsuda Y, Ohara K, Uda T: Targeted destruction of the HIV-1 coat protein gp41 by a catalytic antibody light chain. J Immunol Methods 2002, 269:283-298.
21. Sun M, Gao QS, Kirnarskiy L, Rees A, Paul S: Cleavage specificity of a proteolytic antibody light chain and effects of the heavy chain variable domain. J Mol Biol 1997, 271:374-385.
22. Paul S: Natural catalytic antibodies. Mol Biotechnol 1996, 5:197-207.
23. Juompan L, Lambin P, Zouali M: Selective deficit in antibodies specific for the superantigen binding site of gp120 in HIV infection. Faseb J 1998, 12:1473-1480.
24. Bermas BL, Petri M, Berzofsky JA, Waisman A, Shearer GM, Mozes E: Binding of glycoprotein 120 and peptides from the HIV-1 envelope by autoantibodies in mice with experimentally induced systemic lupus erythematosus and in patients with the disease. AIDS Res Hum Retroviruses 1994, 10:1071-1077.
25. Chang BG, Markowitz GS, Seshan SV, Seigle RL, D'Agati VD: Renal manifestations of concurrent systemic lupus erythematosus and HIV infection. Am J Kidney Dis 1999, 33:441-449.
26. Daikh BE, Holyst MM: Lupus-specific autoantibodies in concomitant human immunodeficiency virus and systemic lupus erythematosus: case report and literature review. Semin Arthritis Rheum 2001, 30:418-425.
27. Palacios R, Santos J, Valdivielso P, Marquez M: Human immunodeficiency virus infection and systemic lupus erythematosus. An unusual case and a review of the literature. Lupus 2002, 11:60-63.
28. Sekigawa I, Lee S, Kaneko H, Iida N, Hashimoto H, Hirose S, Kaneko Y: The possible role of interleukin-16 in the low incidence of HIV infection in patients with systemic lupus erythematosus. Lupus 2000, 9:155-156.
29. Wallace DJ: Lupus, acquired immunodeficiency syndrome, and antimalarial agents. Arthritis Rheum 1991, 34:372-373.
30. Nishiyama Y, Karle S, Planque S, Taguchi H, Paul S: Antibodies to the superantigenic site of HIV-1 gp120: hydrolytic and binding activities of the light chain subunit. Mol Immunol 2007, 44:2707-2718.
31. Urnovitz HB, Murphy WH: Human endogenous retroviruses: nature, occurrence, and clinical implications in human disease. Clin Microbiol Rev 1996, 9:72-99.
32. Paul S, Tramontano A, Gololobov G, Zhou YX, Taguchi H, Karle S, Nishiyama Y, Planque S, George S: Phosphonate ester probes for proteolytic antibodies. J Biol Chem 2001, 276:28314-28320.
33. Taguchi H, Burr G, Karle S, Planque S, Zhou YX, Paul S, Nishiyama Y: A mechanism-based probe for gp120-Hydrolyzing antibodies. Bioorg Med Chem Lett 2002, 12:3167-3170.
34. Paul S, Planque S, Zhou YX, Taguchi H, Bhatia G, Karle S, Hanson C, Nishiyama Y: Specific HIV gp120-cleaving antibodies induced by covalently reactive analog of gp120. J Biol Chem 2003, 278:20429-20435.
35. Karle S, Planque S, Nishiyama Y, Taguchi H, Zhou YX, Salas M, Lake D, Thiagarajan P, Arnett F, Hanson CV, et al.: Cross-clade HIV-1 neutralization by an antibody fragment from a lupus phage display library. Aids 2004, 18:329-331.
36. Brown BK, Karasavvas N, Beck Z, Matyas GR, Birx DL, Polonis VR, Alving CR: Monoclonal antibodies to phosphatidylinositol phosphate neutralize human immunodeficiency virus type 1: role of phosphate-binding subsites. J Virol 2007, 81:2087-2091.
37. Brown BK, Wieczorek L, Rosa Borges A, Sanders-Buell E, Horak J, Robb M, Birx D, Michael N, McCutchan F, Polonis V: HIV neutralization is profoundly affected by the cell model system used in vitro when a multiclade virus panel and polyclonal antibodies are employed. AIDS Vaccine 2007. Seattle, WA; Aug 20-23, 2007 Abstract# OA05-08 (http://www.hivvaccineenterprise.org/_dwn/Oral_Sessions.pdf).
38. Nishiyama Y, Bhatia G, Bangale Y, Planque S, Mitsuda Y, Taguchi H, Karle S, Paul S: Toward selective covalent inactivation of pathogenic antibodies: a phosphate diester analog of vasoactive intestinal peptide that inactivates catalytic autoantibodies. J Biol Chem 2004, 279:7877-7883.
39. Reed J, Kinzel V: Primary structure elements responsible for the conformational switch in the envelope glycoprotein gp120 from human immunodeficiency virus type 1: LPCR is a motif governing folding. Proc Natl Acad Sci U S A 1993, 90:6761-6765.
40. Karle S, Nishiyama Y, Taguchi H, Zhou YX, Luo J, Planque S, Hanson C, Paul S: Carrier-dependent specificity of antibodies to a conserved peptide determinant of gp120. Vaccine 2003, 21:1213-1218.

SEQUENCE LISTING

<110>   Sudhir, Paul
Planque, Stephanie Brown, Eric L Smith, Keri C Nishiyama, Yasuhiro Taguchi,
Hiroaki

<120>   Immunoglobulins Directed to Bacterial, Viral and Endogenous
Polypeptides

<130>   P-0118-EP2

<140>   EP08767415.6
<141>   2008-04-23
<150>   us 60/913,335
<151>   2007-04-23

<160>   69

<170>   PatentIn version 3.3

<210>   1
<211>   33
<212>   DNA
<213>   Artificial

<220>
<223>   reverse primer used in VH amplification

<400>   1
ggtagtgcac ttcaggtgca gctgttgcag tct    33

<210>   2
<211>   35
<212>   DNA
<213>   Artificial

<220>
<223>   forward primer used in VH amplification

<400>   2
atgtgcggcc gcggggaaaa gggttggggg catgc  35


<210>   3
<211>   29
<212>   DNA
<213>   Artificial

<220>
<223>   NcoI primer used in VL amplification

<400>   3



taagatctca gtctgccctg actcagcct 29

<210>   4
<211>   31
<212>   DNA
<213>   Artificial

<220>
<223>   XhoI primer used in VL amplification

<400>   4
tagcggccgc gggctgacct aaaacggtga g      31

<210>   5
<211>   25
<212>   DNA
<213>   Artificial

<220>
<223>   ApaLl primer used in VL amplification

<400>   5
tagaattcca gttgacccag tctcc     25

<210>   6
<211>   28
<212>   DNA
<213>   Artificial

<220>
<223>   NotI primer used in VL amplification

<400>   6
taaagcttgc acgtttgatt tccagctt  28

<210>   7
<211>   40
<212>   PRT
<213>   Artificial

<220>
<223>   amyloid beta 40 analog peptide


<220>
<221>   MISC- FEATURE
<222>   (16)..(16)
<223>   Xaa is any amino acid

<220>
<221>   MISC- FEATURE
<222>   (28)..(28)
<223>   Xaa is any amino acid




<400>   7
Asp Ala Glu Phe Arg His Asp Ser Gly Tyr Glu Val His His Gln Xaa 1       5
10      15
Leu Val Phe Phe Ala Glu Asp Val Gly Ser Asn Xaa Gly Ala Ile Ile 20      25
30
Gly Leu Met Val Gly Gly Val Val 35      40

<210>   8
<211>   262
<212>   PRT
<213>   Artificial

<220>
<223>   IgVL2-t 2E6 amino acid sequence

<400>   8
Gln Ser Ala Leu Thr Gln Pro Ala Ser Val Ser Gly Ser Pro Gly Gln 1       5
10      15
Ser Ile Thr Ile Ser Cys Thr Gly Thr Ser Ser Asp Val Gly Gly Tyr 20      25
30
Asn Tyr Val Ser Trp Tyr Gln Gln His Pro Gly Lys Ala Pro Lys Leu 35      40
45
Met Ile Ser Glu Val Ser Asn Arg Pro Ser Gly Val Ser Asn Arg Phe 50      55
60
Ser Gly Ser Lys Ser Gly Asn Thr Ala Ser Leu Thr Ile Ser Gly Pro 65      70
75      80
Gln Thr Glu Asp Glu Ala Asp Tyr Tyr Cys Ser Ser Tyr Thr Ser Ser 85      90
95
Ser Thr Pro Val Val Phe Gly Gly Gly Thr Gln Leu Thr Val Leu Gly 100     105
110
Ser Ser Gly Gly Gly Gly Ser Gly Gly Gly Gly Ser Gly Gly Ser Ala 115     120
125

Leu Asp Ile Gln Leu Thr Gln Ser Pro Ser Ser Leu Pro Ala Ser Val 130     135
140
Gly Asp Arg Val Thr Ile Thr Cys Arg Ala Ser Gln Ser Ile Ser Ser 145     150
155     160
Tyr Leu Asn Trp Tyr Gln Gln Lys Pro Gly Lys Ala Pro Lys Leu Leu 165     170
175
Ile Tyr Ala Ala Ser Ser Leu Gln Ser Gly Val Pro Ser Arg Phe Ser 180     185
190
Gly Ser Gly Ser Gly Thr Asp Phe Thr Leu Thr Ile Ser Ser Leu Gln 195     200
205
Pro Glu Asp Phe Ala Thr Tyr Tyr Cys Gln Gln Ser Tyr Ser Thr His 210     215
220
Thr Phe Gly Gln Gly Thr Lys Leu Glu Ile Lys Arg Ala Ala Ala His 225     230
235     240
His His His His His Gly Ala Ala Glu Gln Lys Leu Ile Ser Glu Glu 245     250
255

Asp Leu Asn Gly Ala Ala
260

<210>   9
<211>   168
<212>   PRT
<213>   Artificial

<220>
<223>   IgVL2-t 5D3 amino acid sequence

<400>   9
Glu Ile Val Leu Thr Gln Ser Pro Gly Thr Leu Ser Leu Ser Pro Gly 1       5
10      15
Glu Arg Ala Thr Leu Ser Cys Arg Ala Ser Gln Ser Val Gly Gly Ser 20      25
30
Tyr Leu Ala Trp Tyr Gln Gln Lys Pro Gly Gln Ala Pro Arg Leu Leu 35      40
45

Ile Tyr Gly Ala Ser Ser Arg Ala Thr Gly Ile Pro Asp Arg Phe Ser 50      55
60
Gly Ser Gly Ser Gly Thr Asp Phe Thr Leu Thr Ile Ser Arg Leu Glu 65      70
75      80
Pro Glu Asp Phe Ala Val Tyr Tyr Cys Gln Leu Tyr Gly Gly Ser Pro 85      90
95

Met Tyr Thr Phe Gly Gln Gly Thr Lys Leu Glu Ile Lys Arg Ser Ser 100 105
110
Gly Gly Gly Gly Ser Gly Gly Gly Gly Ser Gly Gly Ser Ala Leu Gln 115 120
125
Val Gln Leu Leu Gln Ser Ala Cys Ala Pro Thr Leu Phe Pro Ala Ala 130 135
140
Ala His His His His His His Gly Ala Ala Glu Gln Lys Leu Ile Ser 145 150
155 160

Glu Glu Asp Leu Asn Gly Ala Ala
165

<210> 10
<211> 182
<212> PRT
<213> Artificial

<220>
<223> IgVL2-t 1E4 amino acid sequence

<400> 10
Gln Ser Val Leu Thr Gln Pro Pro Ser Val Ser Ala Ala Pro Gly Gln 1 5
10 15
Lys Val Thr Ile Ser Cys Ser Gly Ser Ser Ser Asn Ile Gly Asn Asn 20 25
30
Tyr Val Ser Trp Tyr Gln Gln Leu Pro Gly Thr Ala Pro Lys Leu Leu 35 40
45
Ile Tyr Asp Asn Glu Arg Arg Pro Ser Gly Ile Pro Asp Arg Phe Ser 50 55
60

Gly Ser Lys Ser Gly Thr Ser Ala Thr Leu Ala Ile Thr Gly Leu Gln 65 70
75 80
Pro Gly Asp Glu Ala Asp Tyr Tyr Cys Ala Ala Trp Asp Asn Thr Leu 85 90
95
Arg Gly Gly Val Phe Gly Gly Gly Thr Gln Leu Thr Val Leu Gly Ser CTA100 105
110
Ser Gly Gly Gly Gly Ser Gly Gly Gly Gly Ser Gly Gly Ser Ala Leu 115 120
125
Gln Val Gln Leu Gln Glu Ser Gly Ala Gly Glu Glu Arg Gly Arg Val 130 135
140
Trp Cys Thr His Thr Lys Gly Pro Ser Val Phe Pro Ala Ala Ala His 145 150
155 160
His His His His His Gly Ala Ala Glu Gln Lys Leu Ile Ser Glu Glu 165 170
175

Asp Leu Asn Gly Ala Ala
180

<210> 11
<211> 172
<212> PRT
<213> Artificial

<220>
<223> IgVL2-t 5H3 amino acid sequence

<400> 11
Gln Ser Val Leu Thr Gln Pro Pro Ser Val Ser Gly Ala Pro Gly Gln 1 5
10 15
Arg Val Thr Ile Ser Cys Thr Gly Ser Ser Ser Asn Ile Gly Ala Gly 20 25

30
Tyr Asp Val His Trp Tyr Gln Gln Leu Pro Gly Thr Ala Pro Lys Leu 35 40
45
Leu Ile Tyr Gly Asn Ser Asn Arg Pro Ser Gly Val Pro Asp Arg Phe

50 55 60
Ser Gly Ser Lys Ser Gly Thr Ser Ala Ser Leu Ala Ile Thr Gly Leu 65 70
75 80
Gln Ala Glu Asp Glu Ala Asp Tyr Tyr Cys Gln Ser Tyr Asp Ser Ser 85 90
95
Leu Ser Gly Val Val Phe Gly Gly Gly Thr Gln Leu Thr Val Leu Gly 100 105
110
Ser Ser Gly Gly Gly Gly Ser Gly Gly Gly Gly Ser Gly Gly Ser Ala 115 120
125
Leu Gln Val Ser Cys Gly Val Gly Ser His Ala Ser Ala Pro Thr Leu 130 135
140
Phe Pro Ala Ala Ala His His His His His His Gly Ala Ala Glu Gln 145 150
155 160

Lys Leu Ile Ser Glu Glu Asp Leu Asn Gly Ala Ala
165 170

<210> 12
<211> 154
<212> PRT
<213> Artificial

<220>
<223> VH 5D3-D4 derivative VH domain sequence, from mutant IgVL-t 5D3

<400> 12
Gln Val Gln Leu Leu Gln Ser Gly Ala Glu Val Lys Lys Pro Gly Ala 1 5
10 15
Ser Val Lys Val Ser Cys Lys Ala Ser Gly Tyr Thr Phe Thr Gly Tyr 20 25
30
Tyr Met His Trp Val Arg Gln Ala Pro Gly Gln Gly Leu Glu Trp Met 35 40
45
Gly Trp Ile Asn Pro Asn Ser Gly Gly Thr Asn Tyr Ala Gln Lys Phe 50 55
60

Gln Gly Arg Val Thr Met Thr Arg Asp Thr Ser Ile Ser Thr Ala Tyr 65 70
75 80
Met Glu Leu Ser Arg Leu Arg Ser Asp Asp Thr Ala Val Tyr Tyr Cys 85 90
95
Ala Arg Trp Ser His Ser Ser Gly Leu Asp Tyr Trp Gly Gln Gly Thr 100 105
110
Leu Val Thr Val Ser Ser Gly Ser Ala Cys Ala Pro Thr Leu Phe Pro 115 120
125
Ala Ala Ala His His His His His His Gly Ala Ala Glu Gln Lys Leu 130 135
140
Ile Ser Glu Glu Asp Leu Asn Gly Ala Ala 145 150


<210> 13
<211> 151
<212> PRT
<213> Artificial

<220>
<223> VH 5D3-D10 derivative VH domain sequence, from mutant IgVL-t 5D3

<400> 13
Gln Val Gln Leu Leu Gln Ser Gly Ala Glu Val Lys Lys Pro Gly Ala 1 5

10      15
Ser Val Lys Val Ser Cys Lys Ala Ser Gly Tyr Thr Phe Thr Ser Tyr 20      25
30
Asp Ile Asn Trp Val Arg Gln Ala Thr Gly Gln Gly Leu Glu Trp Met 35      40
45
Gly Trp Met Asn Pro Asn Ser Gly Asn Thr Gly Tyr Ala Gln Lys Phe 50      55
60
Gln Gly Arg Val Thr Met Thr Arg Asn Thr Ser Ile Ser Thr Ala Tyr 65      70
75      80
Met Glu Leu Ser Ser Leu Arg Ser Glu Gly Thr Ala Val Tyr Tyr Cys

85      90      95
Ala Thr Gly Ser Arg Tyr Asp Tyr Trp Gly Gln Gly Thr Leu Val Thr 100     105
110
Val Ser Ser Gly Ser Ala Cys Ala Pro Thr Leu Phe Pro Ala Ala Ala 115     120
125
His His His His His His Gly Ala Ala Glu Gln Lys Leu Ile Ser Glu 130     135
140
Glu Asp Leu Asn Gly Ala Ala 145 150

<210>   14
<211>   155
<212>   PRT
<213>   Artificial

<220>
<223>   VH 5D3-E4 derivative VH domain sequence, from mutant IgVL-t 5D3

<400>   14
Gln Val Gln Leu Leu Gln Ser Gly Pro Gly Leu Val Lys Pro Ser Glu 1       5
10      15
Thr Leu Ser Leu Thr Cys Thr Val Ser Gly Gly Ser Ile Ser Ser Gly 20      25
30
Ser Tyr Tyr Trp Ser Trp Ile Arg Gln Pro Ala Gly Lys Gly Leu Glu 35      40
45
Trp Ile Gly Arg Ile Tyr Thr Ser Gly Ser Thr Asn Tyr Asn Pro Ser 50      55
60
Leu Lys Ser Arg Val Thr Ile Ser Val Asp Thr Ser Lys Asn Gln Phe 65      70
75      80
Ser Leu Lys Leu Ser Ser Val Thr Ala Ala Asp Thr Ala Val Tyr Tyr 85      90
95
Cys Ala Arg Val Gly Arg Leu Asn Trp Phe Asp Pro Trp Gly Gln Gly 100     105
110

Thr Leu Val Thr Val Ser Ser Gly Ser Ala Cys Ala Pro Thr Leu Phe 115     120
125
Pro Ala Ala Ala His His His His His His Gly Ala Ala Glu Gln Lys 130     135
140
Leu Ile Ser Glu Glu Asp Leu Asn Gly Ala Ala 145 150     155


<210>   15
<211>   156
<212>   PRT
<213>   Artificial

<220>
<223>   VH 5D3-E6 derivative VH domain sequence, from mutant IgVL-t 5D3

<400>   15
Gln Val Gln Leu Leu Gln Ser Gly Pro Gly Leu Val Lys Pro Ser Glu 1       5
10      15
Thr Leu Ser Leu Thr Cys Thr Val Ser Gly Gly Ser Ile Ser Ser Gly 20      25

30
Gly Tyr Tyr Trp Ser Trp Ile Arg Gln His Pro Gly Lys Gly Leu Glu 35 40
45
Trp Ile Gly Tyr Ile Tyr Tyr Ser Gly Ser Thr Tyr Tyr Asn Pro Ser 50 55
60
Leu Lys Ser Arg Val Thr Ile Ser Val Asp Thr Ser Lys Asn Gln Phe 65 70
75 80
Ser Leu Lys Leu Ser Ser Val Thr Ala Ala Asp Thr Ala Val Tyr Tyr 85 90
95
Cys Ala Arg Ala Gly Asp Ser Ser Gly Pro Gly Asp Tyr Trp Gly Gln 100 105
110
Gly Thr Leu Val Thr Val Ser Ser Gly Ser Ala Cys Ala Pro Thr Leu 115 120
125
Phe Pro Ala Ala Ala His His His His His His Gly Ala Ala Glu Gln

130 135 140
Lys Leu Ile Ser Glu Glu Asp Leu Asn Gly Ala Ala 145 150 155

<210> 16
<211> 242
<212> PRT
<213> Artificial

<220>
<223> full length L-t 5D3 sequence from mutant IgVL-t 5D3

<400> 16
Glu Ile Val Leu Thr Gln Ser Pro Gly Thr Leu Ser Leu Ser Pro Gly 1 5
10 15
Glu Arg Ala Thr Leu Ser Cys Arg Ala Ser Gln Ser Val Gly Gly Ser 20 25
30
Tyr Leu Ala Trp Tyr Gln Gln Lys Pro Gly Gln Ala Pro Arg Leu Leu 35 40
45
Ile Tyr Gly Ala Ser Ser Arg Ala Thr Gly Ile Pro Asp Arg Phe Ser 50 55
60
Gly Ser Gly Ser Gly Thr Asp Phe Thr Leu Thr Ile Ser Arg Leu Glu 65 70
75 80
Pro Glu Asp Phe Ala Val Tyr Tyr Cys Gln Leu Tyr Gly Gly Ser Pro 85 90
95
Met Tyr Thr Phe Gly Gln Gly Thr Lys Leu Glu Ile Lys Arg Thr Val 100 105
110
Ala Ala Pro Ser Val Phe Ile Phe Pro Pro Ser Asp Glu Gln Leu Lys 115 120
125
Ser Gly Thr Ala Ser Val Val Cys Leu Leu Asn Asn Phe Tyr Pro Arg 130 135
140
Glu Ala Lys Val Gln Trp Lys Val Asp Asn Ala Leu Gln Ser Gly Asn 145 150
155 160

Ser Gln Glu Ser Val Thr Glu Gln Asp Ser Lys Asp Ser Thr Tyr Ser 165 170
175
Leu Ser Ser Thr Leu Thr Leu Ser Lys Ala Asp Tyr Glu Lys His Lys 180 185
190
Val Tyr Ala Cys Glu Val Thr His Gln Gly Leu Ser Ser Pro Val Thr 195 200
205
Lys Ser Phe Asn Arg Gly Glu Cys Ala Ala Ala His His His His His 210 215
220
His Gly Ala Ala Glu Gln Lys Leu Ile Ser Glu Glu Asp Leu Asn Gly 225 230
235 240

Ala Ala


<210> 17

<211>   263
<212>   PRT
<213>   Artificial

<220>
<223>   homodimeric IgVL2-t sequence from mutant IgVL-t 5D3

<400>   17
Glu Ile Val Leu Thr Gln Ser Pro Gly Thr Leu Ser Leu Ser Pro Gly 1       5
10      15
Glu Arg Ala Thr Leu Ser Cys Arg Ala Ser Gln Ser Val Gly Gly Ser 20      25
30
Tyr Leu Ala Trp Tyr Gln Gln Lys Pro Gly Gln Ala Pro Arg Leu Leu 35      40
45
Ile Tyr Gly Ala Ser Ser Arg Ala Thr Gly Ile Pro Asp Arg Phe Ser 50      55
60
Gly Ser Gly Ser Gly Thr Asp Phe Thr Leu Thr Ile Ser Arg Leu Glu 65      70
75      80
Pro Glu Asp Phe Ala Val Tyr Tyr Cys Gln Leu Tyr Gly Gly Ser Pro 85      90
95

Met Tyr Thr Phe Gly Gln Gly Thr Lys Leu Glu Ile Lys Arg Ser Ser 100     105
110
Gly Gly Gly Gly Ser Gly Gly Gly Gly Ser Gly Gly Ser Ala Leu Glu 115     120
125
Ile Val Leu Thr Gln Ser Pro Gly Thr Leu Ser Leu Ser Pro Gly Glu 130     135
140
Arg Ala Thr Leu Ser Cys Arg Ala Ser Gln Ser Val Gly Gly Ser Tyr 145     150
155     160
Leu Ala Trp Tyr Gln Gln Lys Pro Gly Gln Ala Pro Arg Leu Leu Ile 165     170
175
Tyr Gly Ala Ser Ser Arg Ala Thr Gly Ile Pro Asp Arg Phe Ser Gly 180     185
190
Ser Gly Ser Gly Thr Asp Phe Thr Leu Thr Ile Ser Arg Leu Glu Pro 195     200
205
Glu Asp Phe Ala Val Tyr Tyr Cys Gln Leu Tyr Gly Gly Ser Pro Met 210     215
220
Tyr Thr Phe Gly Gln Gly Thr Lys Leu Glu Ile Lys Arg Ala Ala Ala 225     230
235     240
His His His His His His Gly Ala Ala Glu Gln Lys Leu Ile Ser Glu 245     250
255

Glu Asp Leu Asn Gly Ala Ala 260

<210>   18
<211>   42
<212>   PRT
<213>   Homo sapiens



<220>
<221>
<223>

<400>


misc feature
amyloid beta 42 peptide 18

 1 5
10 15
 20 25
30
 35 40

<210> 19
<211> 150
<212> PRT
<213> Staphylococcus aureus

<220>
<221>
<223>

<400>

misc feature
Efb amino acid sequence 19

Met Arg Gly Ser His His His His His His Gly Ala Asp Ala Ser Glu 1 5
10 15
Gly Tyr Gly Pro Arg Glu Lys Lys Pro Val Ser Ile Asn His Asn Ile 20 25
30
Val Glu Tyr Asn Asp Gly Thr Phe Lys Tyr Gln Ser Arg Pro Lys Phe 35 40
45
Asn Ser Thr Pro Lys Tyr Ile Lys Phe Lys His Asp Tyr Asn Ile Leu 50 55
60
Glu Phe Asn Asp Gly Thr Phe Glu Tyr Gly Ala Arg Pro Gln Phe Asn 65 70
75 80
Lys Pro Ala Ala Lys Thr Asp Ala Thr Ile Lys Lys Glu Gln Lys Leu 85 90
95
Ile Gln Ala Gln Asn Leu Val Arg Glu Phe Glu Lys Thr His Thr Val 100 105
110
Ser Ala His Arg Lys Ala Gln Lys Ala Val Asn Leu Val Ser Phe Glu 115 120
125
Tyr Lys Val Lys Lys Met Val Leu Gln Glu Arg Ile Asp Asn Val Leu

130 135 140
Lys Gln Gly Leu Val Lys 145 150

<210> 20
<211> 330
<212> DNA
<213> Artificial

<220>
<223> VL domain nucleotide sequence of scFv 1C7

<400> 20
cagtctgtgt tgacgcagcc gccctcagtg tctgcggccc caggacagat ggtcaccatc tcctgctccg
gaagcagttc caacattaag gataatattg tttcgtggta ccagaagttc ccaggaacag cccccaaact
cctcatttat gacaatgaga ggcgaccctc agggattcct gaccgattct ctggctccaa gtctggcacg
tcagccaccc tggccatcac cggactccag cccggggacg aggccgatta ttactgcgca gcttgggata
acacccttcg tggtggggtg ttcggcggag ggaccaaggtc accgtccta

<210> 21
<211> 333
<212> DNA

<213> Artificial

<220>
<223> VL domain nucleotide sequence of Vl-19

<400> 21
cagtctgtgt tgacgcagcc gccctcagtg tctgcggccc caggacagaa ggtcaccatc tcctgctctg
gaagcagctc caacattggg aataattatg tatcctggta ccagcagctc ccaggaacag cccccaaact
cctcatttat gacaataata agcgaccctc agggattcct gaccgattct ctggctccaa gtctggcacg
tcagccaccc tgggcatcac cggactccag actggggacg aggccgatta ttactgcgga acatgggata
gcagcctgag tgctggttgg gtgttcggcg gagggaccaa gctgaccgtc cta

60

120

180

240

300

330

60

120

180

240

300

333

<210> 22
<211> 111
<212> PRT
<213> Artificial

<220>

<223> VL domain amino acid sequence of scFv 1C7

<220>
<221>
<222>
<223>

<400>

MISC FEATURE (100)..(100)
Xaa is any amino acid 22

Gln Ser Val Leu Thr Gln Pro Pro Ser Val Ser Ala Ala Pro Gly Gln 1 5
10 15
Met Val Thr Ile Ser Cys Ser Gly Ser Ser Ser Asn Ile Lys Asp Asn 20 25
30
Ile Val Ser Trp Tyr Gln Lys Phe Pro Gly Thr Ala Pro Lys Leu Leu 35 40
45
Ile Tyr Asp Asn Glu Arg Arg Pro Ser Gly Ile Pro Asp Arg Phe Ser 50 55
60
Gly Ser Lys Ser Gly Thr Ser Ala Thr Leu Ala Ile Thr Gly Leu Gln 65 70
75 80
Pro Gly Asp Glu Ala Asp Tyr Tyr Cys Ala Ala Trp Asp Asn Thr Leu 85 90
95
Arg Gly Gly Xaa Val Phe Gly Gly Gly Thr Lys Val Thr Val Leu 100 105 110

<210> 23
<211> 111
<212> PRT
<213> Artificial

<220>
<223> VL domain amino acid sequence of Vl-19

<220>
<221>
<222>
<223>

<400>

MISC FEATURE (100)..(100)
Xaa is any amino acid 23

Gln Ser Val Leu Thr Gln Pro Pro Ser Val Ser Ala Ala Pro Gly Gln 1 5
10 15

Lys Val Thr Ile Ser Cys Ser Gly Ser Ser Ser Asn Ile Gly Asn Asn 20 25
30
Tyr Val Ser Trp Tyr Gln Gln Leu Pro Gly Thr Ala Pro Lys Leu Leu 35 40
45
Ile Tyr Asp Asn Asn Lys Arg Pro Ser Gly Ile Pro Asp Arg Phe Ser 50 55
60
Gly Ser Lys Ser Gly Thr Ser Ala Thr Leu Gly Ile Thr Gly Leu Gln 65 70
75 80
Thr Gly Asp Glu Ala Asp Tyr Tyr Cys Gly Thr Trp Asp Ser Ser Leu 85 90
95
Ser Ala Gly Xaa Val Phe Gly Gly Gly Thr Lys Leu Thr Val Leu 100 105 110

<210> 24
<211> 372
<212> DNA
<213> Artificial

<220>
<223>   VH domain nucleotide sequence of scFv 1C7


<400>   24
gaggtgcagc tggtggagtc tgggggaggc gtggtccagc ctgggaggtc cctgagactc tcctgtgcag
cgtctggatt caccttcagt agctatggca tgcactgggt ccgccaggct ccaggcaagg ggctggagtg
ggtggcagtt atatggtatg atggaagtaa taaatactat gcagactccg tgaagggccg attcaccatc
tccagagaca attccaagaa cacgctgtat ctgctgcaaa tgaacagcct gagagccgag gacacggctg
tgtattactg tgcgagagat ccaggggatt gtagtggtgg tagctgctac tttgactact ggggccaggg
aaccctggtc accgtctcct ca


60

120

180

240

300

360

372


<210>   25
<211>   127
<212>   PRT
<213>   Artificial

<220>
<223>   VH domain amino acid sequence of scFv 1C7




<220>
<221>   MISC FEATURE
<222>   (112)..(112)
<223>   Xaa is any amino acid

<220>
<221>   MISC FEATURE
<222>   (113)..(113)
<223>   Xaa is any amino acid

<220>
<221>   MISC FEATURE
<222>   (114)..(114)
<223>   Xaa is any amino acid

<400>   25
Glu Val Gln Leu Val Glu Ser Gly Gly Gly Val Val Gln Pro Gly Arg 1       5
10      15
Ser Leu Arg Leu Ser Cys Ala Ala Ser Gly Phe Thr Phe Ser Ser Tyr 20      25
30
Gly Met His Trp Val Arg Gln Ala Pro Gly Lys Gly Leu Glu Trp Val 35      40
45
Ala Val Ile Trp Tyr Asp Gly Ser Asn Lys Tyr Tyr Ala Asp Ser Val 50      55
60

Lys Gly Arg Phe Thr Ile Ser Arg Asp Asn Ser Lys Asn Thr Leu Tyr 65 70
75 80
Leu Leu Gln Met Asn Ser Leu Arg Ala Glu Asp Thr Ala Val Tyr Tyr 85 90
95
Cys Ala Arg Asp Pro Gly Asp Cys Ser Gly Gly Ser Cys Tyr Phe Xaa 100 105
110
Xaa Xaa Asp Tyr Trp Gly Gln Gly Thr Leu Val Thr Val Ser Ser 115 120 125

<210> 26
<211> 380
<212> DNA
<213> Artificial

<220>
<223> VH domain nucleotide sequence of VH3-33

<220>
<221> misc feature
<222> (300)..(300)
<223> n is an underdetermined nucleotide

<220>
<221> misc- feature
<222> (301)..(301)
<223> n is an underdetermined nucleotide

<220>
<221> misc feature
<222> (302)..(302)
<223> n is an underdetermined nucleotide

<400> 26
gaggtgcagc tggtggagtc tgggggaggc gtggtccagc ctgggaggtc cctgagactc tcctgtgcag
cgtctggatt caccttcagt agctatggca tgcactgggt ccgccaggct ccaggcaagg ggctggagtg
ggtggcagtt atatggtatg atggaagtaa taaatactat gcagactccg tgaagggccg attcaccatc
tccagagaca attccaagaa cacgctgtat ctgctgcaaa tgaacagcct gagagccgag gacacggctg
tgtattactg tgcgagagan nnaggatatt gtagtggtgg tagctgctac tccactactt tgactactgg
ggccaaggaa ccctggtcac cgtctcctca

60

120

180

240

300

360

380

<210> 27
<211> 127
<212> PRT

<213>   Artificial

<220>
<223>   VH domain amino acid sequence of VH3-33

<220>
<221>
<222>
<223>

<400>

misc feature (112)..(112)
Xaa is any amino acid 27

Glu Val Gln Leu Val Glu Ser Gly Gly Gly Val Val Gln Pro Gly Arg 1       5
10      15
Ser Leu Arg Leu Ser Cys Ala Ala Ser Gly Phe Thr Phe Ser Ser Tyr 20      25
30

Gly Met His Trp Val Arg Gln Ala Pro Gly Lys Gly Leu Glu Trp Val 35      40
45
Ala Val Ile Trp Tyr Asp Gly Ser Asn Lys Tyr Tyr Ala Asp Ser Val 50      55
60
Lys Gly Arg Phe Thr Ile Ser Arg Asp Asn Ser Lys Asn Thr Leu Tyr 65      70
75      80
Leu Leu Gln Met Asn Ser Leu Arg Ala Glu Asp Thr Ala Val Tyr Tyr 85      90
95
Cys Ala Arg Glu Pro Gly Asp Cys Ser Gly Gly Ser Cys Tyr Phe Xaa 100     105
110
Tyr Phe Asp Tyr Trp Gly Gln Gly Thr Leu Val Thr Val Ser Ser 115 120     125

<210>   28
<211>   324
<212>   DNA
<213>   Artificial

<220>
<223>   N-terminal VL domain nucleotide sequence of IgVL2-t 1B4

<400>   28
tcttctgagc tgactcagga ccctgctgtg tctgtggcct tgggacagac agtcaggatc acatgccaag
gagacagcct cagaagctat tatgcaagct ggtaccagca gaagccagga caggcccctg tacttgtcat
ctatggtaaa aacaaccggc cctcagggat cccagaccga ttctctggct ccagctcagg aaacacagct
tccttgacca tcactggggc tcaggcggaa gatgaggctg actattactg taactcccgg gacagcagtg
gtaaccattg ggtgttcggc ggagggacca aggtcaccgt ccta

60

120

180

240

300

324

<210>   29
<211>   327
<212>   DNA
<213>   Artificial

<220>
<223>   N-terminal VL domain nucleotide sequence of V2-13

<400>   29
tcttctgagc tgactcagga ccctgctgtg tctgtggcct tgggacagac agtcaggatc       60

acatgccaag gagacagcct cagaagctat tatgcaagct ggtaccagca gaagccagga       120
caggcccctg tacttgtcat ctatggtaaa aacaaccggc cctcagggat cccagaccga       180
ttctctggct ccagctcagg aaacacagct tccttgacca tcactggggc tcaggcggaa       240
gatgaggctg actattactg taactcccgg gacagcagtg gtaaccatct ttgggtgttc       300
ggcggaggga ccaagctgac cgtccta   327

<210>   30
<211>   109
<212>   PRT
<213>   Artificial

<220>
<223>   N-terminal VL domain amino acid sequence of IgVL2-t 1B4

<220>
<221>
<222>
<223>

<400>

MISC FEATURE (97)..(97)
Xaa is any amino acid 30

Ser Ser Glu Leu Thr Gln Asp Pro Ala Val Ser Val Ala Leu Gly Gln 1       5
10      15
Thr Val Arg Ile Thr Cys Gln Gly Asp Ser Leu Arg Ser Tyr Tyr Ala 20      25
30
Ser Trp Tyr Gln Gln Lys Pro Gly Gln Ala Pro Val Leu Val Ile Tyr 35      40
45
Gly Lys Asn Asn Arg Pro Ser Gly Ile Pro Asp Arg Phe Ser Gly Ser 50      55
60
Ser Ser Gly Asn Thr Ala Ser Leu Thr Ile Tyr Gly Ala Gln Ala Glu 65      70
75      80
Asp Glu Ala Asp Tyr Tyr Cys Asn Ser Arg Asp Ser Ser Gly Asn His 85      90
95
Xaa Trp Val Phe Gly Gly Gly Thr Lys Val Thr Val Leu 100 105
<210>   31

<211>   109
<212>   PRT
<213>   Artificial

<220>
<223>   N-terminal VL domain amino acid sequence of V2-13;

<220>
<221>
<222>
<223>

<400>

MISC FEATURE (97)..(97)
Xaa is any amino acid 31

Ser Ser Glu Leu Thr Gln Asp Pro Ala Val Ser Val Ala Leu Gly Gln 1 5
10 15
Thr Val Arg Ile Thr Cys Gln Gly Asp Ser Leu Arg Ser Tyr Tyr Ala 20 25
30
Ser Trp Tyr Gln Gln Lys Pro Gly Gln Ala Pro Val Leu Val Ile Tyr 35 40
45
Gly Lys Asn Asn Arg Pro Ser Gly Ile Pro Asp Arg Phe Ser Gly Ser 50 55
60
Ser Ser Gly Asn Thr Ala Ser Leu Thr Ile Tyr Gly Ala Gln Ala Glu 65 70
75 80
Asp Glu Ala Asp Tyr Tyr Cys Asn Ser Arg Asp Ser Ser Gly Asn His 85 90
95
Xaa Trp Val Phe Gly Gly Gly Thr Lys Leu Thr Val Leu 100 105

<210> 32
<211> 318
<212> DNA
<213> Artificial

<220>
<223> C-terminal VL domain nucleotide sequence of IgVL2-t 1B4

<400> 32
gacatccagt tgacccagtc tccatcctcc ctgcctgcat ctgtaggaga cagagtcacc 60
atcacttgcc gggcaagtca gagcattagc agctatttaa actggtatca gcagaaacca 120

gggaaagccc ctaagctcct gatctatgct gcatccagtt tgcaaagtgg ggtcccatca 180
aggttcagtg gcagtggatc tgggacagat ttcactctca ccatcagcag tctgcaacct 240
gaagattttg caacttacta ctgtcaacaa agttacagta cccacacgtt cggccaaggg 300
accaagctgg aaatcaaa 318

<210> 33
<211> 325
<212> DNA
<213> Artificial

<220>
<223> C-terminal VL domain nucleotide sequence of 02/012

<400> 33
gacatccaga tgacccagtc tccatcctcc ctgtctgcat ctgtaggaga cagagtcacc atcacttgcc
gggcaagtca gagcattagc agctatttaa attggtatca gcagaaacca gggaaagccc ctaagctcct
gatctatgct gcatccagtt tgcaaagtgg ggtcccatca aggttcagtg gcagtggatc tgggacagat
ttcactctca ccatcagcag tctgcaacct gaagattttg caacttacta ctgtcaacaa agttacagta
cccctccgtg gacgttcggc caagggacca agctggaaat caaac

60

120

180

240

300

325

<210>   34
<211>   108
<212>   PRT
<213>   Artificial

<220>
<223>   C-terminal VL domain nucleotide sequence of IgVL2-t 1B4

<220>
<221>
<222>
<223>

<400>

MISC FEATURE
(96) .. (97)
Xaa is Lys, Asn, Arg, Ser Ile, Met, Glu Asp, Gly, Val, Tyr, Trp, Cys, Leu,
or Phe

34

Asp Ile Gln Leu Thr Gln Ser Pro Ser Ser Leu Pro Ala Ser Val Gly 1       5
10      15
Asp Arg Val Thr Ile Thr Cys Arg Ala Ser Gln Ser Ile Ser Ser Tyr 20      25
30
Leu Asn Thr Tyr Gln Gln Lys Pro Gly Lys Ala Pro Lys Leu Leu Ile

35      40      45
Tyr Ala Ala Ser Ser Leu Gln Ser Gly Val Pro Ser Arg Phe Ser Gly 50      55
60
Ser Gly Ser Gly Thr Asp Phe Thr Leu Thr Ile Ser Ser Leu Gln Pro 65      70
75      80
Glu Asp Phe Ala Thr Tyr Tyr Cys Gln Gln Ser Tyr Ser Thr His Xaa 85      90
95
Xaa Thr Phe Gly Gln Gly Thr Lys Leu Glu Ile Lys 100     105

<210>   35
<211>   108
<212>   PRT
<213>   Artificial

<220>
<223>   C-terminal VL domain nucleotide sequence of 02/012

<220>
<221>
<222>
<223>

<400>

MISC FEATURE
(96) .. (97)
Xaa is Lys, Asn, Arg, Ser Ile, Met, Glu Asp, Gly, Val, Tyr, Trp, Cys, Leu,
or Phe

35

Asp Ile Gln Met Thr Gln Ser Pro Ser Ser Leu Ser Ala Ser Val Gly 1 5
10 15
Asp Arg Val Thr Ile Thr Cys Arg Ala Ser Gln Ser Ile Ser Ser Tyr 20 25
30
Leu Asn Thr Tyr Gln Gln Lys Pro Gly Lys Ala Pro Lys Leu Leu Ile 35 40
45
Tyr Ala Ala Ser Ser Leu Gln Ser Gly Val Pro Ser Arg Phe Ser Gly 50 55
60
Ser Gly Ser Gly Thr Asp Phe Thr Leu Thr Ile Ser Ser Leu Gln Pro 65 70
75 80
Glu Asp Phe Ala Thr Tyr Tyr Cys Gln Gln Ser Tyr Ser Thr Pro Xaa

85 90 95
Xaa Thr Phe Gly Gln Gly Thr Lys Val Glu Ile Lys 100 105

<210> 36
<211> 20
<212> PRT
<213> Artificial

<220>
<223> HIV gp120 306-325 Clade B peptide

<400> 36

Tyr Asn Lys Arg Lys Arg Ile His Ile Gly Pro Gly Arg Ala Phe Tyr 1 5
10 15

Thr Thr Lys Arg
20

<210> 37
<211> 18
<212> PRT
<213> Artificial

<220>
<223> HIV gp120 306-325 97ZA009 peptide

<400> 37

Thr Arg Lys Ser Met Arg Ile Gly Pro Gly Gln Val Phe Tyr Ala Thr 1 5
10 15

Asn Gly

<210> 38
<211> 13
<212> PRT
<213> Artificial

<220>
<223> HIV gp120 421-433 Clade B peptide

<400> 38

Lys Gln Ile Ile Asn Met Trp Gln Glu Val Gly Lys Ala 1 5 10

<210> 39
<211> 13
<212> PRT
<213> Artificial

<220>
<223> HIV gp120 421-433 97ZA009 peptide

<400> 39
Lys Gln Ile Ile Asn Met Trp Gln Glu Val Gly Arg Ala 1 5 10

<210> 40
<211> 19
<212> PRT
<213> Artificial

<220>
<223> HIV gp120 421-433 peptide epitope

<400> 40

Cys Leu Pro Ser Arg Ile Lys Gln Ile Ile Asn Met Trp Gln Glu Val 1 5
10 15

Gly Lys Ala

<210> 41
<211> 11
<212> PRT
<213> Artificial

<220>
<223> HIV gp120 421-433 peptide epitope

<400> 41

Lys Gln Ile Ile Asn Met Trp Gln Glu Val Gly 1 5 10

<210> 42
<211> 16
<212> PRT
<213> Artificial

<220>
<223> HIV gp120 421-436 peptide epitope

<400>   42

Lys Gln Ile Ile Asn Met Trp Gln Glu Val Gly Lys Ala Met Tyr Ala

1       5       10      15

<210>   43
<211>   107
<212>   PRT
<213>   Artificial

<220>
<223>   Light chain SK18 VL domain

<400>   43
Asp Ile Gln Met Thr Gln Ser Pro Ser Ser Leu Ser Ala Ser Val Gly 1       5
10      15
Asp Arg Val Thr Val Thr Cys Arg Ala Ser Gln Ser Ile Ser Ser Tyr 20      25
30
Leu Asn Trp Tyr Gln Gln Gln Pro Gly Lys Ala Pro Lys Leu Leu Ile 35      40
45
Tyr Ala Ala Ser Ser Leu Gln Ser Gly Val Pro Ser Arg Phe Ser Gly 50      55
60
Ser Gly Ser Gly Thr Asp Phe Thr Leu Thr Ile Ser Ser Leu Gln Pro 65      70
75      80
Glu Asp Phe Ala Thr Tyr Phe Cys Gln Gln Ser Tyr Ser Ile Pro Arg 85      90
95
Thr Phe Gly Gln Gly Thr Lys Val Glu Ile Lys 100 105

<210>   44
<211>   107
<212>   PRT
<213>   Artificial

<220>
<223>   Light chain SK45 VL domain

<400>   44
Asp Ile Gln Met Thr Gln Ser Pro Ser Ser Leu Ser Ala Ser Val Gly 1       5
10      15
Asp Arg Val Thr Ile Thr Cys Arg Ala Ser Gln Gly Ile Arg Asn Asp 20      25
30

Leu Gly Trp Tyr Gln Gln Lys Pro Gly Lys Ala Pro Lys Ala Leu Ile 35      40
45
Tyr Ala Ala Ser Ser Leu Gln Ser Gly Val Pro Ser Arg Phe Ser Gly 50      55
60
Ser Gly Ser Gly Thr Glu Phe Thr Leu Thr Ile Ser Ser Leu Gln Pro 65      70
75      80
Glu Asp Phe Ala Thr Tyr Tyr Cys Leu Gln His Asn Ser Tyr Pro Leu 85      90
95
Thr Phe Gly Gly Gly Thr Lys Val Glu Ile Lys 100 105

<210>   45
<211>   107
<212>   PRT
<213>   Artificial

<220>
<223>   Light chain SKL6 VL domain

<400>   45
Asp Ile Gln Met Thr Gln Ser Pro Ser Ser Leu Ser Ala Ser Val Gly 1       5
10      15
Asp Gly Val Thr Ile Thr Cys Arg Ala Ser Gln Ser Ile Ser Ser Tyr 20      25
30
Leu His Trp Tyr Gln Gln Lys Pro Gly Lys Ala Pro Lys Leu Leu Ile 35      40
45
Tyr Val Ala Ser Ser Leu Gln Ser Gly Val Pro Ser Arg Phe Ser Gly 50      55
60
Ser Gly Ser Gly Thr Asp Phe Thr Leu Thr Ile Ser Ser Leu Gln Pro 65      70
75      80
Glu Asp Phe Ala Thr Tyr Tyr Cys Gln Gln Ser Tyr Ser Thr Pro Ile 85      90
95
Thr Phe Gly Gln Gly Thr Arg Leu Glu Ile Lys

100     105

<210>   46
<211>   11
<212>   PRT
<213>   Artificial

<220>
<223>   HIV gp120 421-431 peptide epitope

<400>   46

Lys Gln Ile Ile Asn Met Trp Gln Glu Val Gly 1   5       10

<210>   47
<211>   108
<212>   PRT
<213>   Artificial

<220>
<223>   VL chain of scFv-t clone GL2

<400>   47

Glu Thr Thr Leu Thr Gln Ser Pro Ala Thr Leu Ser Leu Ser Pro Gly 1       5
10      15

Glu Arg Ala Thr Leu Ser Cys Arg Ala Ser Gln Ser Val Ser Ser Tyr 20      25
30

Leu Ala Trp Tyr Gln Gln Lys Pro Gly Gln Ala Pro Arg Leu Leu Ile 35      40
45

Tyr Asp Ala Ser Asn Arg Ala Thr Gly Ile Pro Ala Arg Phe Ser Gly 50      55
60

Ser Gly Ser Gly Thr Asp Phe Thr Leu Thr Ile Ser Ser Leu Glu Pro 65      70
75      80

Glu Asp Phe Ala Val Tyr Tyr Cys Gln Gln Arg Ser Asn Trp Pro Leu 85      90
95

Thr Phe Gly Gly Gly Thr Lys Leu Glu Ile Lys Arg 100 105

<210> 48
<211> 136

<212> PRT
<213> Artificial

<220>
<223> VH chain of scFv-t clone GL2

<400> 48
Leu Gln Val Gln Leu Gln Gln Ser Gly Pro Arg Leu Val Lys Pro Ser 1 5
10 15
Gly Thr Leu Ser Leu Thr Cys Thr Val Ser Gly Gly Ser Ile Ser Ser 20 25
30
Ser Ser Tyr Tyr Trp Gly Trp Ile Arg Gln His Pro Gly Lys Gly Leu 35 40
45
Glu Trp Ile Gly Tyr Ile Tyr Tyr Ser Gly Ser Thr Tyr Tyr Asn Pro 50 55
60
Ser Leu Lys Ser Arg Val Thr Ile Ser Val Asp Thr Ser Lys Asn Gln 65 70
75 80
Phe Ser Leu Lys Leu Ser Ser Val Thr Ala Ala Asp Thr Ala Val Tyr 85 90
95
Tyr Cys Ala Arg Leu Gln Gly Ala Arg Gly Tyr Ser Tyr Gly Tyr Gly 100 105
110
Ile Asp Tyr Trp Gly Gln Gly Thr Leu Val Thr Val Ser Ser Gly Ser 115 120
125
Ala Ser Ala Pro Thr Leu Phe Pro 130 135

<210> 49
<211> 111
<212> PRT
<213> Artificial

<220>
<223> VL chain of scFv-t clone JL427

<400> 49
Gln Ser Val Leu Thr Gln Pro Pro Ser Val Ser Gly Ala Pro Gly Gln 1 5
10 15

Arg Val Thr Ile Ser Cys Ser Gly Ser Ser Ser Asn Phe Gly Leu Asn 20 25
30
Tyr Val Tyr Trp Tyr Gln His Phe Pro Gly Thr Ala Pro Lys Leu Leu 35 40
45
Ile Tyr Arg Asn Asp Gln Arg Pro Leu Gly Val Pro Ala Arg Phe Ser 50 55
60
Gly Ser Lys Ser Gly Thr Ser Ala Ser Leu Ala Ile Ser Gly Leu Arg 65 70
75 80
Ser Glu Asp Glu Ala Asp Tyr Tyr Cys Gln Ser Tyr Asp Asn Ser Leu 85 90
95
Ser Gly Trp Val Phe Gly Gly Gly Thr Gln Leu Thr Val Leu Gly 100 105 110

<210> 50
<211> 129
<212> PRT
<213> Artificial

<220>
<223>   VH chain of scFv-t clone JL427

<400>   50
Leu Gln Val Gln Leu Gln Gln Ser Gly Gly Gly Leu Val Gln Pro Gly 1       5
10      15
Arg Ser Leu Arg Leu Ser Cys Ala Ala Ser Gly Phe Thr Phe Ser Ser 20      25
30
Tyr Gly Met His Trp Val Arg Gln Ala Pro Gly Lys Gly Leu Glu Trp 35      40
45
Val Ser Tyr Ile Gly Arg Ser Gly Ser His Thr Asn Tyr Ala Asp Ser 50      55
60
Val Lys Gly Arg Phe Thr Ile Ser Arg Asp Asn Ser Lys Asn Thr Leu 65      70
75      80
Tyr Leu Gln Ile Asn Ser Leu Arg Ala Glu Asp Thr Ala Val Tyr Tyr 85      90
95

Cys Ala Arg Gly Leu Pro Asn Tyr Gly Met Asp Ile Trp Gly Gln Gly 100     105
110
Thr Thr Val Thr Val Ser Ser Gly Ser Ala Ser Ala Pro Thr Leu Phe 115     120
125

Pro

<210>   51
<211>   109
<212>   PRT
<213>   Artificial

<220>
<223>   VL chain of scFv-t clone JL606

<400>   51
Glu Thr Thr Leu Thr Gln Ser Pro Gly Ile Leu Ser Leu Ser Pro Gly 1       5
10      15
Glu Arg Ala Thr Leu Ser Cys Arg Ala Ser Gln Arg Val Ser Ser Ser 20      25
30
Tyr Leu Ala Trp Tyr Gln Gln Lys Pro Gly Gln Ala Pro Arg Leu Leu 35      40
45
Ile Tyr Gly Ala Ser Thr Arg Ala Thr Gly Ile Pro Asp Arg Phe Ser 50      55
60
Gly Ser Gly Ser Gly Thr Asp Phe Thr Leu Thr Ile Thr Arg Leu Glu 65      70
75      80
Pro Glu Asp Phe Ala Val Tyr Tyr Cys Gln Gln Tyr Ser Ser Ser Arg 85      90
95
Ser Thr Phe Gly Gln Gly Thr Lys Val Glu Ile Lys Arg 100 105

<210>   52
<211>   132
<212>   PRT
<213>   Artificial

<220>
<223>   VH chain of scFv-t clone JL606

<400>   52
Leu Gln Val Gln Leu Gln Glu Ser Gly Pro Gly Leu Val Lys Pro Ser 1       5

10      15
Glu Thr Leu Ser Leu Thr Cys Thr Val Ser Gly Gly Ser Ile Ser Ser 20      25
30
Tyr Tyr Trp Ser Trp Ile Arg Gln Pro Ala Gly Lys Gly Leu Glu Trp 35      40
45
Ile Gly Arg Ile Tyr Thr Ser Gly Ser Thr Asn Tyr Asn Pro Ser Leu 50      55
60
Lys Ser Arg Val Thr Met Ser Val Asp Thr Ser Lys Asn Gln Phe Ser 65      70
75      80
Leu Lys Leu Ser Ser Val Thr Ala Ala Asp Thr Ala Val Tyr Tyr Cys 85      90
95
Ala Arg His Gln Arg Asp Ile Val Val Val Pro Ala Leu Asp Val Trp 100      105
110
Gly Lys Gly Thr Thr Val Thr Val Ser Ser Gly Ser Ala Ser Ala Pro 115      120
125

Thr Leu Phe Pro 130

<210>   53
<211>   108
<212>   PRT
<213>   Artificial

<220>
<223>   VL chain of scFv-t clone GL59

<400>   53
Ser Tyr Val Leu Thr Gln Pro Pro Ser Val Ser Val Ala Pro Gly Gln 1      5
10      15
Thr Ala Arg Ile Thr Cys Gly Gly Tyr Asn Ile Gly Ser Ser Ser Val 20      25
30

His Trp Tyr Gln Gln Lys Ser Gly Gln Ala Pro Val Leu Val Val Tyr 35      40
45
Asp Asp Ser Asp Arg Pro Ser Gly Ile Pro Glu Arg Phe Ser Gly Ser 50      55
60
Asn Ser Gly Thr Thr Ala Thr Leu Thr Ile Ser Arg Val Glu Ala Gly 65      70
75      80
Asp Glu Ala Asp Tyr Tyr Cys Gln Val Trp Asp Arg Gly Ser Asp Ser 85      90
95
Tyr Val Phe Gly Thr Gly Thr Glu Val Thr Val Leu 100      105

<210>   54
<211>   136
<212>   PRT
<213>   Artificial

<220>
<223>   VH chain of scFv-t clone GL59

<400>   54
Leu Gln Val Gln Leu Val Gln Ser Gly Ala Glu Val Lys Lys Pro Gly 1      5
10      15
Ser Ser Val Lys Val Ser Cys Lys Ala Ser Gly Gly Ala Phe Ser Asn 20      25
30
Tyr Leu Ile Thr Trp Val Arg Gln Ala Pro Gly Gln Gly Leu Glu Tyr 35      40
45
Leu Gly Arg Ile Ile Pro Ile Leu Gln Lys Ser Ser Tyr Ala Gln Lys 50      55
60
Phe Glu Gly Arg Val Thr Phe Thr Ala Asp Lys Ser Thr Ser Thr Ala 65      70
75      80
Tyr Met Glu Leu Thr Arg Leu Thr Ser Asp Asp Thr Ala Val Phe Tyr 85      90

95
Cys Ala Ala Val Arg Ile Val Pro Val Pro Ser Leu Pro Pro Gly Ser

100 105 110
Phe Phe Tyr Trp Gly Leu Gly Thr Val Val Thr Val Ser Ser Ala Ser 115 120
125
Thr Lys Gly Pro Ser Val Phe Pro 130 135

<210> 55
<211> 111
<212> PRT
<213> Artificial

<220>
<223> VL chain of scFv-t clone JL678

<400> 55
Gln Ala Val Leu Thr Gln Pro Ser Ser Ala Ser Gly Thr Pro Gly Arg 1 5
10 15
Arg Val Thr Ile Ser Cys Ser Gly Ser Ser Ser Asn Ile Gly Ser Asn 20 25
30
Thr Val Asn Trp Tyr Gln Gln Leu Pro Gly Thr Ala Pro Lys Leu Leu 35 40
45
Ile Tyr Ser Asn Asn Gln Trp Pro Ser Gly Val Pro Asp Arg Phe Ser 50 55
60
Gly Ser Lys Ser Gly Thr Ser Ala Ser Leu Ala Ile Ser Gly Leu Gln 65 70
75 80
Ser Glu Asp Glu Ala Asp Tyr Tyr Cys Ala Ala Trp Asp Asp Ser Leu 85 90
95
Asn Gly Pro Val Phe Gly Gly Gly Thr Gln Leu Thr Val Leu Gly 100 105 110

<210> 56
<211> 130
<212> PRT
<213> Artificial

<220>
<223> VH chain of scFv-t clone JL678

<400> 56
Leu Gln Val Gln Leu Gln Gln Ser Gly Ser Gly Leu Val Lys Pro Ser 1 5
10 15
Gln Thr Leu Ser Leu Thr Cys Thr Val Ser Gly Val Ser Ile Glu Ser 20 25
30
Gly Ala Ser Tyr Trp Ser Trp Ile Arg Gln Arg Pro Gly Asn Gly Leu 35 40
45
Glu Trp Ile Gly Tyr Ile Ser Tyr Ser Gly Ser Thr Asn Tyr Asn Pro 50 55
60
Ser Leu Lys Ser Arg Val Phe Ile Ser Gly Asp Thr Ser Lys Asn Gln 65 70
75 80
Phe Ser Leu Arg Leu Ser Ser Val Thr Ala Ala Asp Thr Ala Ile Tyr 85 90
95
Tyr Cys Ala Arg Arg Pro Arg Thr Gly Arg Phe Asp Ser Trp Gly Gln 100 105
110
Gly Ala Leu Val Ile Val Ser Ser Ala Ser Thr Lys Gly Pro Ser Val 115 120
125

Phe Pro
130

<210>   57
<211>   16
<212>   PRT
<213>   Artificial

<220>
<223>   SCFV-T VL and VH chain linker

<400>   57

Ser Ser Gly Gly Gly Gly Ser Gly Gly Gly Gly Ser Gly Gly Ser Ala 1       5
10      15


<210>   58
<211>   26
<212>   PRT
<213>   Artificial



<220>
<223>   VH chain of scFv-t clone GL2

<400>   58
Ala Ala Ala His His His His His His Gly Ala Ala Glu Gln Lys Leu 1       5
10      15

Ile Ser Glu Glu Asp Leu Asn Gly Ala Ala
20      25


<210>   59
<211>   110
<212>   PRT
<213>   Artificial

<220>
<223>   VL1 chain of scFv-t clone GL1

<400>   59
Gln Ser Val Leu Thr Gln Pro Pro Ser Val Ser Ala Ala Pro Gly Gln 1       5
10      15
Met Val Thr Ile Ser Cys Ser Gly Ser Ser Ser Asn Ile Lys Asp Asn 20      25
30
Ile Val Ser Trp Tyr Gln Lys Phe Pro Gly Thr Ala Pro Lys Leu Leu 35      40
45
Ile Tyr Asp Asn Glu Arg Arg Pro Ser Gly Ile Pro Asp Arg Phe Ser 50      55
60
Gly Ser Lys Ser Gly Thr Ser Ala Thr Leu Gly Ile Thr Gly Leu Gln 65      70
75      80
Thr Gly Asp Glu Ala Asp Tyr Tyr Cys Gly Thr Trp Asp Asn Ser Leu 85      90
95
Ser Phe Trp Val Phe Gly Gly Gly Thr Lys Val Thr Val Leu 100     105     110

<210>   60
<211>   123
<212>   PRT
<213>   Artificial

<220>

<223>   VH chain of scFv-t clone JL683

<400>   60
Gln Val Gln Leu Gln Glu Ser Gly Pro Gly Leu Val Lys Pro Ser Glu 1         5
10      15
Thr Leu Ser Leu Thr Cys Ala Val Ser Gly Tyr Ser Ile Ser Ser Gly 20        25
30
Tyr Tyr Trp Gly Trp Ile Arg Gln Pro Pro Gly Lys Gly Leu Glu Trp 35        40
45
Ile Gly Glu Ile Tyr His Ser Gly Ser Thr Asn Tyr Asn Pro Ser Leu 50        55
60
Lys Ser Arg Val Thr Ile Ser Val Asp Lys Ser Lys Asn Gln Phe Ser 65        70
75      80
Leu Lys Leu Ser Ser Val Thr Ala Ala Asp Thr Ala Val Tyr Tyr Cys 85        90
95
Ala Ser Arg Leu Gly Tyr Trp Gly Gln Gly Thr Leu Val Thr Val Ser 100       105
110
Ser Gly Ser Ala Ser Ala Pro Thr Leu Phe Pro 115 120

<210>   61
<211>   107
<212>   PRT
<213>   Artificial

<220>
<223>   VL chain of scFv-t clone JL651

<400>   61
Ser Tyr Val Leu Thr Gln Pro Pro Ser Val Ser Val Ser Pro Gly Gln 1         5
10      15
Pro Ala Ser Ile Thr Cys Ser Gly Asp Lys Leu Gly Asp Lys Tyr Ala 20        25
30
Cys Trp Tyr Gln Gln Lys Pro Gly Arg Ser Pro Val Leu Val Ile Tyr 35        40
45

Glu Asp Asn Lys Arg Pro Ser Gly Ile Pro Glu Arg Leu Ser Gly Ser 50        55
60
Asn Ser Gly Asn Thr Ala Thr Leu Thr Ile Ser Gly Thr Gln Ala Leu 65        70
75      80
Asp Glu Ala Asp Tyr Tyr Cys Gln Ala Trp Asp Ser Ser Thr Val Val 85        90
95
Phe Gly Gly Gly Thr Lys Val Thr Val Leu Gly 100 105


<210>   62
<211>   60
<212>   PRT
<213>   Artificial

<220>
<223>   IgVLt tag sequence

<400>   62
Ser Ser Gly Gly Gly Gly Ser Gly Gly Gly Gly Ser Gly Gly Ser Ala 1         5
10      15
Leu Gln Val Gln Leu Gln Gln Ser Gly Gln Thr Lys Gly Pro Ser Val 20        25
30
Phe Pro Ala Ala Ala His His His His His His Gly Ala Ala Glu Gln 35        40
45
Lys Leu Ile Ser Glu Glu Asp Leu Asn Gly Ala Ala 50        55        60

<210>   63
<400>   63
000


<210>   64
<211>   162
<212>   PRT
<213>   Artificial

<220>
<223>   GL2-FR1m VH chain

<400>   64
Leu Gln Val Gln Leu Gln Gln Ser Gly Gly Gly Leu Val Gln Pro Gly 1       5
10      15
Arg Ser Leu Arg Leu Ser Cys Ala Ala Ser Gly Phe Thr Phe Ser Ser 20      25
30
Ser Ser Tyr Tyr Trp Gly Trp Ile Arg Gln His Pro Gly Lys Gly Leu 35      40
45
Glu Trp Ile Gly Tyr Ile Tyr Tyr Ser Gly Ser Thr Tyr Tyr Asn Pro 50      55
60
Ser Leu Lys Ser Arg Val Thr Ile Ser Val Asp Thr Ser Lys Asn Gln 65      70
75      80
Phe Ser Leu Lys Leu Ser Ser Val Thr Ala Ala Asp Thr Ala Val Tyr 85      90
95
Tyr Cys Ala Arg Leu Gln Gly Ala Arg Gly Tyr Ser Tyr Gly Tyr Gly 100     105
110
Ile Asp Tyr Trp Gly Gln Gly Thr Leu Val Thr Val Ser Ser Gly Ser 115     120
125
Ala Ser Ala Pro Thr Leu Phe Pro Ala Ala Ala His His His His His 130     135
140
His Gly Ala Ala Glu Gln Lys Leu Ile Ser Glu Glu Asp Leu Asn Gly 145     150
155     160


Ala Ala


<210>   65
<211>   162
<212>   PRT
<213>   Artificial

<220>
<223>   GL2-FR3m VH chain

<400>   65
Leu Gln Val Gln Leu Gln Gln Ser Gly Pro Arg Leu Val Lys Pro Ser 1       5
10      15
Gly Thr Leu Ser Leu Thr Cys Thr Val Ser Gly Gly Ser Ile Ser Ser 20      25
30
Ser Ser Tyr Tyr Trp Gly Trp Ile Arg Gln His Pro Gly Lys Gly Leu 35      40
45
Glu Trp Ile Gly Tyr Ile Tyr Tyr Ser Gly Ser Thr Tyr Tyr Asn Pro 50      55
60
Ser Leu Lys Ser Arg Phe Thr Ile Ser Arg Asp Asn Ser Lys Asn Thr 65      70
75      80

Leu Tyr Leu Gln Ile Asn Ser Leu Arg Ala Glu Asp Thr Ala Val Tyr 85      90
95
Tyr Cys Ala Arg Leu Gln Gly Ala Arg Gly Tyr Ser Tyr Gly Tyr Gly 100     105
110

Ile Asp Tyr Trp Gly Gln Gly Thr Leu Val Thr Val Ser Ser Gly Ser 115 120
125
Ala Ser Ala Pro Thr Leu Phe Pro Ala Ala Ala His His His His His 130 135
140
His Gly Ala Ala Glu Gln Lys Leu Ile Ser Glu Glu Asp Leu Asn Gly 145 150
155 160

Ala Ala

<210> 66
<400> 66
000

<210> 67
<211> 160
<212> PRT
<213> Artificial

<220>
<223> GL2-CDR1m VH chain

<400> 67
Leu Gln Val Gln Leu Gln Gln Ser Gly Pro Arg Leu Val Lys Pro Ser 1 5
10 15
Gly Thr Leu Ser Leu Thr Cys Thr Val Ser Gly Gly Ser Ile Ser Ser 20 25
30
Tyr Gly Met His Trp Ile Arg Gln His Pro Gly Lys Gly Leu Glu Trp 35 40
45
Ile Gly Tyr Ile Tyr Tyr Ser Gly Ser Thr Tyr Tyr Asn Pro Ser Leu 50 55
60
Lys Ser Arg Val Thr Ile Ser Val Asp Thr Ser Lys Asn Gln Phe Ser 65 70
75 80
Leu Lys Leu Ser Ser Val Thr Ala Ala Asp Thr Ala Val Tyr Tyr Cys 85 90
95
Ala Arg Leu Gln Gly Ala Arg Gly Tyr Ser Tyr Gly Tyr Gly Ile Asp 100 105
110
Tyr Trp Gly Gln Gly Thr Leu Val Thr Val Ser Ser Gly Ser Ala Ser 115 120
125
Ala Pro Thr Leu Phe Pro Ala Ala Ala His His His His His His Gly 130 135
140
Ala Ala Glu Gln Lys Leu Ile Ser Glu Glu Asp Leu Asn Gly Ala Ala 145 150
155 160

<210> 68
<211> 163
<212> PRT
<213> Artificial

<220>
<223> GL2-CDR2m VH chain

<400> 68
Leu Gln Val Gln Leu Gln Gln Ser Gly Pro Arg Leu Val Lys Pro Ser 1 5
10 15
Gly Thr Leu Ser Leu Thr Cys Thr Val Ser Gly Gly Ser Ile Ser Ser 20 25
30
Ser Ser Tyr Tyr Trp Gly Trp Ile Arg Gln His Pro Gly Lys Gly Leu 35 40
45
Glu Trp Ile Gly Tyr Ile Gly Arg Ser Gly Ser His Thr Asn Tyr Ala 50 55
60
Asp Ser Val Lys Gly Arg Val Thr Ile Ser Val Asp Thr Ser Lys Asn 65 70
75 80
Gln Phe Ser Leu Lys Leu Ser Ser Val Thr Ala Ala Asp Thr Ala Val 85 90

95
Tyr Tyr Cys Ala Arg Leu Gln Gly Ala Arg Gly Tyr Ser Tyr Gly Tyr 100 105
110
Gly Ile Asp Tyr Trp Gly Gln Gly Thr Leu Val Thr Val Ser Ser Gly 115 120
125
Ser Ala Ser Ala Pro Thr Leu Phe Pro Ala Ala Ala His His His His 130 135
140
His His Gly Ala Ala Glu Gln Lys Leu Ile Ser Glu Glu Asp Leu Asn 145 150
155 160

Gly Ala Ala

<210> 69
<211> 108
<212> PRT
<213> Artificial

<220>
<223> IgVL2-t VL1 chain

<400> 69

Leu Asp Ile Gln Leu Thr Gln Ser Pro Ser Ser Leu Pro Ala Ser Val 1 5
10 15

Gly Asp Arg Val Thr Ile Thr Cys Arg Ala Ser Gln Ser Ile Ser Ser 20 25
30

Tyr Leu Asn Trp Tyr Gln Gln Lys Pro Gly Lys Ala Pro Lys Leu Leu 35 40
45

Ile Tyr Ala Ala Ser Ser Leu Gln Ser Gly Val Pro Ser Arg Phe Ser 50 55
60

Gly Ser Gly Ser Gly Thr Asp Phe Thr Leu Thr Ile Ser Ser Leu Gln 65 70
75 80

Pro Glu Asp Phe Ala Thr Tyr Tyr Cys Gln Gln Ser Tyr Ser Thr His 85 90
95

Thr Phe Gly Gln Gly Thr Lys Leu Glu Ile Lys Arg 100 105

## Claims

1. An immunoglobulin (Ig) variable (V) domain derived from a light chain subunit (VL) or heavy chain subunit (VH) of an immunoglobulin and having a defined antigen-directed catalytic activity of specifically hydrolyzing a peptide bond in a protein antigen selected from endogenous antigens, bacterial antigens, and the viral antigens.

2. The specific IgV domain of claim 1, which is selected from an organism using an electrophilic analog of said protein antigen containing a synthetic electrophilic group that is bound covalently to a nucleophilic site of a catalytic IgV domain.

3. The antigen-directed IgV domain of claims 1 and 2, that does not indiscriminately hydrolysis of proteins other than the protein from which the electrophilic analog is prepared.

4. The IgV domain of claims 1 to 3, wherein said IgV domain is the first IgV domain that is covalently linked to a second IgV domain to form a dimer having two VL domains or two VH domains or a VL domain linked to a VH domain, wherein said second IgV domain regulates the catalytic rate of the first IgV domain.

5. The IgV domains of claims 1 to 4, wherein one or both V domains have a mutation effective to increase the catalytic activity.

6. The IgV domains of claims 1 to 5, wherein antigen is a B cell superantigen.

7. The IgV domain of claim 1, which has the defined antigenic-catalytic activity of specifically hydrolyzing a peptide bond in a protein selected from the group consisting of: amyloid β polypeptide antigen, Staphylococcus protein antigens Efb, SDR-E, Protein A, Map19, ClfA, LukF and HCV E2 protein antigen.

8. The IgV domain of claims 1 and 2, which has the defined antigenic-catalytic activity of specifically hydrolyzing a peptide bond in amyloid β protein, wherein said defined catalytic activity means a rate equal to or greater than 0.3 mole antigen hydrolyzed per mole IgV domain per min.

9. The IgV domain of claims 1 and 7, which hydrolyzes the His14-Gln15 peptide bond in Aβ40.

10. The IgV domain of claim 1, which comprises an amino acid sequence selected from the groups consisting of:
- IgVL-t' 5D3 (SEQ NO: 1), a single domain IgV: 
EIVLTQSPGTLSLSPGERATLSCRASQSVGGSYLAWYQQKPGQAPRLLIYGASSRAT
GIPDRFSGSGSGTDFTLTISRLEPEDFAVYYCQLYGGSPMYTFGQGTKLEIKRSSGGG
GSGGGGSGGSALQVQLLQSACAPTLFPAAAHHHHHHGAAEQKLISEEDLNGAA;
- IgVL-t' 1E4 (SEQ NO: 2), a single domain IgV: 
QSVLTQPPSVSAAPGQKVTISCSGSSSNIGNNYVSWYQQLPGTAPKLLIYDNERRPSG
IPDRFSGSKSGTSATLAITGLQPGDEADYYCAAWDNTLRGGVFGGGTQLTVLGSSG
GGGSGGGGSGGSALQVQLQESGAGEERGRVWCTHTKGPSVFPAAAHHHHHHGAA
EQKLISEEDLNGAA;
- IgVL-t' 5H3 (SEQ NO: 3), a single domain IgV: 
QSVLTQPPSVSGAPGQRVTISCTGSSSNIGAGYDVHWYQQLPGTAPKLLIYGNSNRP
SGVPDRFSGSKSGTSASLAITGLQAEDEADYYCQSYDSSLSGVVFGGGTQLTVLGSS
GGGGSGGGGSGGSALQVSCGVGSHASAPTLFPAAAHHHHHHGAAEQKLISEEDLN
GAA;
- L-t 5D3 (SEQ NO: 4), a full-length light chain: 
EIVLTQSPGTLSLSPGERATLSCRASQSVGGSYLAWYQQKPGQAPRLLIYGASSRAT
GIPDRFSGSGSGTDFTLTISRLEPEDFAVYYCQLYGGSPMYTFGQGTKLEIKRTVAAP
SVFIFPPSDEQLKSGTASVVCLLNNFYPREAKVQWKVDNALQSGNSQESVTEQDSK
DSTYSLSSTLTLSKADYEKHKVYACEVTHQGLSSPVTKSFNRGECAAAHHHHHHGA
AEQKLISEEDLNGAA;
- IgVL2-t 5D3 (SEQ NO: 5), a two domain homodimeric IgV: 
EIVLTQSPGTLSLSPGERATLSCRASQSVGGSYLAWYQQKPGQAPRLLIYGASSRAT
GIPDRFSGSGSGTDFTLTISRLEPEDFAVYYCQLYGGSPMYTFGQGTKLEIKRSSGGG
GSGGGGSGGSALEIVLTQSPGTLSLSPGERATLSCRASQSVGGSYLAWYQQKPGQAP
 
RLLIYGASSRATGIPDRFSGSGSGTDFTLTISRLEPEDFAVYYCQLYGGSPMYTFGQG
TKLEIKRAAAHHHHHHGAAEQKLISEEDLNGAA;
- IgVL2-t 2E6 (SEQ ID NO: 6), a two domain heterodimeric IgV: 
QSALTQPASVSGSPGQSITISCTGTSSDVGGYNYVSWYQQHPGKAPKLMISEVSNRP
SGVSNRFSGSKSGNTASLTISGPQTEDEADYYCSSYTSSSTPVVFGGGTQLTVLGSSG
GGGSGGGGSGGSALDIQLTQSPSSLPASVGDRVTITCRASQSISSYLNWYQQKPGKA
PKLLIYAASSLQSGVPSRFSGSGSGTDFTLTISSLQPEDFATYYCQQSYSTHTFGQGTK
LEIKRAAAHHHHHHGAAEQKLISEEDLNGAA;
- VH domain 5D3-D4 used to construct two domain scFv-t 5D3-D4 (SEQ NO: 7), a single IgV domain: 
QVQLLQSGAEVKKPGASVKVSCKASGYTFTGYYMHWVRQAPGQGLEWMGWINP
NSGGTNYAQKFQGRVTMTRDTSISTAYMELSRLRSDDTAVYYCARWSHSSGLDYW
GQGTLVTVSSGSACAPTLFPAAAHHHHHHGAAEQKLISEEDLNGAA;
- scFv-t 5D3-D4 containing VH domain 5D3-D4 and VL domain 5D3 (SEQ NO: 8), a two domain scFv: 
EIVLTQSPGTLSLSPGERATLSCRASQSVGGSYLAWYQQKPGQAPRLLIYGASSRAT
GIPDRFSGSGSGTDFTLTISRLEPEDFAVYYCQLYGGSPMYTFGQGTKLEIKRSSGGG
GSGGGGSGGSALQVQLLQSGAEVKKPGASVKVSCKASGYTFTGYYMHWVRQAPG
QGLEWMGWINPNSGGTNYAQKFQGRVTMTRDTSISTAYMELSRLRSDDTAVYYCA
RWSHSSGLDYWGQGTLVTVSSGSACAPTLFPAAAHHHHHHGAAEQKLISEEDLNG
AA;
- VH domain 5D3-D10 used to construct two domain scFv-t 5D3-D10 (SEQ NO: 9), a single IgV domain: 
QQVQLLQSGAEVKKPGASVKVSCKASGYTFTSYDINWVRQATGQGLEWMGWMNP
NSGNTGYAQKFQGRVTMTRNTSISTAYMELSSLRSEGTAVYYCATGSRYDYWGQG
TLVTVSSGSACAPTLFPAAAHHHHHHGAAEQKLISEEDLNGAA;
- scFv-t 5D3-D10 containing VH domain 5D3-D10 and VL domain 5D3 (SEQ NO: 10), a two domain IgV: EIVLTQSPGTLSLSPGERATLSCRASQSVGGSYLAWYQQKPGQAPRLLIYGASSRAT 
GIPDRFSGSGSGTDFTLTISRLEPEDFAVYYCQLYGGSPMYTFGQGTKLEIKRSSGGG
GSGGGGSGGSALQQVQLLQSGAEVKKPGASVKVSCKASGYTFTSYDINWVRQATG
QGLEWMGWMNPNSGNTGYAQKFQGRVTMTRNTSISTAYMELSSLRSEGTAVYYC
ATGSRYDYWGQGTLVTVSSGSACAPTLFPAAAHHHHHHGAAEQKLISEEDLNGAA;
- VH domain 5D3-E4 used to construct two domain scFv-t 5D3-E4 (SEQ NO: 11), a single IgV domain: 
QVQLLQSGPGLVKPSETLSLTCTVSGGSISSGSYYWSWIRQPAGKGLEWIGRIYTSGS
TNYNPSLKSRVTISVDTSKNQFSLKLSSVTAADTAVYYCARVGRLNWFDPWGQGTL
VTVSSGSACAPTLFPAAAHHHHHHGAAEQKLISEEDLNGAA;
- scFv-t 5D3-E4 containing VH domain 5D3-E4 and VL domain 5D3 (SEQ NO: 12), a two domain IgV: 
EIVLTQSPGTLSLSPGERATLSCRASQSVGGSYLAWYQQKPGQAPRLLIYGASSRAT
GIPDRFSGSGSGTDFTLTISRLEPEDFAVYYCQLYGGSPMYTFGQGTKLEIKRSSGGG
GSGGGGSGGSALQVQLLQSGPGLVKPSETLSLTCTVSGGSISSGSYYWSWIRQPAGK
GLEWIGRIYTSGSTNYNPSLKSRVTISVDTSKNQFSLKLSSVTAADTAVYYCARVGR
LNWFDPWGQGTLVTVSSGSACAPTLFPAAAHHHHHHGAAEQKLISEEDLNGAA;
- VH domain 5D3-E6 used to construct two domain scFv-t 5D3-E6 (SEQ NO: 13), a single IgV domain: 
QVQLLQSGPGLVKPSETLSLTCTVSGGSISSGGYYWSWIRQHPGKGLEWIGYIYYSG
STYYNPSLKSRVTISVDTSKNQFSLKLSSVTAADTAVYYCARAGDSSGPGDYWGQG
TLVTVSSGSACAPTLFPAAAHHHHHHGAAEQKLISEEDLNGAA;
- scFv-t 5D3-E6 containing VH domain 5D3-E6 and VL domain 5D3 (SEQ NO: 14), a two domain IgV: 




;
- scFv-t 5D3-E6 containing VH domain 5D3-E6 and VL domain 5D3 (SEQ NO: 14), a two domain IgV: 
QSVLTQPPSVSAAPGQMVTISCSGSSSNIKDNIVSWYQKFPGTAPKLLIYDNERRPSGI
PDRFSGSKSGTSATLAITGLQPGDEADYYCAAWDNTLRGGVFGGGTKVTVL;
- VH domain 1C7 used to construct scFv-t 1C7 (SEQ NO: 18), a single IgV domain: 
EVQLVESGGGVVQPGRSLRLSCAASGFTFSSYGMHWVRQAPGKGLEWVAVIWYD
GSNKYYADSVKGRFTISRDNSKNTLYLLQMNSLRAEDTAVYYCARDPGDCSGGSC
YFDYWGQGTLVTVSS;
- scFv-t 1C7 containing VL domain 1C7 and VH domain 1C7 (SEQ NO: 19), a two domain IgV: 
QSVLTQPPSVSAAPGQMVTISCSGSSSNIKDNIVSWYQKFPGTAPKLLIYDNERRPSGI
PDRFSGSKSGTSATLAITGLQPGDEADYYCAAWDNTLRGGVFGGGTKVTVLGSSGG
GGSGGGGSGGSALEVQLVESGGGVVQPGRSLRLSCAASGFTFSSYGMHWVRQAPG
KGLEWVAVIWYDGSNKYYADSVKGRFTISRDNSKNTLYLQMNSLRAEDTAVYYC
ARDPGDCSGGSCYFDYWGQGTLVTVSSGSASAPTLFPAAAHHHHHHGAAEQKLISE
EDDLNGAA;
- N-terminal VL domain used to construct IgVL2-t 1B4 (SEQ NO: 21), a single IgV domain: 
SSELTQDPAVSVALGQTVRITCQGDSLRSYYASWYQQKPGQAPVLVIYGKNNRPSGI
PDRFSGSSSGNTASLTIYGAQAEDEADYYCNSRDSSGNHWVFGGGTKVTVL;
- C-terminal VL domain used to construct IgVL2-t 1B4 (SEQ NO: 23), a single domain IgV: 
DIQLTQSPSSLPASVGDRVTITCRASQSISSYLNTYQQKPGKAPKLLIYAASSLQSGVP
SRFSGSGSGTDFTLTISSLQPEDFATYYCQQSYSTHTFGQGTKLEIK;
- IgVL2-t 1B4 (SEQ NO: 24), a two domain heterodimeric IgV:

SSELTQDPAVSVALGQTVRITCQGDSLRSYYASWYQQKPGQAPVLVIYGKNNRPSGI
PDRFSGSSSGNTASLTITGAQAEDEADYYCNSRDSSGNHWVFGGGTKVTVLGSSGG
GGSGGGGSGGSALDIQLTQSPSSLPASVGDRVTITCRASQSISSYLNWYQQKPGKAP
KLLIYAASSLQSGVPSRFSGSGSGTDFTLTISSLQPEDFATYYCQQSYSTHTFGQGTK
LEIKRAAAHHHHHHGAAEQKLISEEDDLNGAA;

11. The IgV domains of claims 1 to 6, wherein one or both 1gV domains are derived from the lymphoid cells of a healthy organism without disease or an organism diagnosed with Alzheimer's disease, lupus, a *Staphylococcus aureus infection,* or an HCV infection.

12. The IgV domains of claims 1 to 11, wherein the IgV domains are recloned as components of a full-length IgG, IgM or IgA.

13. The IgV domain of claim 4, wherein the two IgV domains of the dimer are covalently linked by a peptide linker between the termini of the IgV domains.

14. The IgV domain of claims 1 and 11, further comprising a peptide or non-peptide tag effective to reduce or to eliminate intermolecular IgV domain aggregation reactions, facilitate delivery of the IgV domain(s) to an anatomic site of interest or reduce IgV metabolic clearance, and wherein the tag is a peptide, protein or antibody.

15. A pharmaceutical composition comprising one or more of the IgVs of any of claims 1 to 14 and an adjuvant or a diluent.

16. The pharmaceutical composition of claim 15 for treating a pathophysiological condition in a patient.

17. The pharmaceutical composition of 16, wherein the pathophysiological condition is Alzheimer's disease, a Staphylococcus aureus infection, an HCV infection, or an HIV infection.

18. A method for identifying the immunoglobulin variable (1gV) domain(s) of any of claims 1 to 17, comprising: performing a random screening of a library containing a plurality of 1gV domains or fractionating the library of 1gV domains using antigen electrophilic analogs.
